# EUROPEAN PATENT APPLICATION

(11) **EP 3 524 607 A1**
(43) Date of publication of application: **14.08.2019**
(21) Application number: 18155632.5
(22) Date of filing: 07.02.2018
(51) Int. Cl.: C07D 487/16, C07D 495/04, C07D 497/04, C07F 9/53

(54) **BENZO-IMIDAZOINDOLIZINE DERIVATIVE, ORGANIC ELECTROLUMINESCENCE ELEMENT MATERIAL USING THE SAME, AND ORGANIC ELECTROLUMINESCENCE ELEMENT AND ELECTRONIC DEVICE USING THE SAME**

(71) Applicant: Idemitsu Kosan Co., Ltd., Chiyoda-ku, Tokyo 100-8321 (JP)
(72) Inventor: Sustac-Roman, Daniela, 4055 Basel (CH); Murer, Peter, 4104 Oberwil (CH); Shiomi, Takushi, Sodegaura, 299-0293 (JP); Chebotareva, Natalia, 68220 Hagenthal le Bas (FR); Nishimae, Yuichi, 4058 Basel (CH)
(74) Representative: Hollah, Dorothee

(57) **Abstract**

The present invention relates to benzo-imidazoindolizine derivatives, organic electroluminescence devices comprising said derivative, and the use of said derivative in organic electroluminescence devices.

## Description

The present invention relates to benzo-imidazoindolizine derivatives, organic electroluminescence devices comprising said derivative, and the use of said derivative in organic electroluminescence devices.

An organic electroluminescence (EL) device is generally composed of an anode, a cathode, and one or more organic thin film layers sandwiched between the anode and the cathode. When a voltage is applied between the electrodes, electrons are injected from the cathode and holes are injected from the anode into a light emitting region. The injected electrons recombine with the injected holes in the light emitting region to form excited states. When the excited states return to the ground state, the energy is released as light.

A lot of research has been made on the applications of organic EL devices to display, etc. because of their possibility of a wide selection of emission colors by using various emitting materials in a light emitting layer. Particularly, research on the materials which emit three primary red, green, blue colors has been made most actively, and intensive research has also been made to improve their properties.

Aginagalde et al., J. Org. Chem. 2010, 75, 2776-2784 relates to the reaction between benzynes and imidazo[1,2-a]pyridines (pyrimidines) to form benzo[a]imidazo-[5,1,2-cd]indolizines and 2,3,9c-triazocyclopenta[j,k]fluorenes. The tetracyclic compounds emit blue light when excited at 365 nm.

Stasyuk et al., New J. Chem., 2014, 38, 189 discloses a new class of ESIPT-(excited state intramolecular proton transfer) capable molecules, benzo[a]imidazo[5,1,2-cd]indolizines, bearing the 2-hydroxyphenyl substituent, which are prepared in a straightforward manner from imidazo[1,2-a]pyridines via a tandem [8+2]cycloaddition-[2+6+2]dehydrogenation reaction.

Wang et al., Synthesis 2015, 47, 2457-2466 discloses a tandem synthesis of benzo[a]imidazo[5,1,2-*cd*]indolizines from imidazo-[1,2-a]pyridines and o-dihaloarenes.

MX 2013014697A describes the synthesis of compounds derived from coumarin-benzo-imidazo-indolizine. Said compounds show non-linear properties and may be used as cell trackers, in the visualization and quantification of heat, generation and manufacture of wave guides in communication systems and optic switches, and in the manufacture of sensors.

An object of the invention is to provide a new material useful for organic EL devices.

As a result of extensive research, the inventors have found that a benzo-imidazoindolizine derivative is useful as a material for organic EL devices.

The present invention therefore relates to a compound of general formula (I) 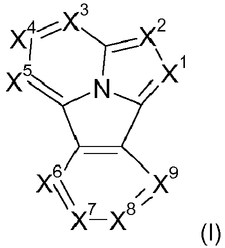 wherein
X¹ is CR¹ or N,
X² is CR² or N,
X³ is CR³ or N,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
wherein at least one of X¹ and X² is N,
R¹, R², R³, R⁴, R⁵, R⁶, R⁷, R⁸ and R⁹
   are independently of each other H, -CN, a C₁-C₂₅alkyl group which is unsubstituted or substituted by at least one group E and/or interrupted by at least one group D, a group of formula -(L¹)ₒ-(L²)ₚ₋(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen,
wherein at least one of X¹, X², X³, X⁴, X⁵, X⁶, X⁷, X⁸ and X⁹ is CR¹, CR², CR³, CR⁴, CR⁵, CR⁶, CR⁷, CR⁸ or CR⁹, wherein R¹, R², R³, R⁴, R⁵, R⁶, R⁷, R⁸ or R⁹ is -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of R³, R⁴, R⁵, R⁶, R⁷, R⁸ and R⁹ if present at adjacent carbon atoms together form at least one C₆-C₁₈aryl or C₁-C₂₄heteroaryl ring or ring system,
L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E,
R¹⁰ is H, -CN or a C₁-C₂₅alkyl group which is unsubstituted or substituted by at least one group E and/or interrupted by at least one group D,
o is independently of each other 0 or 1, p is independently of each other 0 or 1, q is independently of each other 0 or 1 and r is independently of each other 0 or 1, wherein at least one of o, p, q and r is 1;
D is independently of each other -CO-, -COO-, -S-, -SO-, -SO₂-, -O-, -CR¹⁵=CR¹⁶-, -NR¹⁷-, -SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, a C₆-C₄₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₂₄heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R^{22,} R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein, in the case that o + p + q + r is 1, L¹, L², L³ respectively L⁴ is selected from a C₁₀-C₄₀arylene group which is unsubstituted or substituted by at least one group E and a heteroarylene group having 10 to 24 ring atoms, which is unsubstituted or substituted by at least one group E.

The invention further relates to an organic electroluminescence device comprising a cathode, an anode, and a plurality of organic layers provided between the cathode and the anode, wherein at least one of the plurality of organic layers is an emitting layer, wherein at least one organic layer comprises at least one inventive benzo-imidazoindolizine derivative represented by formula (I).

The invention further relates to the use of an inventive benzo-imidazoindolizine derivative represented by formula (I) in an organic electroluminescence device.

The present invention provides a novel material useful as a material for organic EL devices and an organic EL device comprising the material. The compounds of formula (I) are suitable as material for electronic devices, preferably OLEDs, especially as a host material, a charge transporting material, charge and/or exciton blocking material, preferably as a host material and/or a charge transporting material, having a good overall performance, especially improved efficiency and/or driving voltage.

FIG. 1 is a schematic illustration showing an example of the structure of an organic electroluminescence device according to an embodiment of the invention.

The term of "XX to YY carbon atoms" referred to by "a substituted or unsubstituted group ZZ having XX to YY carbon atoms" used herein is the number of carbon atoms of the unsubstituted group ZZ and does not include any carbon atom in the substituent of the substituted group ZZ. "YY" is larger than "XX" and each of "XX" and "YY" represents an integer of 1 or more.

The term of "XX to YY atoms" referred to by "a substituted or unsubstituted group ZZ having XX to YY atoms" used herein is the number of atoms of the unsubstituted group ZZ and does not include any atom in the substituent of the substituted group ZZ. "YY" is larger than "XX" and each of "XX" and "YY" represents an integer of 1 or more.

The term "C_{ZZ}-C_{YY}-" referred to by "C_{ZZ}-C_{YY}-ZZ group which is unsubstituted or substituted" used herein is the number of carbon atoms of the unsubstituted group ZZ and does not include any heteroatom or any atom in the substituent of the substituted group ZZ. "YY" is larger than "XX" and each of "XX" and "YY" represents an integer of 1 or more.

The term of "unsubstituted group ZZ" referred to by "a substituted or unsubstituted group ZZ" used herein means the group ZZ wherein no hydrogen atom is substituted by a substituent.

The number of "ring carbon atoms" referred to herein means the number of the carbon atoms included in the atoms which are members forming the ring itself of a compound in which a series of atoms is bonded to form the ring (for example, a monocyclic compound, a fused ring compound, a cross-linked compound, a carbocyclic compound, and a heterocyclic compound). If the ring has a substituent, the carbon atom in the substituent is not included in the ring carbon atom. The same applies to the number of "ring carbon atom" described below, unless otherwise noted. For example, a benzene ring has 6 ring carbon atoms, a naphthalene ring has 10 ring carbon atoms, a pyridinyl group has 5 ring carbon atoms, and a furanyl group has 4 ring carbon atoms. If a benzene ring or a naphthalene ring has, for example, an alkyl substituent, the carbon atom in the alkyl substituent is not counted as the ring carbon atom of the benzene or naphthalene ring. In case of a fluorene ring to which a fluorene substituent is bonded (inclusive of a spirofluorene ring), the carbon atom in the fluorene substituent is not counted as the ring carbon atom of the fluorene ring.

The number of "ring atom" referred to herein means the number of the atoms which are members forming the ring itself (for example, a monocyclic ring, a fused ring, and a ring assembly) of a compound in which a series of atoms is bonded to form the ring (for example, a monocyclic compound, a fused ring compound, a cross-linked compound, a carbocyclic compound, and a heterocyclic compound). The atom not forming the ring (for example, hydrogen atom(s) for saturating the valence of the atom which forms the ring) and the atom in a substituent, if the ring is substituted, are not counted as the ring atom. The same applies to the number of "ring atoms" described below, unless otherwise noted. For example, a pyridine ring has 6 ring atoms, a quinazoline ring has 10 ring atoms, and a furan ring has 5 ring atoms. The hydrogen atom on the ring carbon atom of a pyridine ring or a quinazoline ring and the atom in a substituent are not counted as the ring atom. In case of a fluorene ring to which a fluorene substituent is bonded (inclusive of a spirofluorene ring), the atom in the fluorene substituent is not counted as the ring atom of the fluorene ring.

The definition of "hydrogen atom" used herein includes isotopes different in the neutron numbers, i.e., light hydrogen (protium), heavy hydrogen (deuterium), and tritium.

The optional substituent may be further substituted with an optional substituent mentioned above. The optional substituents may be bonded to each other to form a ring.

The residues mentioned in the specification of the present application generally have the following preferred meanings, if said residues are not further specified in specific embodiments mentioned below:
Halogen is fluorine, chlorine, bromine and iodine.

C₁-C₂₅alkyl, preferably C₁-C₁₈alkyl, is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups, preferably C₁-C₁₈alkoxy groups, are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octade-cyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₄₀aryl, preferably C₆-C₂₄aryl, particularly preferably C₆-C₁₈aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₆-C₂₄aryloxy, which optionally can be substituted, is typically C₆-C₁₀aryloxy, which optionally can be substituted by one, or more C₁-C₈alkyl and/or C₁-C₈alkoxy groups, such as, for example, phenoxy, 1-naphthoxy, or 2-naphthoxy.

C₁-C₂₄heteroaryl, preferably C₂-C₂₀heteroaryl, particularly preferably C₂-C₁₃heteroaryl, which optionally can be substituted, represents a ring with five to seven ring atoms or a condensed ring system, wherein nitrogen, oxygen or sulfur are the possible hetero atoms, and is typically a heterocyclic group with 5 to 40 atoms having at least six conjugated π-electrons such as thienyl, benzothiophenyl, dibenzothiophenyl, thianthrenyl, furyl, furfuryl, 2H-pyranyl, benzofuranyl, iso-benzofuranyl, dibenzofuranyl, phenoxythienyl, pyrrolyl, imidazolyl, pyrazolyl, pyridyl, bipyridyl, triazyl, pyrimidyl, pyrazinyl, pyridazinyl, indolizinyl, isoindolyl, indolyl, indazolyl, purinyl, quinolizinyl, quinolyl, isoquinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₇-C₂₅aralkyl, which optionally can be substituted, is for example benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₅-C₁₂cycloalkyl, which optionally can be substituted, is for example cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

Examples of the alkylene group (i.e. alkane-diyl group) having 1 to 25 carbon atoms represented include methylene group, ethylene group, n-propylene group, isopropylene group, n-butylene group, s-butylene group, isobutylene group, t-butylene group, n-pentylene group, n-hexylene group, n-heptylene group, n-octylene group, n-nonylene group, n-decylene group, n-undecylene group, n-dodecylene group, n-tridecylene group, n-tetradecylene group, n-pentadecylene group, n-hexadecylene group, n-heptadecylene group, n-octadecylene group, neopentylene group, 1-methylpentylene group, with methylene group, ethylene group, n-propylene group, isopropylene group, n-butylene group, s-butylene group, isobutylene group, t-butylene group being preferred. The alkylene group is substituted or unsubstituted.

Examples of the cycloalkylene group (i.e. cycloalkane-diyl group) having 5 to 12 carbon atoms include cyclopropylene group, cyclobutylene group, cyclopentylene group, cyclohexylene group, cyclooctylene group, and adamantylene group, with cyclopentylene group, and cyclohexylene group being preferred. The cycloalkylene group is substituted or unsubstituted.

The arylene group is preferably a divalent aromatic hydrocarbon group having 6 to 40 ring carbon atoms, more preferably having 6 to 24 ring carbon atoms, most preferably having 6 to 18 ring carbon atoms, may be a non-condensed divalent aromatic hydrocarbon group or a condensed divalent aromatic hydrocarbon group. Specific examples thereof include phenylene group, naphthylene group, phenanthrylene group, biphenyl-diyl group, terphenyl-diyl group, quaterphenyl-diyl group, fluoranthen-diyl group, triphenylenylene-diyl group, phenanthrene-diyl group, fluorene-diyl group, spirofluorene-diyl group, 9,9-diphenylfluorene-diyl group, 9,9'-spirobi[9H-fluorene]-2-diyl group, 9,9-dimethylfluorene-diyl group, benzo[c]phenanthrene-diyl group, benzo[a]triphenylene-diyl group, naphtho[1,2-c]phenanthrene-diyl group, naphtho[1,2-a]triphenylenylene-diyl group, dibenzo[a,c]triphenylenylene-diyl group, and benzo[b]fluoranthene-diyl group, with phenylene group, naphthylene group, biphenyl-diyl group, terphenyl-diyl group, phenanthryl-diyl group, triphenylenylen-diyl group, fluorene-diyl group, spirobifluorene-diyl group, and fluoranthene-diyl group being preferred, and 1,2-phenylene group, 1,3-phenylene group, 1,4-phenylene group, 1,4-naphthylene group, 1,8-naphthylene group, 2,6-naphthylene group, 2,7-naphthylene group, biphenyl-2,2'-diyl group, biphenyl-2,3'-diyl group, biphenyl-2,4'-diyl group, biphenyl-2,5'-diyl group, biphenyl-2,6'-diyl group, biphenyl-3,3'-diyl group, biphenyl-3,4'-diyl group, biphenyl-3,5'-diyl group, biphenyl-3,6'-diyl group, biphenyl-4,4'-diyl group, biphenyl-4,5'-diyl group, biphenyl-4,6'-diyl group, biphenyl-5,5'-diyl group, biphenyl-5,6'-diyl group, biphenyl-6,6'-diyl group, phenanthrene-9,10-diyl group, phenanthrene-2,3-diyl group, phenanthrene-2,7-diyl group, phenanthrene-2,8-diyl group, phenanthrene-2,6-diyl group, phenanthrene-2,9-diyl group, phenanthrene-2,10-diyl group, phenanthrene-3,9-diyl group, phenanthrene-3,10-diyl group, triphenylene-2,3-diyl group, triphenylene-2,5-diyl group, triphenylene-2,6-diyl group, triphenylene-2,7-diyl group, triphenylene-2,8-diyl group, 9,9-dimethylfluorene-2,7-diyl group, 9,9-dimethylfluorene-3,7-diyl group, 9,9-dimethylfluorene-1,4-diyl group, fluoranthene-3,9-diyl group, fluoranthene-3,8-diyl group, fluoranthene-3,4-diyl group, fluoranthene-3,5-diyl group, fluoranthene-3,6-diyl group, fluoranthene-2,9-diyl group, fluoranthene-2,8-diyl group, fluoranthene-2,4-diyl group, fluoranthene-2,5-diyl group, fluoranthene-2,6-diyl group, fluoranthene-1,9-diyl group, fluoranthene-1,8-diyl group, fluoranthene-1,4-diyl group, fluoranthene-1,5-diyl group, fluoranthene-1,6-diyl group being more preferred. The arylene group is substituted or unsubstituted.

The heteroarylene group is preferably a divalent heterocyclic group having 5 to 30 ring atoms, more preferably having 5 to 22 ring carbon atoms, most preferably having 5 to 18 ring carbon atoms, may be a non-condensed heterocyclic group or a condensed heterocyclic group. Specific examples thereof include the divalent residues of pyrrole ring, isoindole ring, benzofuran ring, isobenzofuran ring, dibenzothiophene ring, isoquinoline ring, quinoxaline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the divalent residues of derivatives of these rings, with the divalent residues of dibenzofuran ring, carbazole ring, dibenzothiophene ring, and derivatives of these divalent rings being preferred, and the dibenzofuran-diyl group, 9-phenylcarbazole-diyl group and dibenzothiophene-diyl group being more preferred. The heteroarylene group is substituted or unsubstituted.

The abovementioned groups are substituted or unsubstituted. Possible preferred optional substituents of the above-mentioned groups are C₁-C₈alkyl, a hydroxyl group, a mercapto group, C₁-C₈alkoxy, C₁-C₈alkylthio, halogen, halo-C₁-C₈alkyl, C₆-C₂₄aryl, C₂-C₃₀heteroaryl, or a cyano group.

The optional substituents mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

### Benzo-imidazoindolizine Derivative

The compound of the present invention in an aspect of the invention is represented by formula (I): 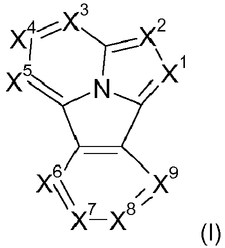 wherein X¹, X², X³, X⁴, X⁵, X⁶, X⁷, X⁸ and X⁹ have the meanings as mentioned above. Preferred embodiments are explained in the following.

X¹ in the general formula (I) is preferably CR¹.

X² in the general formula (I) is preferably N.

Therefore the present invention preferably relates to compounds according to the following general formula (Ia), 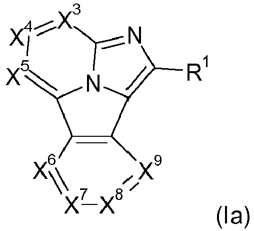 wherein independently of each other X³, X⁴, X⁵, X⁶, X⁷, X⁸ and X⁹ have the same meanings as mentioned above.

Preferably, 0, 1 or 2 of X³, X⁴, X⁵, X⁶, X⁷, X⁸ and X⁹ in the compounds of formula (I) or (Ia) are N, more preferably, 0 of X³, X⁴, X⁵, X⁶, X⁷, X⁸ and X⁹ in the compounds of formula (I) or (Ia) are N, i.e. X³ is CR³, X⁴ is CR⁴, X⁵ is CR⁵, X6 is CR⁶, X⁷ is CR⁷, X⁸ is CR⁸ and X⁹ is CR⁹.

More preferred compounds of formula (I) are therefore compounds of the following general formula (Ia1):

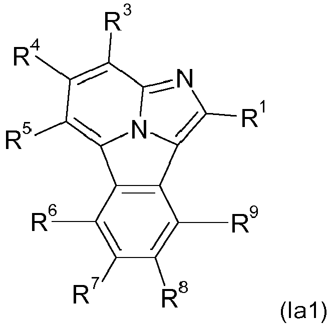

*In formula (I), (Ia) and (Ia1):*
At least one of X¹ and X² is N, preferably X¹ is CR¹ and X² is N.
R¹, R², R³, R⁴, R⁵, R⁶, R⁷, R⁸ and R⁹
   are independently of each other H, -CN, a C₁-C₂₅alkyl group which is unsubstituted or substituted by at least one group E and/or interrupted by at least one group D, a group of formula -(L¹)ₒ-(L²)ₚ₋(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen,
wherein at least one of X¹, X², X³, X⁴, X⁵, X⁶, X⁷, X⁸ and X⁹ is CR¹, CR², CR³, CR⁴, CR⁵, CR⁶, CR⁷, CR⁸ or CR⁹, wherein R¹, R², R³, R⁴, R⁵, R⁶, R⁷, R⁸ or R⁹ is -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of R³, R⁴, R⁵, R⁶, R⁷, R⁸ and R⁹ if present at adjacent carbon atoms together form at least one C₆-C₁₈aryl or C₁-C₂₄heteroaryl ring or ring system.

D is -CO-, -COO-, -S-, -SO-, -SO₂-, -O-, -CR¹⁵=CR¹⁶-, -NR¹⁷-, -SiR²²R²³-, -POR²⁵-, -C≡C-, preferably -O-, -NR¹⁷-, -SiR²²R²³-.

E is -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; a C₁-C₂₅alkyl group, which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; or a C₅-C₁₂cycloalkyl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group; preferably E is independently of each other selected from -NR¹⁷R¹⁸, -CN, -SiR²²R²³R²⁴ , -POR²⁵R^{26'}, a C₁-C₁₈ alkyl group, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one C₁-C₁₈alkyl group, a C₆-C₁₈aryl group which is unsubstituted or substituted by at least one C₆-C₁₂aryl or by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group.

R¹⁵ and R¹⁶ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group which is interrupted by at least one O, preferably H.

R¹⁷ and R¹⁸ are independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H or a C₁-C₁₈ alkyl group.

R¹⁷ and R¹⁸ together form a five or six membered aliphatic, aromatic or heteroaromatic ring, preferably five or six membered aliphatic ring.

R¹⁹ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²⁰ is H or a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈ alkyl group or a C₁-C₁₈alkyl group, which is interrupted by at least one O, preferably H, a C₆-C₁₈aryl group which is unsubstituted or a C₁-C₁₈alkyl group.

R²¹ is independently of each other H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R^{22,} R²³ and R²⁴ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group.

R²⁵, R^{26'} and R²⁷ are independently of each other H, a C₁-C₁₈alkyl group, a C₆-C₁₈aryl group which is unsubstituted or substituted by at least one C₆-C₁₂aryl or by at least one C₁-C₁₈alkyl group, a C₇-C₂₅aralkyl which is unsubstituted or substituted by at least one group E, a C₅-C₁₂cycloalkyl group which is unsubstituted or substituted by at least one group E, preferably H, a C₆-C₁₈aryl group which is unsubstituted or a C₁-C₁₈alkyl group.

Preferably, R¹, R², R³, R⁴, R⁵, R⁶, R⁷, R⁸ and R⁹
are independently of each other H, -CN, a C₁-C₂₅alkyl group which is unsubstituted or substituted by at least one group E and/or interrupted by at least one group D, a group of formula -(L¹)ₒ-(L²)ₚ₋(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, preferably H,
wherein at least one of X¹, X², X³, X⁴, X⁵, X⁶, X⁷, X⁸ and X⁹ is CR¹, CR², CR³, CR⁴, CR⁵, CR⁶, CR⁷, CR⁸ or CR⁹, wherein R¹, R², R³, R⁴, R⁵, R⁶, R⁷, R⁸ or R⁹, preferably one or both of R¹ and R⁸ or one or both of R¹ and R⁷, is -CN, a C₁-C₂₅alkyl group which is unsubstituted or substituted by at least one group E and/or interrupted by at least one group D, a group of formula -(L¹)ₒ-(L²)ₚ₋(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen.

More preferably, R¹, R², R³, R⁴, R⁵, R⁶, R⁷, R⁸ and R⁹
are independently of each other H or a group of formula -(L¹)ₒ-(L²)ₚ-(L³)_{q}-(L⁴)ᵣ-R¹⁰;
wherein at least one of R¹, R², R³, R⁴, R⁵, R⁶, R⁷, R⁸ and R⁹ is a group of formula -(L¹)ₒ-(L²)ₚ-(L³)_{q}-(L⁴)ᵣ-R¹⁰.

In a further preferred embodiment, R³, R⁴, R⁵, R⁶, R⁷ and R⁹ are H, and one or both of R¹ and R⁸ are independently of each other a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, preferably a group of formula - (L')ₒ-(L²)ₚ-(L³)_{q}-(L⁴)ᵣ-R¹⁰; and the other of R¹ and R⁸ which is not a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E is H or
R³, R⁴, R⁵, R⁶, R⁸ and R⁹ are H, and one or both of R¹ and R⁷ are independently of each other a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, preferably a group of formula -(L¹)ₒ-(L²)ₚ-(L³)_{q}-(L⁴)ᵣ-R¹⁰ and the other of R¹ and R⁷ which is not a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E is H.

Most preferred are therefore compounds of the following formulae: 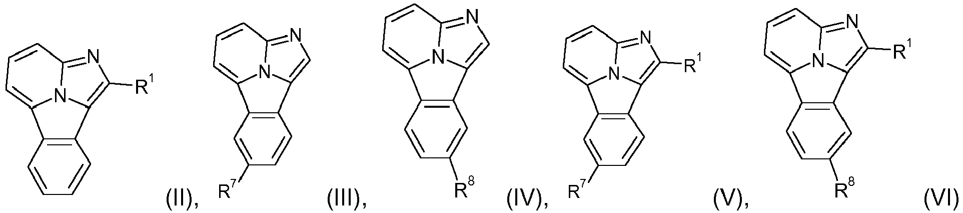 wherein R¹, R⁷ and R⁸ are each independently a group of formula -(L¹)ₒ-(L²)ₚ-(L³)_{q}-(L⁴)ᵣ-R¹⁰ as described above and below.

Further suitable compounds are for example: 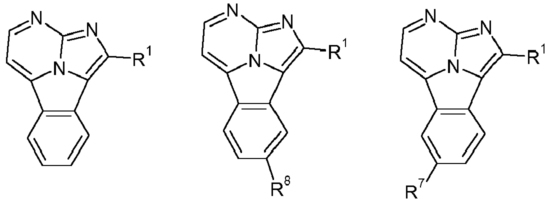 wherein R¹, R⁴, R⁷ and R⁸ are each independently a group of formula -(L¹)ₒ-(L²)ₚ-(L³)_{q}-(L⁴)ᵣ-R¹⁰ as described above and below.

### The group of formula -(L¹)ₒ-(L²)ₚ-(L³)_{q}-(L⁴)ᵣ-R¹⁰

In formula (I), in the group of formula -(L¹)ₒ-(L²)ₚ-(L³)_{q}-(L⁴)ᵣ-R¹⁰, L¹, L², L³ and L⁴, o, p, q, r and R¹⁴ have the following meanings:
L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E. Preferably L¹. L², L³ and L⁴ are independently of each other selected from a C₆-C₄₀arylene group which is unsubstituted and from a C₁-C₂₄heteroarylene group which is unsubstituted.

R¹⁰ is H, -CN or a C₁-C₂₅alkyl group which is unsubstituted or substituted by at least one group E and/or interrupted by at least one group D. More preferably, R¹⁰ is H or C₁-C₈alkyl, which is linear or branched. Most preferably, R¹⁰ is H.

o is independently of each other 0 or 1, p is independently of each other 0 or 1, q is independently of each other 0 or 1 and r is independently of each other 0 or 1, wherein at least one of o, p, q and r is 1, preferably, the sum of o, p, q and r is 1 or 2.

Most preferably, the group of formula -(L¹)ₒ-(L²)ₚ-(L³)_{q}-(L⁴)ᵣ-R¹⁰ comprises a C₁-C₂₄N-heteroaryl group or a C₁-C₄₀aryl group, further most preferably a C₁-C₂₄N-heteroaryl group of one of the following formulae (XII), (XIII), (XV), (XVII), (XXI), (XXIV) or (XXV) or a C₁-C₄₀aryl group of the following formulae (XXXIV), (XXXV), (XXXVI), (XXXVII), (XXXVIII) or (XXXIX): 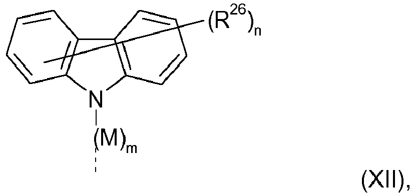 wherein
n is an integer of 0 to 8, preferably 0, 1, 2, 3 or 4, more preferably 0, 1 or 2, most preferably 1,
m is an integer of 0 to 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D,
R²⁶ is independently of each other H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E,
   or
at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
wherein the dotted line is a bonding site;
or 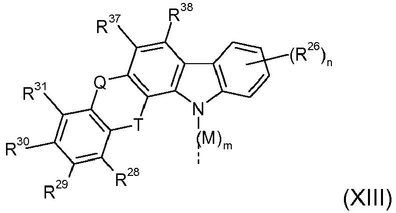
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
   or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 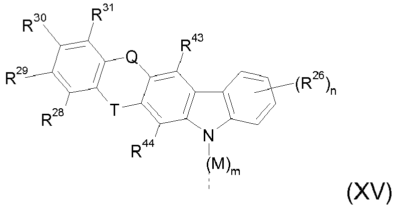
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused, R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
   or
at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably, R³⁴ and R³⁵ are independently of each other H, ethyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group, for example phenyl,
wherein the dotted line is a bonding site,
or 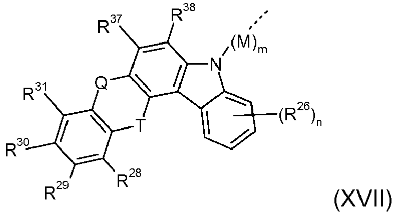
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
   or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site;
or 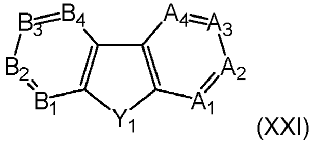 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is NR⁷⁰, CR⁷¹R⁷², O or S;
R⁶², R⁶³, R⁶⁴, R⁶⁵, R⁶⁶, R⁶⁷, R⁶⁸ and R⁶⁹ are independently of each other H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   or
at least two of R⁶², R⁶³, R⁶⁴ and R⁶⁵ may be directly bonded to the moiety represented by the general formula (XXII) via the two *-locations; 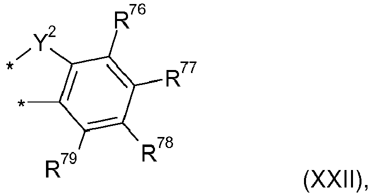
wherein
Y² is NR⁷³, CR⁷⁴R⁷⁵, O or S,
   or
at least two of R⁶⁶, R⁶⁷, R⁶⁸ and R⁶⁹ may be directly bonded to the moiety represented by the general formula (XXIII) via the two *-locations. 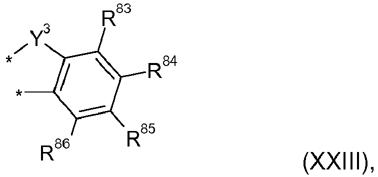
wherein
Y³ is NR⁸⁰, CR⁸¹R⁸², O or S,
R⁷⁰, R⁷³ and R⁸⁰ are independently of each other a direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
R⁷¹, R⁷², R⁷⁴, R⁷⁵, R⁸¹ and R⁸² are, independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
R⁷⁶ and R⁷⁷, R⁷⁷ and R⁷⁸ and/or R⁷⁸ and R⁷⁹ together may form at least one C₆-C₁₈aryl or C₂-C₁₈heteroaryl ring or ring system, and/or
R⁸³ and R⁸⁴, R⁸⁴ and R⁸⁵ and/or R⁸⁵ and R⁸⁶ together form at least one C₆-C₁₈aryl or C₂-C₁₈heteroaryl ring or ring system,
wherein the heterocyclic group according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, wherein this respective residue R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
m is 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D;
or 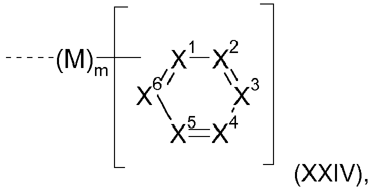 wherein
X¹, X², X³, X⁴, X⁵ and X⁶ are independently of each other CR^{c} or N, wherein in formula at least one of X¹, X², X³, X⁴, X⁵ and X⁶ is N;
R^{c} is in each case independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{c}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites, and
wherein the group - - -(M)ₘ- is either attached to the ring formed by X¹ to X⁶, wherein one of X¹ to X⁶ is a group CR^{c}, wherein R^{c} is a bonding site to - - -(M)ₘ-, or
the group - - -(M)ₘ- is attached to the ring formed of at least two of R^{c}, if present at adjacent carbon atoms;
or 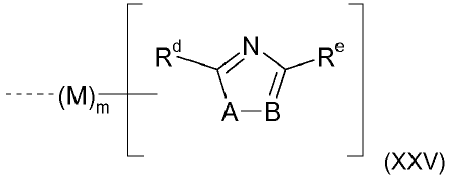 wherein
B is CR^{g} or N;
A is O, S or NR^{h};
R^{d}, R^{e}, R^{g} and R^{h} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{d}, R^{e}, R^{g} and R^{h}, if present at adjacent atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites;
wherein the group - - -(M)ₘ- is either attached to the five membered ring formed by N, A, B and two carbon atoms, wherein one of R^{d}, R^{e}, R^{g} or R^{h} is a bonding site to - - -(M)ₘ-, or the group - - -(M)ₘ- is attached to the ring formed of at least two of R^{d}, R^{e}, R^{g} or R^{h}, if present at adjacent carbon atoms.
or 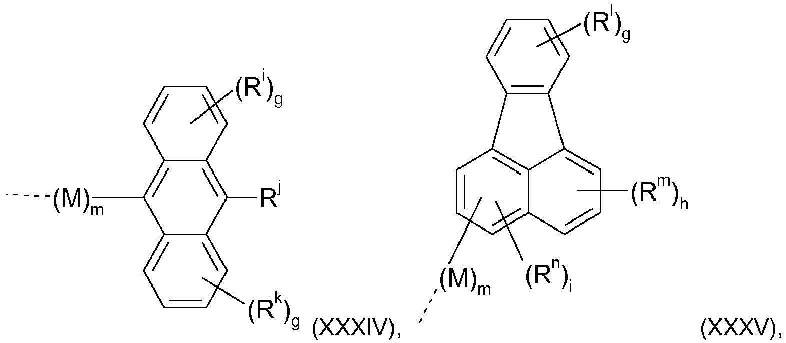 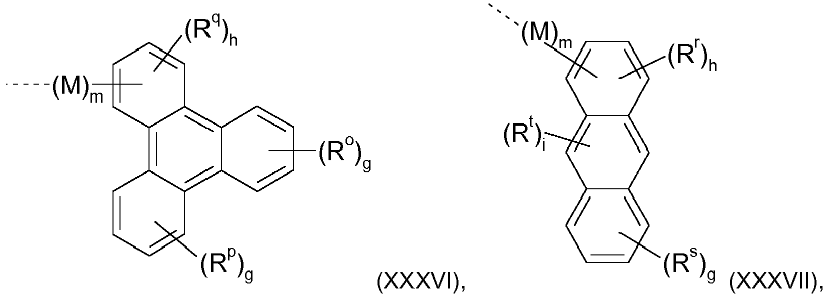 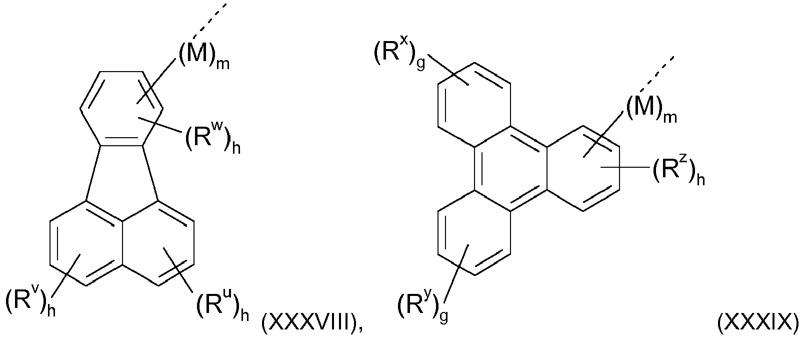 wherein
Rⁱ, R^{j}, R^{k}, R^{l}, R^{m}, Rⁿ, R^{o}, R^{p}, R^{q}, R^{s}, R^{t}, R^{u}, R^{v}, R^{w}, R^{x}, R^{y} and R^{z} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of Rⁱ, R^{k}, R^{l}, R^{m}, Rⁿ, R^{o}, R^{p}, R^{q}, R^{s}, R^{t}, R^{u}, R^{v}, R^{w}, R^{x}, R^{y} and R^{z}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
g is 0, 1, 2, 3 or 4;
h is 0, 1, 2 or 3,
i is 0, 1 or 2;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites.

*The C₁-C₂₄N-heteroaryl groups according to general formulae (XII), (XIII), (XV), (XVII), (XXI), (XXIV) or (XXV) and the C₆-C₄₀aryl groups according to general formulae (XXXIV), (XXXV), (XXXVI), (XXXVII), (XXXVIII) or (XXXIX) are described in the following:*

### N-heteroaryl groups according to general formula (XXI)

In one embodiment of the present invention, the N-heteroaryl group is represented by the general formula (XXI) 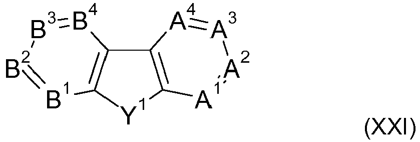 wherein
A¹ is CR⁶² or N, preferably CR⁶²,
A² is CR⁶³ or N, preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
   and/or
at least two of R⁶², R⁶³, R⁶⁴ and R⁶⁵ , if present are directly bonded to the moiety represented by the general formula (XXII) by the two *-locations. 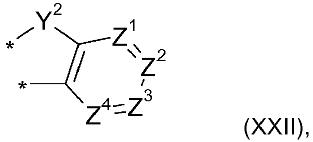
wherein
Y² is NR⁷³, CR⁷⁴R⁷⁵, O or S,
Z¹ is N or CR⁷⁶, preferably CR⁷⁶,
Z² is N or CR⁷⁷, preferably CR⁷⁷,
Z³ is N or CR⁷⁸, preferably CR⁷⁸,
Z⁴ is N or CR⁷⁹, preferably CR⁷⁹,
   and/or
R⁶², R⁶³, R⁶⁴ or R⁶⁵, if present at adjacent carbon atoms, together form at least one C₆-C₁₈aryl or C₂-C₁₈heteroaryl ring or ring system,
   and/or
at least two of R⁶⁶, R⁶⁷, R⁶⁸ and R⁶⁹, if present are directly bonded to the moiety represented by the general formula (XXIII) by the two *-locations. 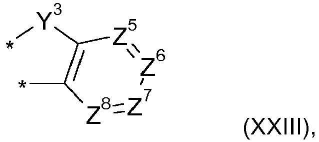
wherein
Y³ is NR⁸⁰, CR⁸¹R⁸², O or S,
Z⁵ is N or CR⁸³, preferably CR⁸³,
Z⁶ is N or CR⁸⁴, preferably CR⁸⁴,
Z⁷ is N or CR⁸⁵, preferably CR⁸⁵,
Z⁸ is N or CR⁸⁶, preferably CR⁸⁶,
   and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably phenyl, biphenyl, naphthyl, phenanthryl, triphenylenyl, fluorenyl, dibenzothienyl, dibenzofuryl, N-phenylcarbazolyl, pyridyl, pyrimidyl, triazyl and quinolyl, which are unsubstituted or substituted by at least one group E, preferably unsubstituted,
R⁷¹, R⁷², R⁷⁴, R⁷⁵, R⁸¹ and R⁸² are, independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably methyl or phenyl, which is unsubstituted or substituted by at least one group E, preferably unsubstituted,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
   and/or
at least two of R⁷⁶, R⁷⁷, R⁷⁸, R⁷⁹, R⁸³, R⁸⁴, R⁸⁵ or R⁸⁶, if present at adjacent carbon atoms, together form at least one C₆-C₁₈aryl or C₂-C₁₈heteroaryl ring or ring system,
wherein the substituent according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, preferably R⁷⁰, R⁷³ or R⁸⁰, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, preferably R⁷⁰, R⁷³ or R⁸⁰, is a direct bond, optionally interrupted by a group of formula -(M)ₘ-, in this case,
m is 0, 1, 2, 3 or 4, preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D, preferably phenylene or naphthylene, which are unsubstituted or substituted by at least one group E, preferably unsubstituted,
wherein D and E have the same meanings as mentioned above.

According to the present invention the heterocyclic group according to general formula (XXI), which may be for example a dibenzofuran, dibenzothiophene or benzofurodibenzofuran group, can be attached to the compound of general formula (I) via any atom present in the heterocyclic ring system. Therefore, any of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, preferably R⁷⁰, R⁷³ or R⁸⁰, if present, can be a direct bond, optionally interrupted by a group of formula -(M)ₘ-, with which the heterocyclic group according to formula (XXI) can be attached to the compound of general formula (I).

Preferably, the heterocyclic group according to general formula (XXI) is represented by any one of general formula (XXIa), (XXIb) or (XXIc) 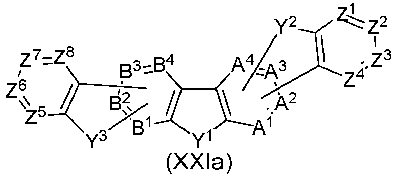 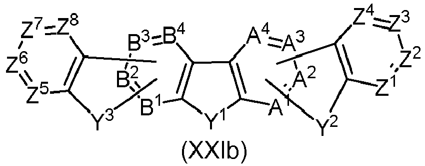 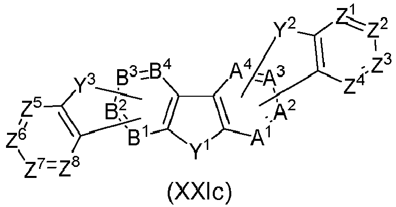 wherein A¹, A², A³, A⁴, B¹, B², B³, B⁴, Z¹, Z2, Z3, Z⁴, Z5, Z6, Z⁷, Z⁸, Y¹, Y² and Y³ have the same meanings as defined above.

According to a further preferred embodiment of the present invention one of Y¹, Y² and Y³ in the compounds according general formulae (XXI), (XXIa), (XXIb) and/or (XXIc) is NR⁷⁰, NR⁷³ or NR⁸⁰ respectively.

According to a further preferred embodiment of the present invention two of Y¹, Y² and Y³ in the compound according to general formulae (XXI), (XXIa), (XXIb) and/or (XXIc) are NR⁷⁰, NR⁷³ or NR⁸⁰ respectively.

Preferably, the N-heteroaryl group according to general formula (XXI) corresponds to a N-heteroaryl group according to general formula (XXI) 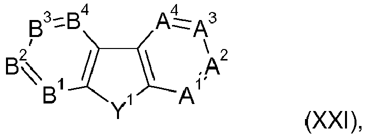
wherein A¹ is CR⁶²,
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
R66, R⁶⁷, R⁶⁸ and R⁶⁹ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
   and
at least two of R⁶², R⁶³, R⁶⁴ and R⁶⁵ are direct bond and, if present at adjacent carbon atoms, are directly connected to the two *-locations in the moiety of general formula (XXII) 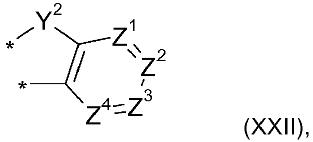
wherein
Y² is NR⁷³, CR⁷⁴R⁷⁵, O or S,
Z¹ is CR⁷⁶,
Z² is CR⁷⁷,
Z³ is CR⁷⁸,
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
wherein R⁷⁶, R⁷⁷, R⁷⁸ and R⁷⁹ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably hydrogen,
R⁷³, R⁷⁴ and R⁷⁵ have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰, CR⁷¹R⁷², O or S
at least two of R⁶², R⁶³, R⁶⁴ and R⁶⁵ are direct bond and, if present at adjacent carbon atoms, are directly connected to the two *-locations in the moiety of general formula (XXII) 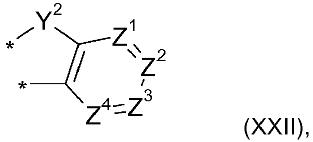 wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl, and
at least two of R⁶⁶, R⁶⁷, R⁶⁸ and R⁶⁹, if present at adjacent carbon atoms, are directly connected to the two *-locations in the moiety of general formula (XXIII) 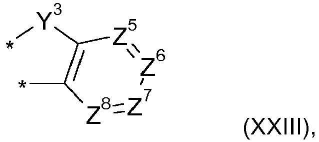 wherein
   Y³ is NR⁸⁰,
   Z⁵ is CR⁸³,
   Z⁶ is CR⁸⁴,
   Z⁷ is CR⁸⁵,
   Z⁸ is CR⁸⁶,
   and the remaining of R⁶⁶, R⁶⁷, R⁶⁸ and R⁶⁹ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
   R⁷⁶, R⁷⁷, R⁷⁸, R⁷⁹, R⁸³, R⁸⁴, R⁸⁵ and R⁸⁶ are independently of each other selected from direct bond, H, E, a C₆-C₄₀aryl group which is unsubstituted or substituted by at least one group E, C₁-C₃₉₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
   R⁷⁰, R⁷¹, R⁷², R⁷³, R⁷⁴ and R⁷⁵ have the same meanings as mentioned above,
   and the substituent according to general formula (XXI) is connected to the compound according to general formula (I) via R⁸⁰ being a direct bond, which is optionally interrupted by a group of formula -(M)ₘ-, wherein M and m have the same meanings as mentioned above, preferably m is 0 or 1.

In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
at least two of R⁶², R⁶³, R⁶⁴ and R⁶⁵ are direct bond and, if present at adjacent carbon atoms, are directly connected to the two *-locations in the moiety of general formula (XXII) 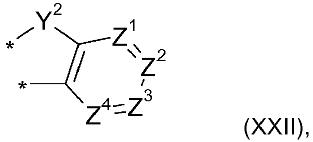 wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
and
at least two of R⁶⁶, R⁶⁷, R⁶⁸ and R⁶⁹, if present at adjacent carbon atoms, are directly connected to the two *-locations in the moiety of general formula (XXIII) 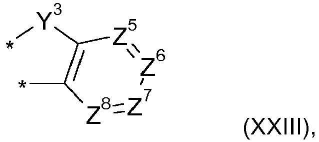 wherein
Y³ is NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and the remaining of R⁶⁶, R⁶⁷, R⁶⁸ and R⁶⁹ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁷⁸, R⁷⁹, R⁸³, R⁸⁴, R⁸⁵ and R⁸⁶ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷³, R⁷⁴, R⁷⁵, R⁸⁰, R⁸¹ and R⁸² have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

### N-heteroaryl groups according to general formula (XIII)

In one embodiment of the present invention, the N-heteroaryl group is represented by the general formula (XIII) 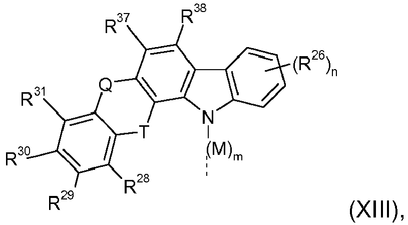
wherein M, m and R²⁶ have the meanings as mentioned above, preferably m is 0; n is 0, 1, 2, 3 or 4, preferably n is 0;
R²⁸, R²⁹, R³⁰, R³¹, R³⁷ and R³⁸ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl, biphenyl, more preferably H;
   or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, preferably NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, wherein R³⁴ and R³⁵ together form a spiro group, preferably H, methyl, ethyl, or phenyl, wherein R³⁴ and R³⁵ together form a spiro group,
R³⁶ is a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, a C₆-C₁₈aryl group which is unsubstituted or substituted by phenyl, or a C₁-C₁₈alkyl group, more preferably a phenyl group;
wherein D and E have the same meanings as mentioned above.

Preferred compounds of formula (XIII) are compounds of the following formula (XIIIa), wherein T is a direct bond, and compounds of formula (XIIIb), wherein Q is a direct bond: 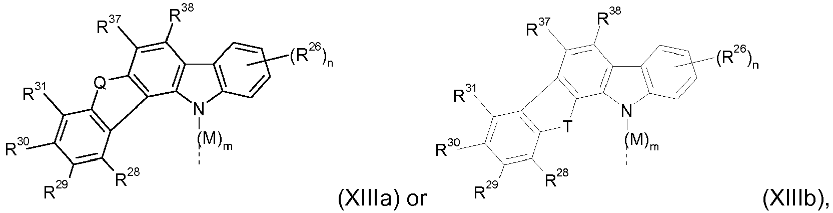 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XIII) are the following compounds: 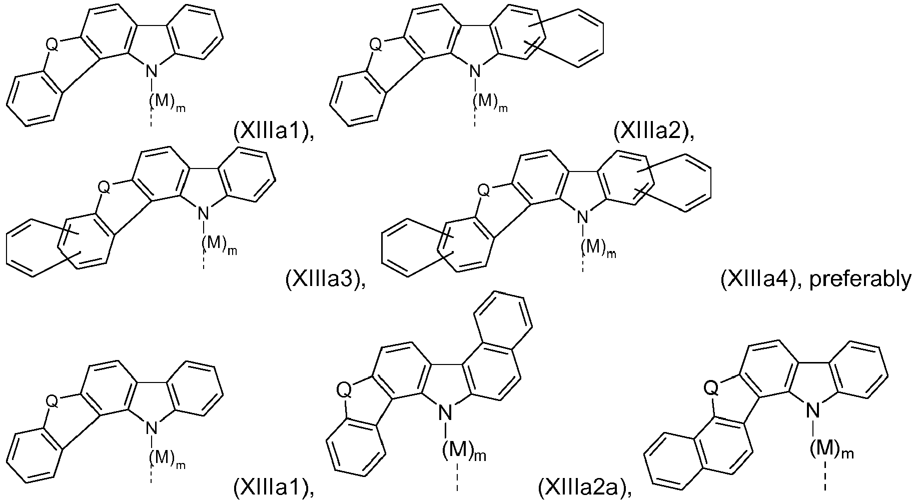 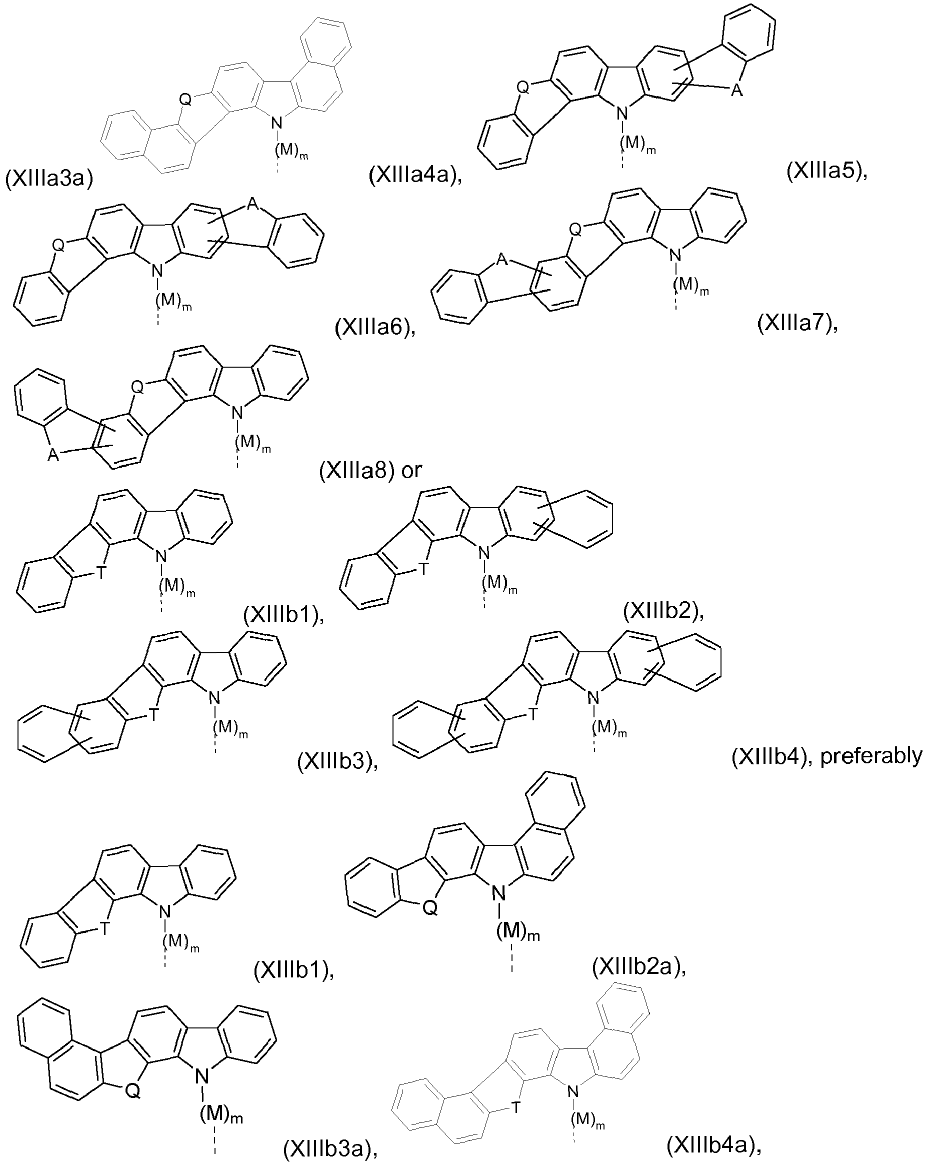 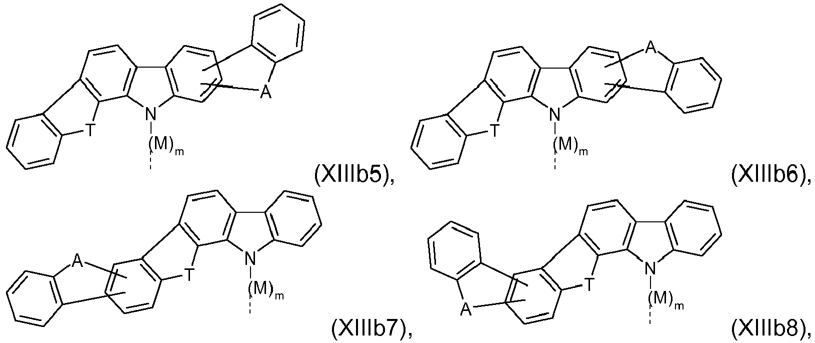 wherein
A is S, O, SiR³²R³³, CR³⁴R³⁵ or NR³⁶, preferably S, O, CR³⁴R³⁵ or NR³⁶, and
Q and T are independently of each other S, O, SiR³²R³³, CR³⁴R³⁵ or NR³⁶, preferably NR³⁶.

According to a particularly preferred embodiment of the present invention, in the compound of formula (XIII), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XIII), Q is direct bond, T is NR³⁶, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XIII), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is NR³⁶, T is direct bond, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

Most preferred compounds of formula (XIII) are compounds of formula (XIII), wherein Q is a direct bond, especially preferred are therefore compounds (XIIIb), more preferably (XIIIb1) to (XIIIb12).

According to a further preferred embodiment, the heteroaryl group according to general formula (XIII) corresponds to general formula (XIV) 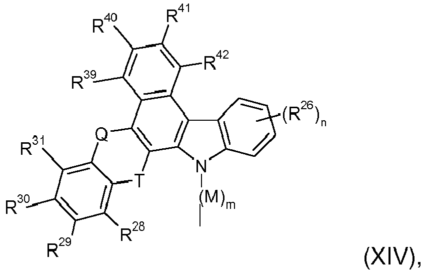
wherein R²⁶, R²⁸, R²⁹, R³⁰, R³¹, M, Q, T, n and m have the same meanings as mentioned above and
R³⁹, R⁴⁰, R⁴¹, R⁴² are independently of each other selected from H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

Preferred compounds of formula (XIV) are compounds of the following formula (XIVa), wherein T is a direct bond, and compounds of formula (XIVb), wherein Q is a direct bond: 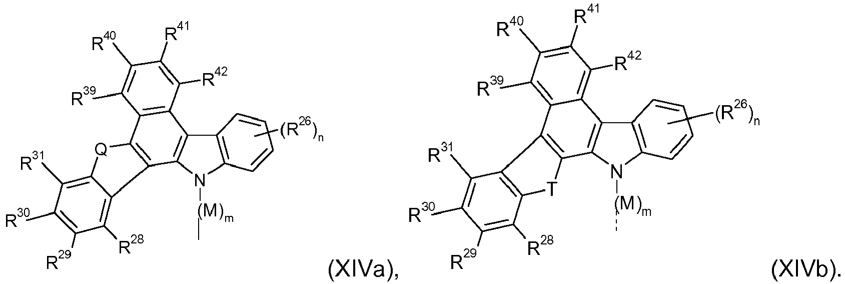 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XIV) are the following compounds: 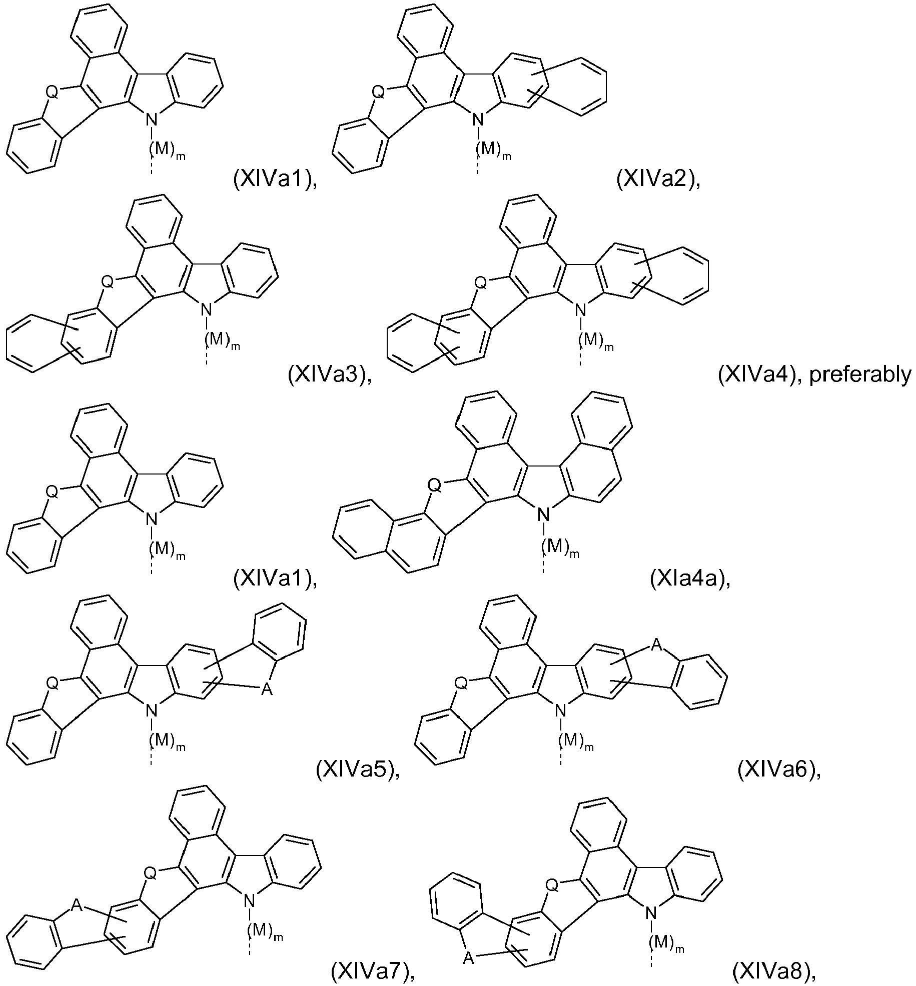 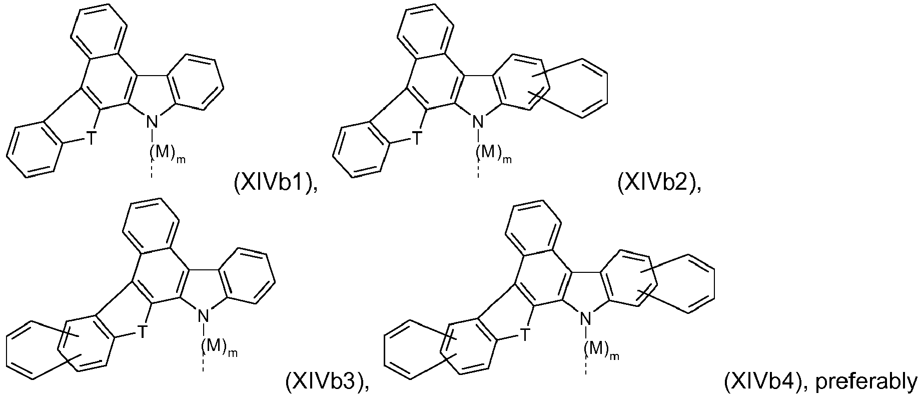 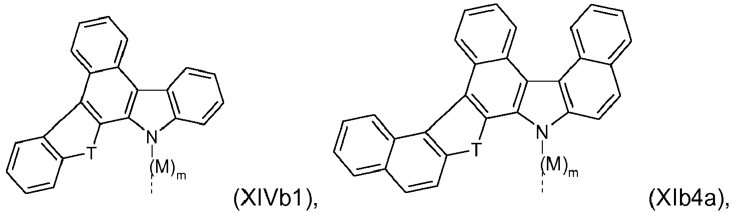 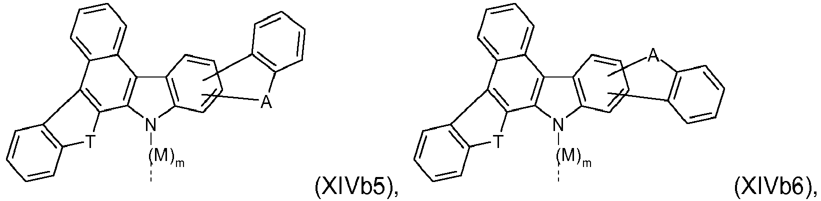 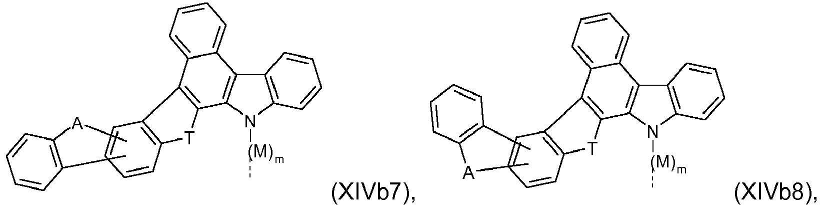 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

### N-heteroaryl groups according to general formula (XV)

In one embodiment of the present invention, the N-heteroaryl group is represented by the general formula 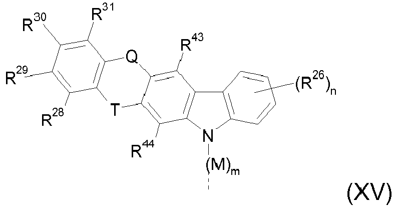
wherein n is 0, 1, 2, 3 or 4, preferably 0, 1 or 2
m is 0, 1, 2, 3 or 4, preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D, preferably, M is a C₆-C₄₀ arylene group which is unsubstituted, a C₁-C₂₄heteroarylene group which is unsubstituted or a C₁-C₂₅ alkylene group which is unsubstituted, particularly preferably, M is a C₆-C₁₈ arylene group which is unsubstituted, most preferably a phenylene group,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
preferably R²⁶ is H or two R²⁶ that are present at adjacent carbon atoms together form a fused phenyl ring,
R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, or at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably, R³⁴ and R³⁵ are independently of each other H, ethyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group, for example phenyl,
wherein the dotted line is a bonding site;
wherein D and E have the same meanings as mentioned above.

Preferred compounds of formula (XV) are compounds of the following formula (XVa), wherein T is a direct bond, and compounds of formula (XVb), wherein Q is a direct bond: 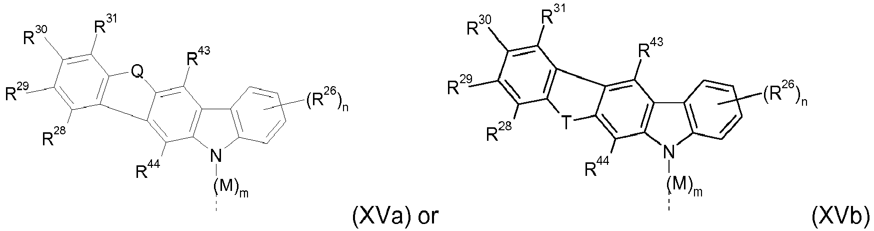 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XV) are the following compounds: 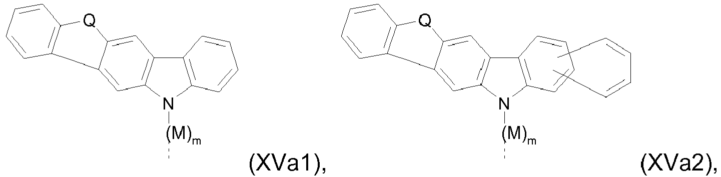 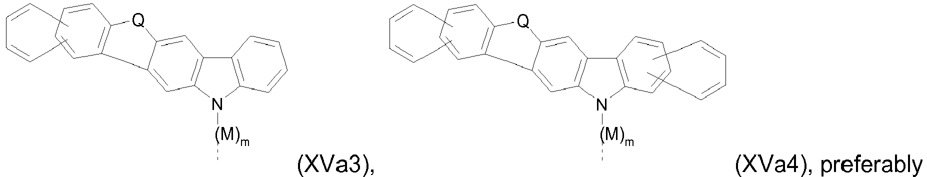 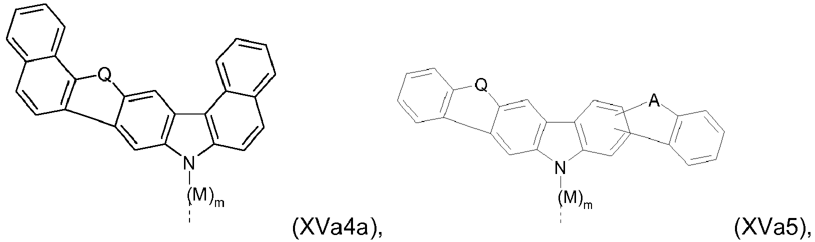 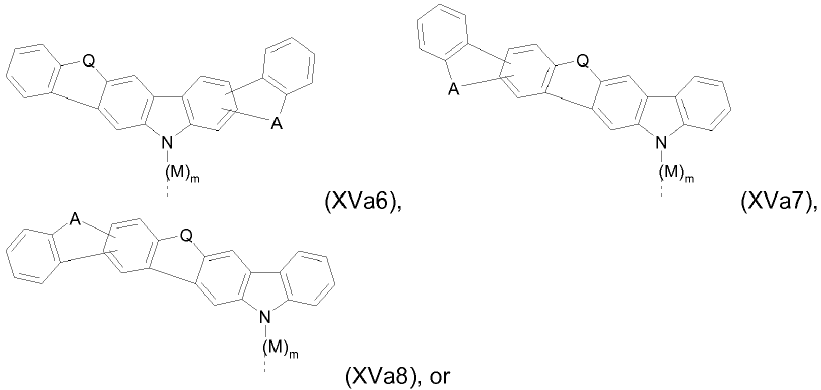 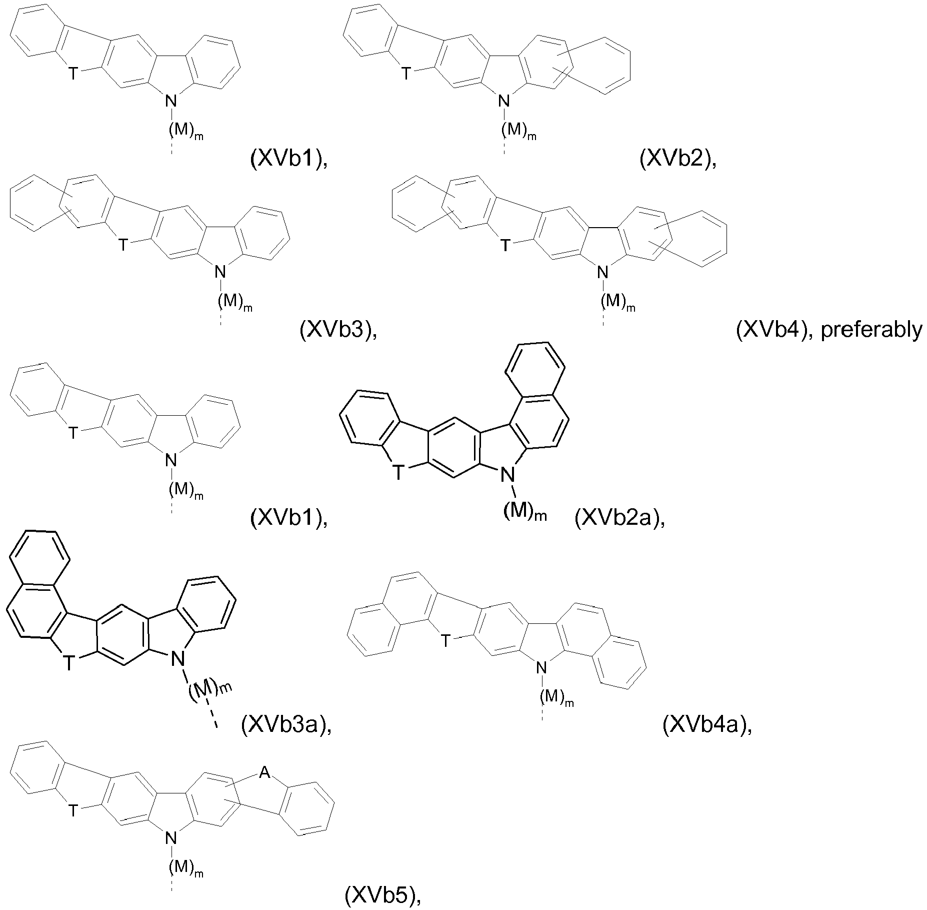 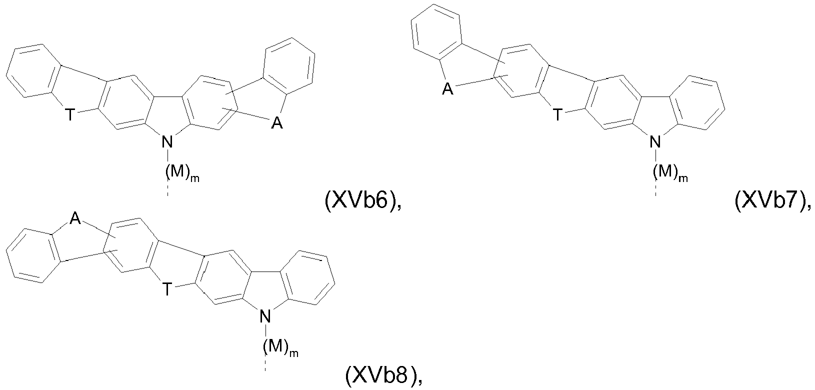 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

According to a particular preferred embodiment of the present invention in substituent according to general formula (XV), m is 0, n is 0, R²⁸, R²⁹, R³⁰, R³¹, R⁴³ and R⁴⁴ are H, Q is a direct bond, T is CR³⁴R³⁵, R³⁴ and R³⁵ are methyl.

According to a further particular preferred embodiment of the present invention in substituent according to general formula (XV), m is 0, n is 2, R²⁸, R²⁹, R³⁰, R³¹, R⁴³ and R⁴⁴ are H, two R²⁶ that are present at adjacent carbon atoms together form a fused phenyl ring, which is preferably present in position of R³⁰ and R³¹, Q is a direct bond, T is CR³⁴R³⁵, R³⁴ and R³⁵ are methyl.

### N-heteroaryl groups according to general formula (XVII)

In one embodiment of the present invention, the N-heteroaryl group is represented by the general formula (XVII) 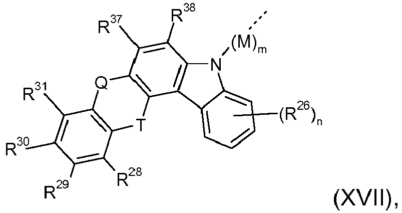
wherein n is 0, 1, 2, 3 or 4, preferably 0, 1 or 2, more preferably 0 or 1, most preferably 0,
m is 0, 1, 2, 3 or 4, preferably 0, 1 or 2, more preferably 0 or 1,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which is unsubstituted or substituted by at least one group E and/or interrupted by at least one group D,
R²⁶ is independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
   or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₆-C₁₂aryl or by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, or a C₅-C₁₂cycloalkyl group which is unsubstituted or substituted by at least one group E, or R³⁴ and R³⁵ together may form a spiro group, preferably R³⁴ and R³⁵ are independently of each other H, methyl, ethyl, or phenyl, or R³⁴ and R³⁵ together may form a spiro group,
R³⁶ is H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₃heteroaryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or substituted by phenyl, or a C₁-C₁₈alkyl group,
wherein the dotted line is a bonding site; and
wherein D and E have the same meanings as mentioned above.

Preferred compounds of formula (XVII) are compounds of the following formula (XVIIa), wherein T is a direct bond, and compounds of formula (XVIIb), wherein Q is a direct bond: 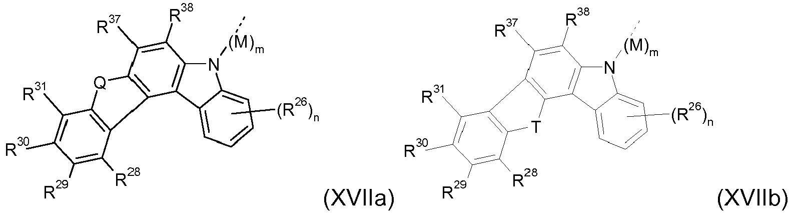 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XVII) are the following compounds: 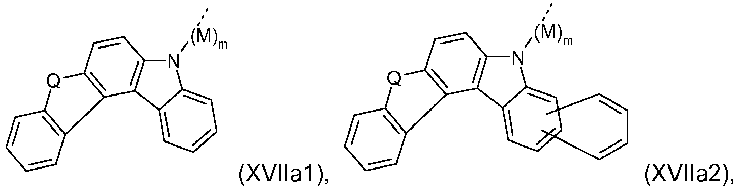 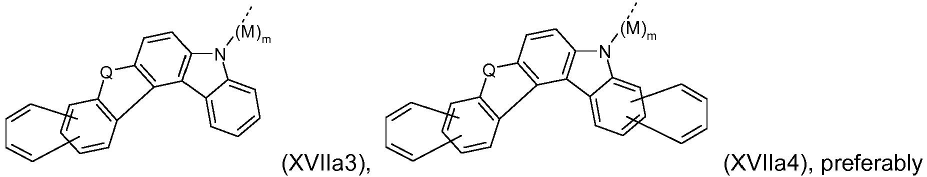 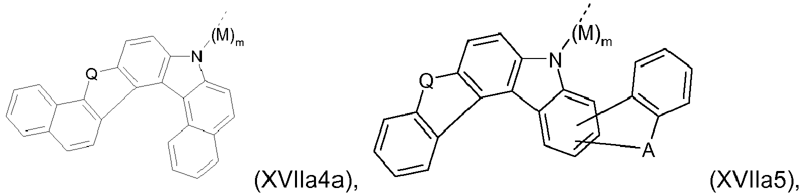 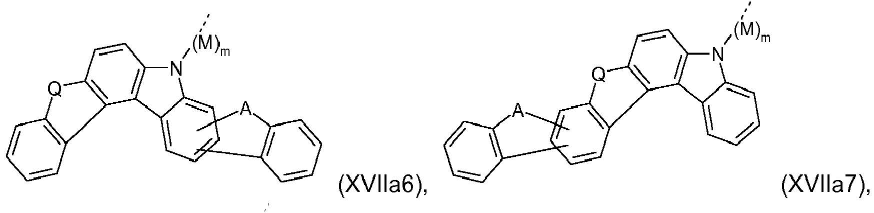 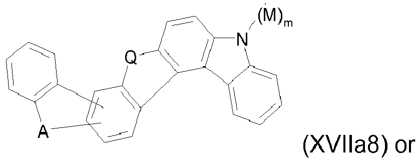 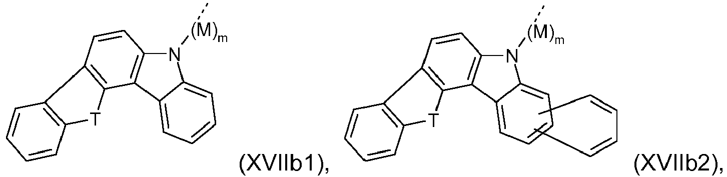 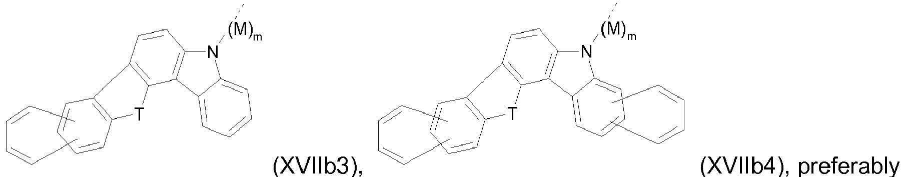 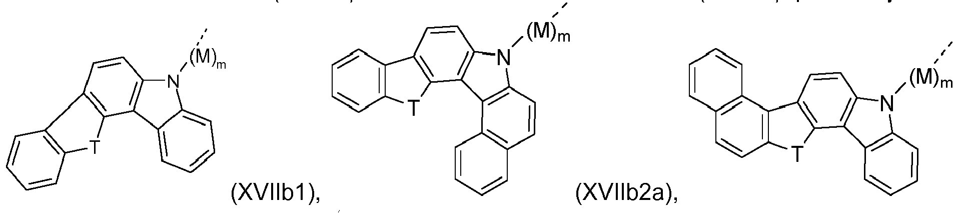 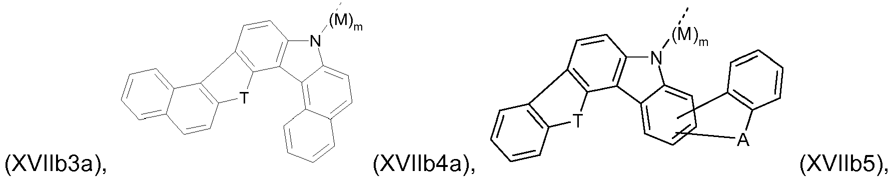 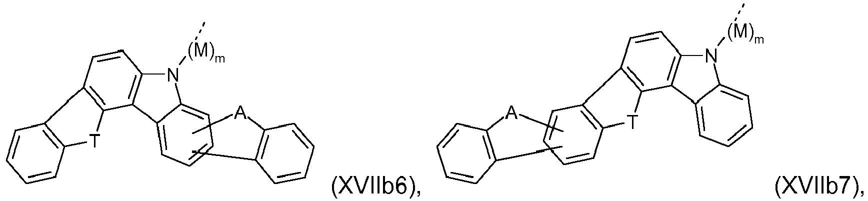 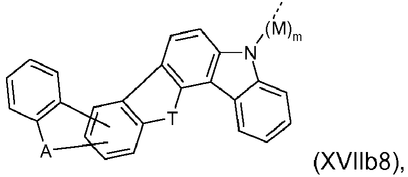 wherein
A is S, O, SiR³²R³³, CR³⁴R³⁵ or NR³⁶, preferably S, O, CR³⁴R³⁵ or NR³⁶, and
Q and T are independently of each other S, O, SiR³²R³³, CR³⁴R³⁵ or NR³⁶, preferably NR³⁶.

According to a particularly preferred embodiment of the present invention, in the compound of formula (XVII), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XVII), Q is direct bond, T is NR³⁶, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XVII), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XVII), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XVII), Q is NR³⁶, T is direct bond, R³⁶ is phenyl, biphenyl or naphthyl, R³⁹, R⁴⁰, R⁴¹ and R⁴² are H, m is 0, n is 0 and R²⁸, R²⁹, R³⁰ and R³¹ are H.

Most preferred compounds of formula (XVII) are compounds of formula (XVII), wherein Q is a direct bond, especially preferred are therefore compounds (XVIIb), more preferably (XVIIb1) to (XVIIb12).

According to a further preferred embodiment, the heteroaryl group according to general formula (XVI) corresponds to general formula (XVII') 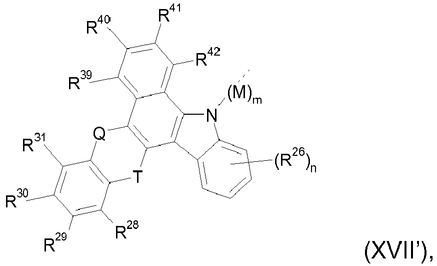
wherein R²⁶, R²⁸, R²⁹, R³⁰, R³¹, M, Q, T, n and m have the same meanings as mentioned above and
R³⁹, R⁴⁰, R⁴¹, R⁴² are independently of each other selected from H, a C₆-C₁₈aryl group which is unsubstituted or substituted by at least one C₆-C₁₂aryl or by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

Preferred compounds of formula (XVII') are compounds of the following formula (XVII'a), wherein T is a direct bond, and compounds of formula (XVII'b), wherein Q is a direct bond: 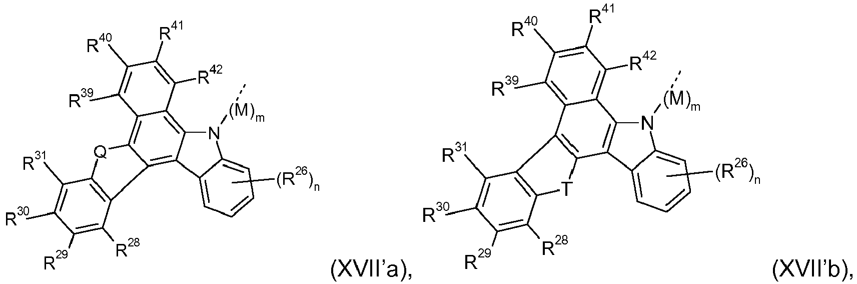 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring.

More preferred compounds of formula (XVII') are the following compounds: 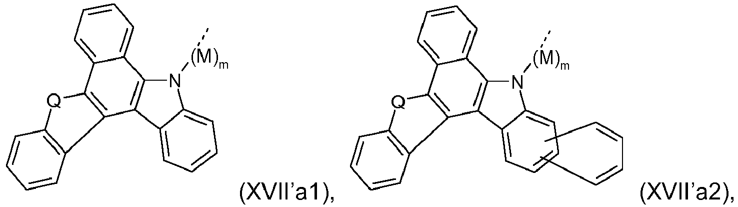 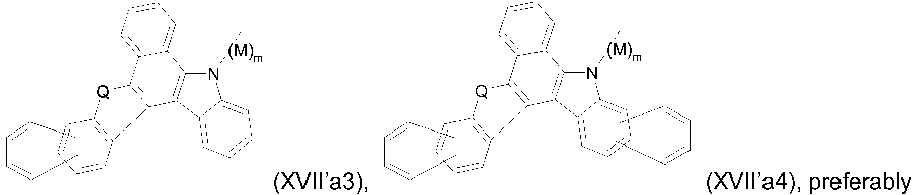 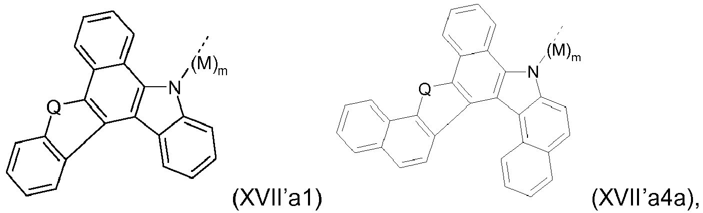 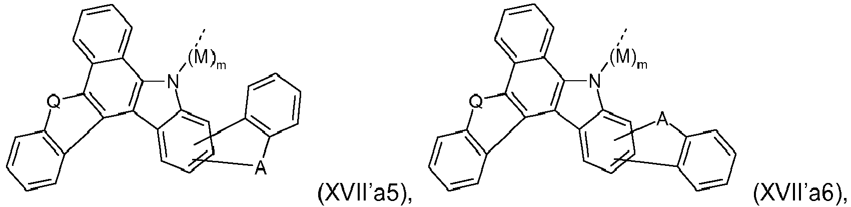 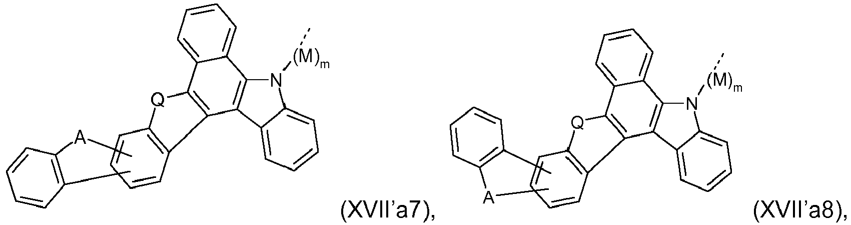 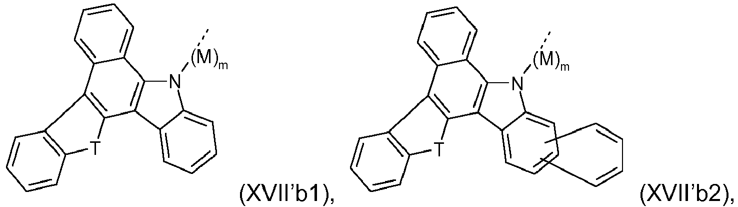 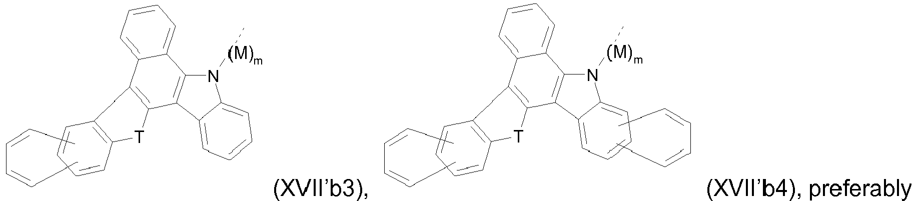 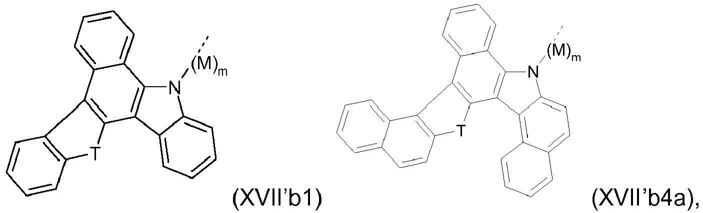 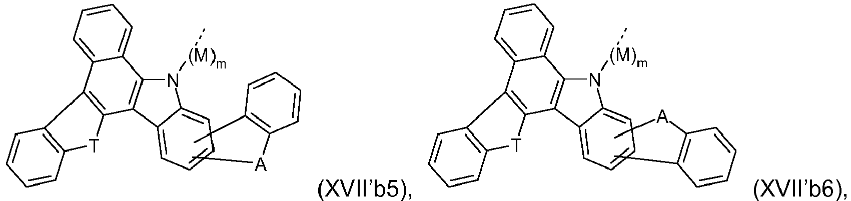 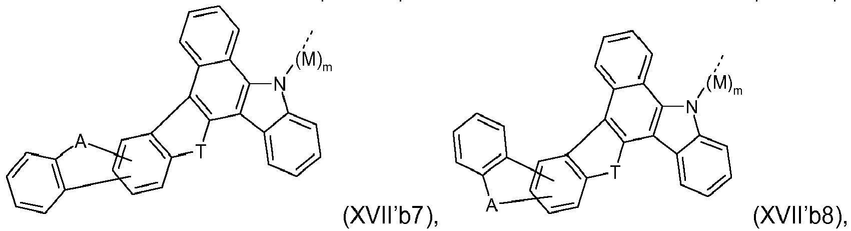 wherein A is S, O, SiR³²R³³, CR³⁴R³⁵ or NR³⁶, preferably S, O, CR³⁴R³⁵ or NR³⁶, and Q and T are independently of each other S, O, SiR³²R³³, CR³⁴R³⁵ or NR³⁶, preferably NR³⁶.

According to a further preferred embodiment of the present invention, R²⁶ in the N-heteroaryl groups according to general formulae (XIII), (XIV), (XV), (XVII) and (XVII') may correspond to the following formula (XVIII) 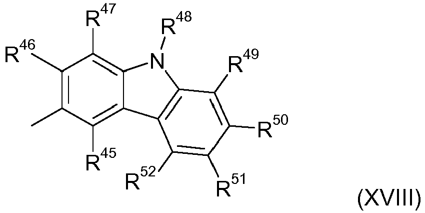
wherein R⁴⁵, R⁴⁶, R⁴⁷, R⁴⁸, R⁴⁹, R⁵⁰, R⁵¹ and R⁵² are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
at least two of R⁴⁶, R⁴⁷, R⁴⁹, R⁵⁰, R⁵¹ or R⁵², if present at adjacent carbon atoms, together form at least one C₆-C₁₈aryl or C₁-C₁₈heteroaryl ring or ring system,
wherein E and D have the same meanings as mentioned above.

Particularly preferred R⁴⁵, R⁴⁶, R⁴⁷, R⁴⁹, R⁵⁰, R⁵¹ and R⁵² are independently of each other H, E, a unsubstituted C₆-C₁₈aryl group or a C₆-C₁₈aryl group substituted with at least one group E, or a

C₁-C₂₅alkyl group, which is unsubstituted or substituted by at least one group E and/or interrupted by at least one group D, preferably H, phenyl, biphenyl, naphthyl, phenanthryl or dimethylfluorenyl.

R⁴⁸ is particularly preferred H, a unsubstituted C₆-C₁₈aryl group or a C₆-C₁₈aryl group substituted with at least one group E, preferably phenyl, biphenhyl or naphthyl.

Further preferred at least two of R⁴⁶, R⁴⁷, R⁴⁹, R⁵⁰, R⁵¹ or R⁵², most preferably R⁵¹ and R⁵² if present at adjacent carbon atoms, together form at least one C₆-C₁₈aryl or C₁-C₁₈heteroaryl ring or ring system, most preferably a fused phenyl ring.

According to a particularly preferred embodiment, in general formula (XVIII) R⁴⁵, R⁴⁶, R⁴⁷, R⁴⁹, R⁵⁰, R⁵¹ and R⁵² are H and R⁴⁸ is phenyl.

According to a further particularly preferred embodiment, in general formula (XVIII) R⁴⁵, R⁴⁶, R⁴⁷, R⁴⁹ and R⁵⁰ are H, R⁵¹ and R⁵² form a fused phenyl ring and R⁴⁸ is phenyl.

### N-heteroaryl groups according to general formula (XXIV)

In one embodiment of the present invention, the N-heteroaryl group is represented by the general formula (XXIV) 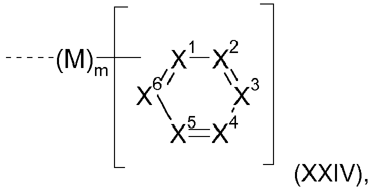 wherein the groups X¹, X², X³, X⁴, X⁵ and X⁶ and M and m are defined above and the dotted line is a bonding site.

Preferred groups of formula (XXIV) are: 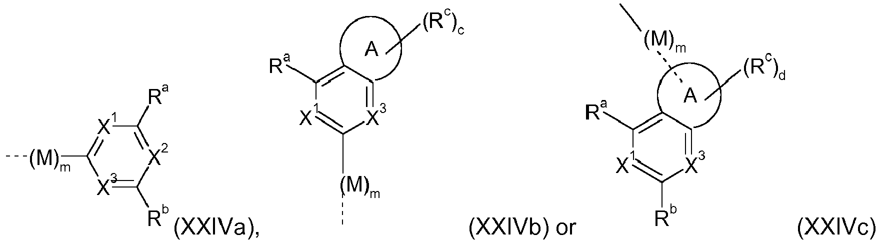 wherein
X¹, X² and X³ are independently of each other CR^{c} or N, wherein in formula (XXIVa) at least one of X¹, X² and X³ is N, and wherein in formulae (XXIVb) and (XXIVc) at least one of X¹ and X³ is N;
R^{a}, R^{b} and R^{c} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{c}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, carbocyclic or heterocyclic, aromatic or heteroaromatic ring may be fused;
the ring A is a five or six membered substituted or unsubstituted, saturated, unsaturated, carbocyclic or heterocyclic, aromatic or heteroaromatic ring, and which may be fused with a further five or six membered substituted or unsubstituted, saturated, unsaturated, carbocyclic or heterocyclic, aromatic or heteroaromatic ring;
c is 0, 1, 2, 3 or 4;
d is 0, 1, 2 or 3;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites.

Examples for suitable N-heteroaryl groups represented by the general formula (XXIV) are: 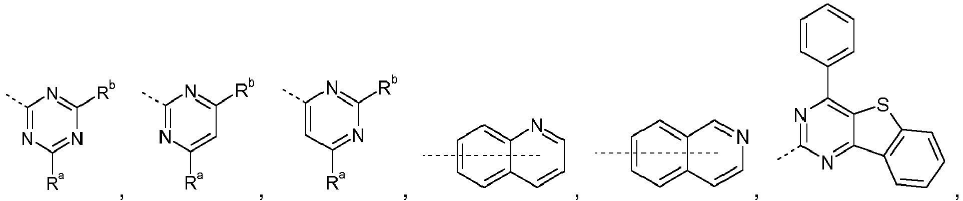 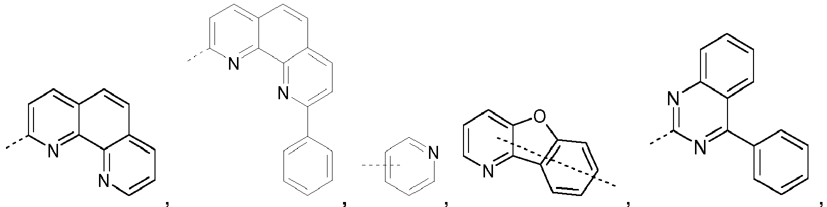 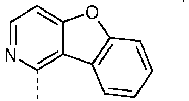 wherein
R^{a} and R^{b} are independently of each other H, a C₆-C₁₈aryl group which is unsubstituted or substituted by at least one C₁-C₁₈alkyl group, by at least one C₆-C₁₈aryl group and/or by at least one C₁-C₁₈alkoxy group, preferably H or an unsubstituted or phenyl substituted phenyl group.

### N-heteroaryl groups according to general formula (XXV)

In one embodiment of the present invention, the N-heteroaryl group is represented by the general formula (XXV) 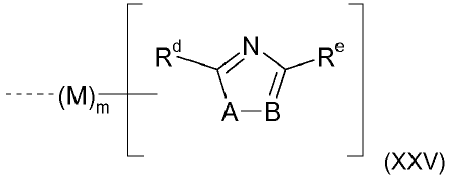 wherein the groups A, B, R^{d} and R^{e} and M and m are defined above and the dotted line is a bonding site.

Preferred groups of formula (XXV) are: 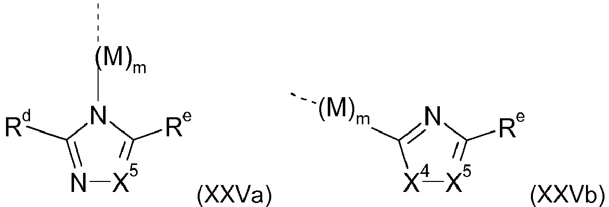 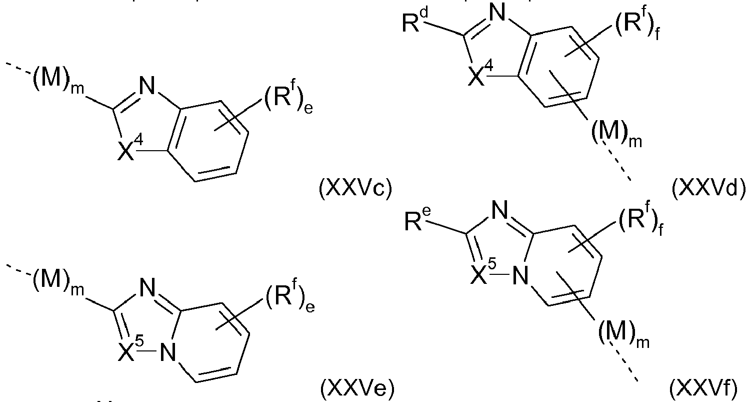 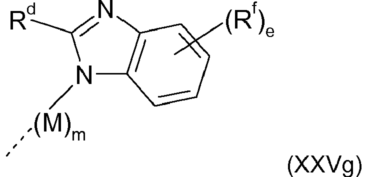 wherein
X⁵ is CR⁹ or N;
X⁴ is O, S or NR^{h};
R^{d}, R^{e}, R^{f}, R^{g} and R^{h} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{f}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
or R^{h} and R^{d} may form at least one five or six membered, substituted or unsubstituted, saturated, unsaturated, carbocyclic or heterocyclic, aromatic or heteroaromatic ring, to which at least one further substituted or unsubstituted, saturated, unsaturated, aromatic or heteroaromatic ring may be fused,
e is 0, 1, 2, 3 or 4;
f is 0, 1, 2 or 3;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites.

Examples for suitable N-heteroaryl groups represented by the general formula (XXV) are:

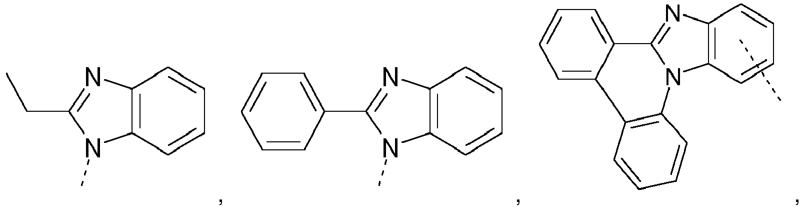

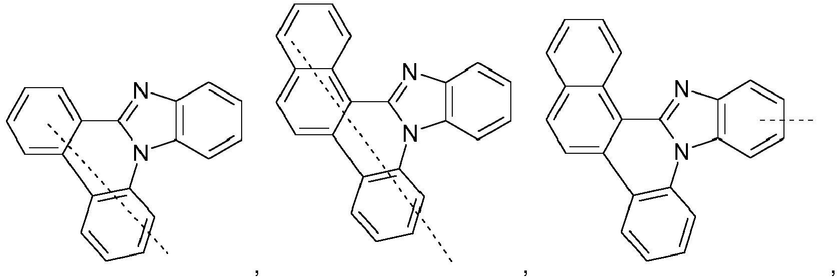

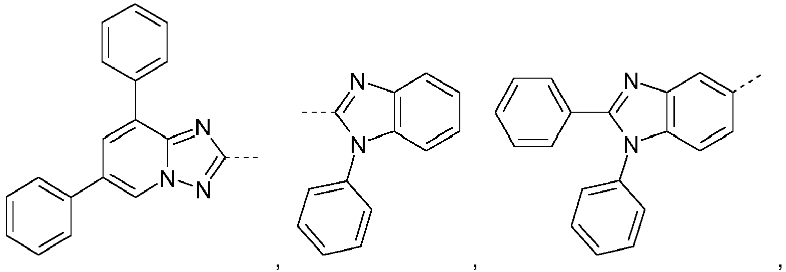

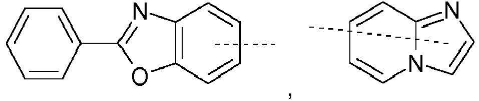

### Aryl groups according to general formulae (XXXIV), (XXXV), (XXXVI), (XXXVII), (XXXVIII) or (XXXIX)

In one embodiment of the present invention, the aryl group is represented by the general formulae (XXXIV), (XXXV), (XXXVI), (XXXVII), (XXXVIII) or (XXXIX) 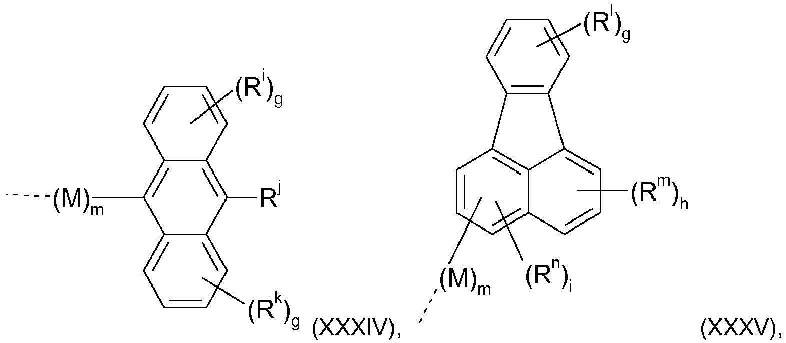 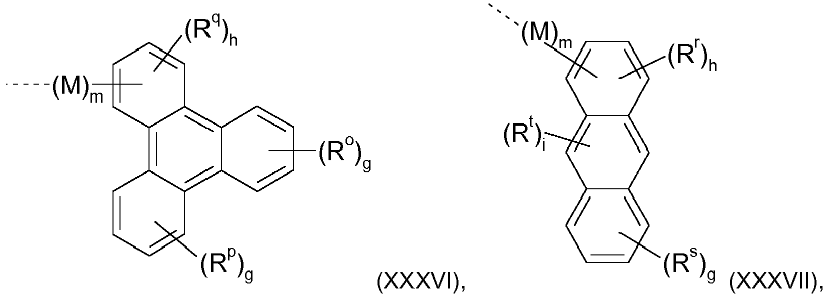 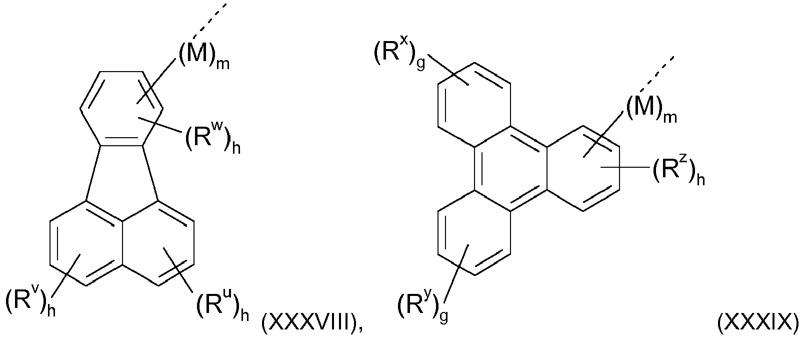 wherein
Rⁱ, R^{j}, R^{k}, R^{l}, R^{m}, Rⁿ, R^{o}, R^{p}, R^{q}, R^{s}, R^{t}, R^{u}, R^{v}, R^{w}, R^{x}, R^{y} and R^{z} are independently of each other H or a C₆-C₁₈aryl group which is unsubstituted or substituted by at least one C₁-C₁₈alkyl group or by at least one C₁-C₁₈alkoxy group,
g is 0, 1 or 2;
h is 0, 1 or 2,
i is 0, 1 or 2;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites.

Examples for suitable aryl groups represented by the general formulae (XXXIV), (XXXV), (XXXVI), (XXXVII), (XXXVIII) or (XXXIX) are: 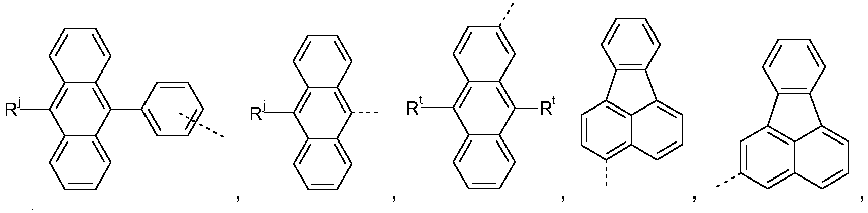 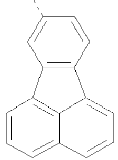 wherein
R^{j} and R^{t} are independently of each a C₆-C₁₈aryl group which is unsubstituted or substituted by at least one C₁-C₁₈alkyl group or by at least one C₁-C₁₈alkoxy group.

*In formulae (XII), (XIII), (XV), (XVII), (XXI), (XXIV) or (XXV) or a C₁-C₄₀aryl group of the following formulae (XXXIV), (XXXV), (XXXVI), (XXXVII), (XXXVIII) or (XXXIX) and their preferred embodiments mentioned above, the following is preferred:*

In general, m is 1, 2, 3 or 4, wherein m describes the number of groups M present. If m is 0, no group M is present, but at least one of the N-heteroaryl groups respectively the aryl groups mentioned above are directly attached to carbon atom within the skeleton of the compound of general formula (I). Preferably, m is 0, 1, 2 or 3, more preferably, m is 0 or 1.

Preferably, M is a C₆-C₄₀ arylene group which is unsubstituted, a C₁-C₂₄heteroarylene group which is unsubstituted or a C₁-C₂₅alkylene group which is unsubstituted. Particularly preferred, M is a C₆-C₁₈ arylene group which is unsubstituted, most preferably a phenylene group or a biphenylene group, for example: 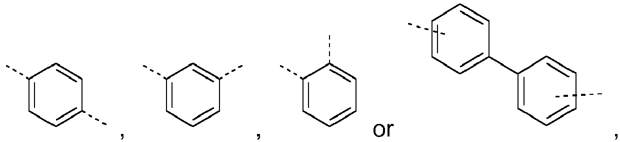 for example 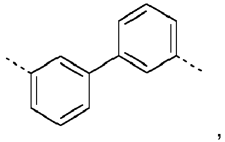 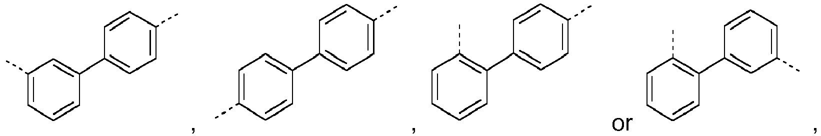 wherein the dotted lines are bonding sites.

Preferably, R²⁶ in the formulae mentioned above is independently of each other selected from H, a C₆-C₄₀aryl group which is unsubstituted or substituted by at least one group E and a C₁-C₃₀heteroaryl group which is unsubstituted or substituted by at least one group E.

The present invention therefore preferably relates to compounds according to formula (I), preferably (Ia), more preferably (Ia1) as mentioned above, wherein R¹, R², R³, R⁴, R⁵, R⁶, R⁷, R⁸ and R9
are independently of each other H, -CN, a C₁-C₂₅alkyl group which is unsubstituted or substituted by at least one group E and/or interrupted by at least one group D, a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, preferably H,
wherein at least one of X¹, X², X³, X⁴, X⁵, X⁶, X⁷, X⁸ and X⁹ is CR¹, CR², CR³, CR⁴, CR⁵, CR⁶, CR⁷, CR⁸ or CR⁹, wherein R¹, R², R³, R⁴, R⁵, R⁶, R⁷, R⁸ or R⁹, preferably one or both of R¹ and R⁸ or one or both of R¹ and R⁷, is -CN, a C₁-C₂₅alkyl group which is unsubstituted or substituted by at least one group E and/or interrupted by at least one group D, a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen.

More preferably in the compounds according to formula (I), preferably (Ia), more preferably (Ia1) as mentioned above, R¹, R², R³, R⁴, R⁵, R⁶, R⁷, R⁸ and R⁹
are independently of each other H or a group of formula (XII), (XIII), (XV), (XVII), (XXI), (XXIV) or (XXV) (XXXIV), (XXXV), (XXXVI), (XXXVII), (XXXVIII) or (XXXIX);
wherein at least one of R¹, R², R³, R⁴, R⁵, R⁶, R⁷, R⁸ and R⁹ is a group of formula (XII), (XIII), (XV), (XVII), (XXI), (XXIV) or (XXV) (XXXIV), (XXXV), (XXXVI), (XXXVII), (XXXVIII) or (XXXIX).

In a further preferred embodiment, in the compounds according to formula (I), preferably (Ia), more preferably (Ia1) as mentioned above, R³, R⁴, R⁵, R⁶, R⁷ and R⁹ are H, and one or both of R¹ and R⁸ are independently of each other a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E, preferably a group of formula (XII), (XIII), (XV), (XVII), (XXI), (XXIV) or (XXV) (XXXIV), (XXXV), (XXXVI), (XXXVII), (XXXVIII) or (XXXIX); and the other of R¹ and R⁸ which is not a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E is H or
R³, R⁴, R⁵, R⁶, R⁸ and R⁹ are H, and one or both of R¹ and R⁷ are independently of each other a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E, preferably a group of formula (XII), (XIII), (XV), (XVII), (XXI), (XXIV) or (XXV) (XXXIV), (XXXV), (XXXVI), (XXXVII), (XXXVIII) or (XXXIX) and the other of R¹ and R⁷ which is not a group of formula (XII), (XIII), (XV), (XVII), (XXI), (XXIV) or (XXV) (XXXIV), (XXXV), (XXXVI), (XXXVII), (XXXVIII) or (XXXIX), a C₇-C₂₅aralkyl group which is unsubstituted or substituted by at least one group E, a C₅-C₁₂cycloalkyl group which is unsubstituted or substituted by at least one group E is H.

Particularly preferred molecules according to general formula (I) according to the present invention are shown in the following.

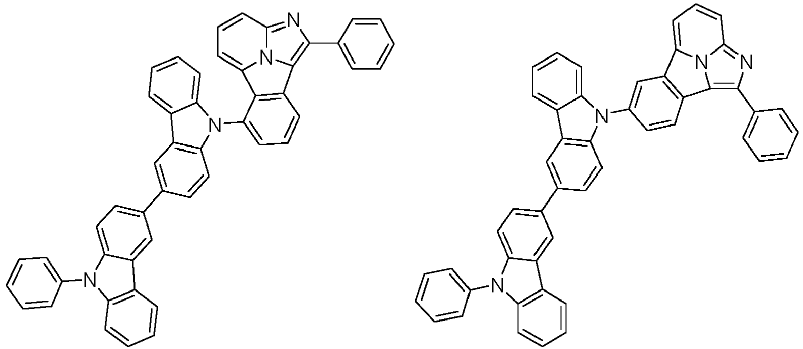

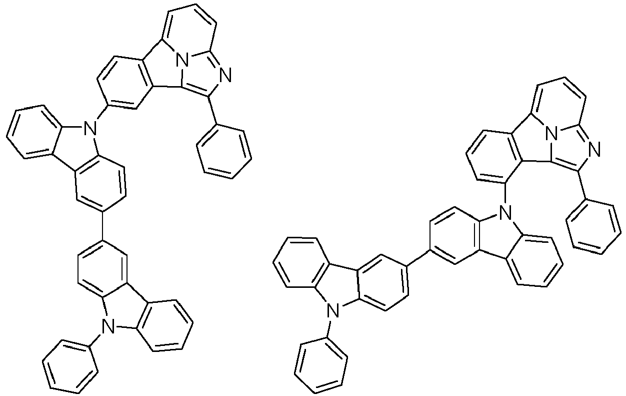

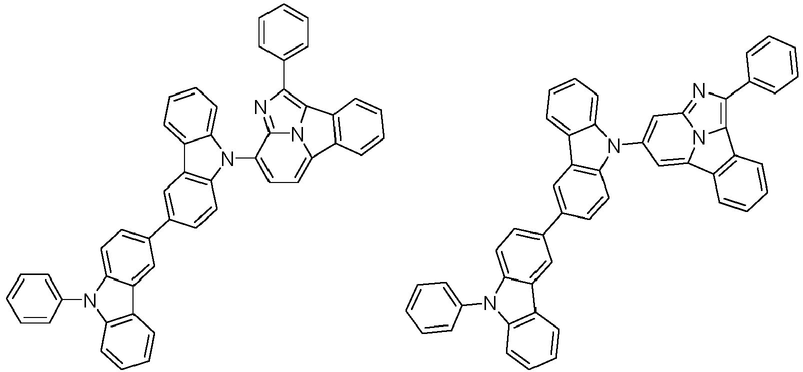

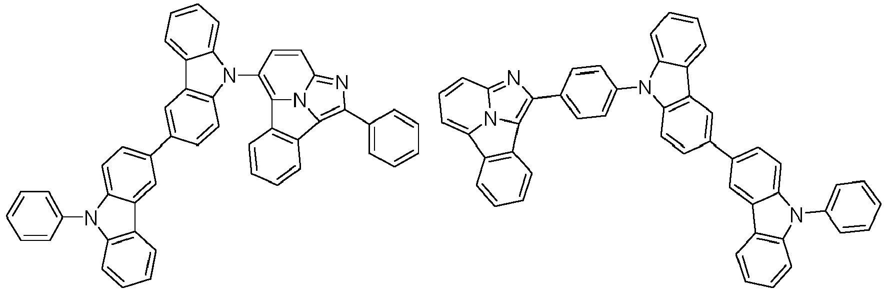

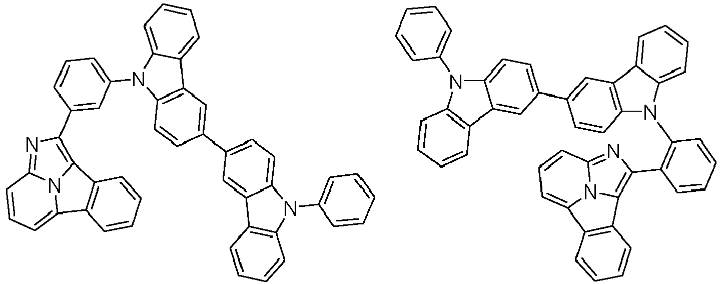

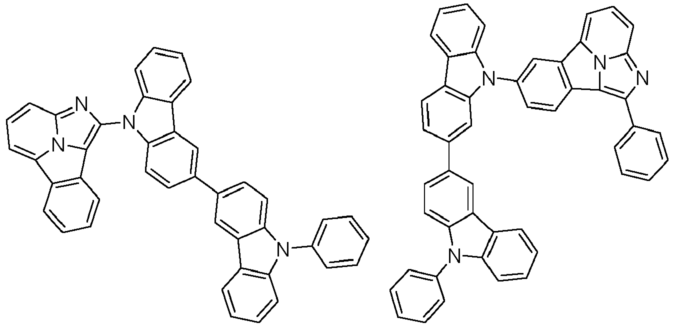

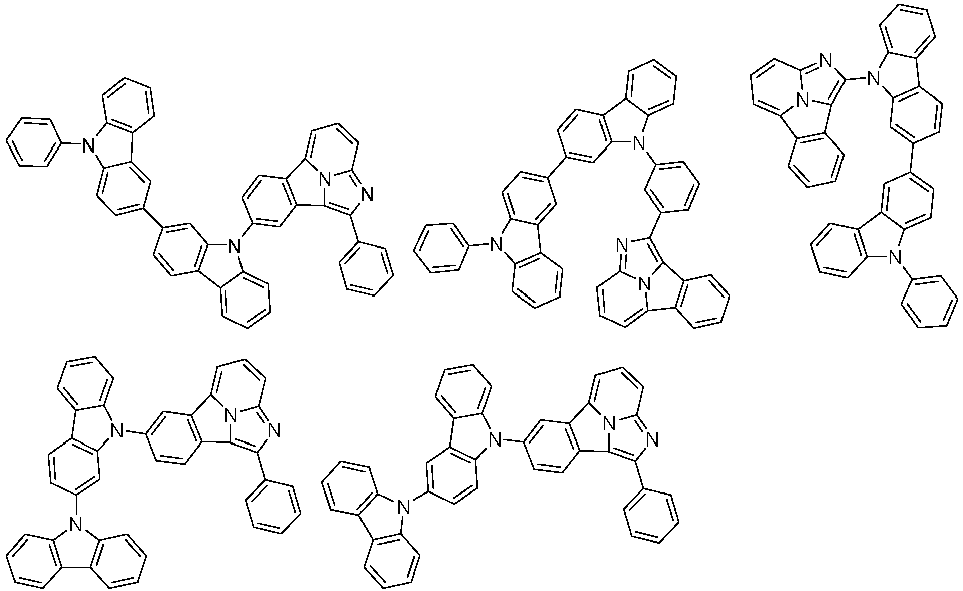

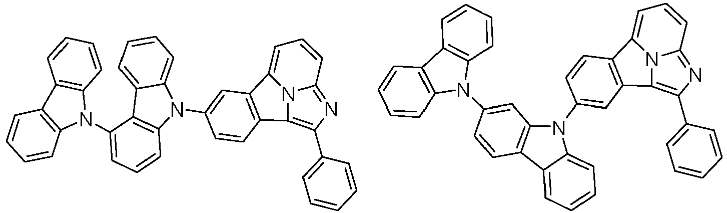

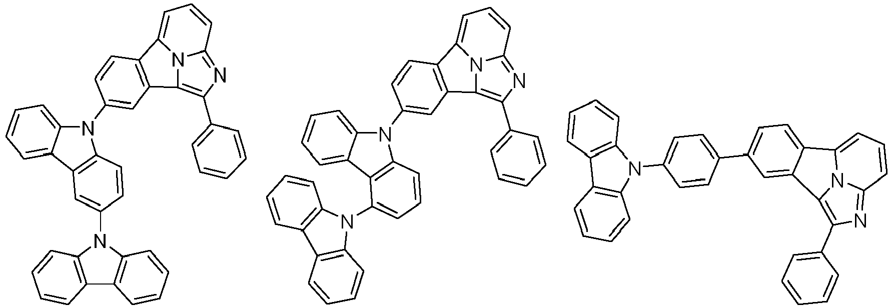

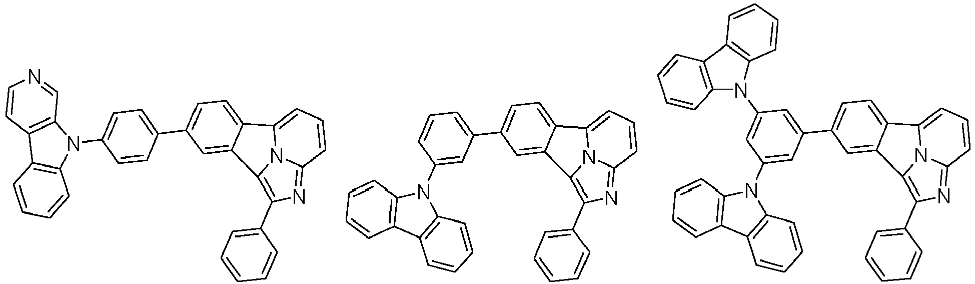

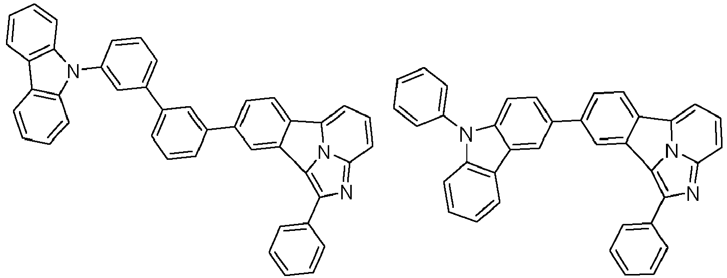

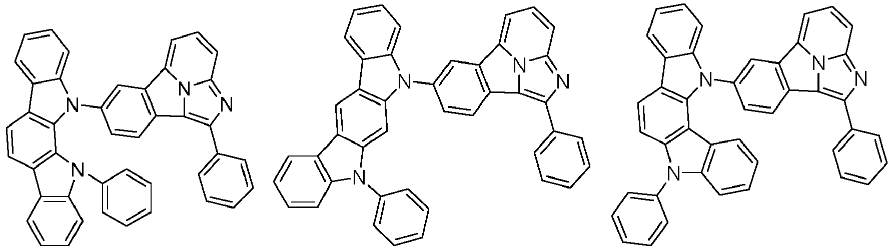

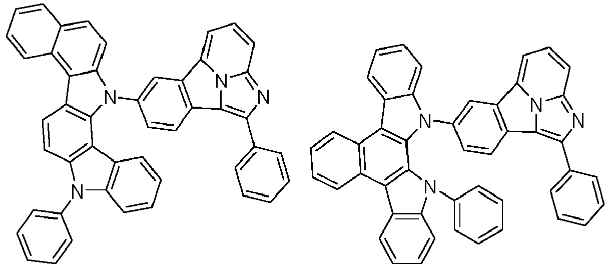

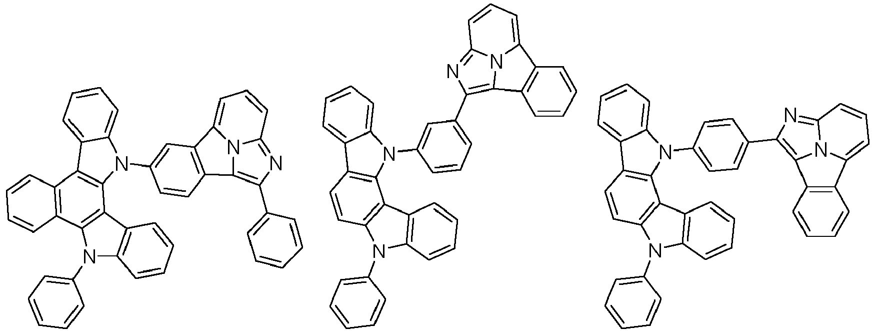

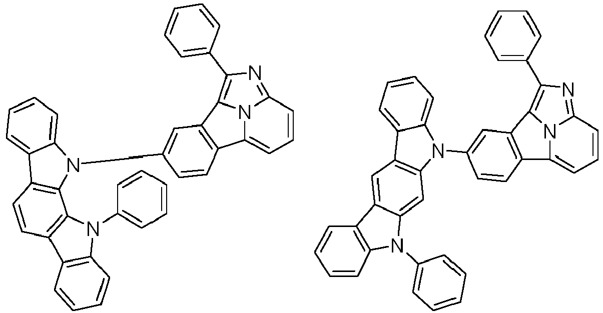

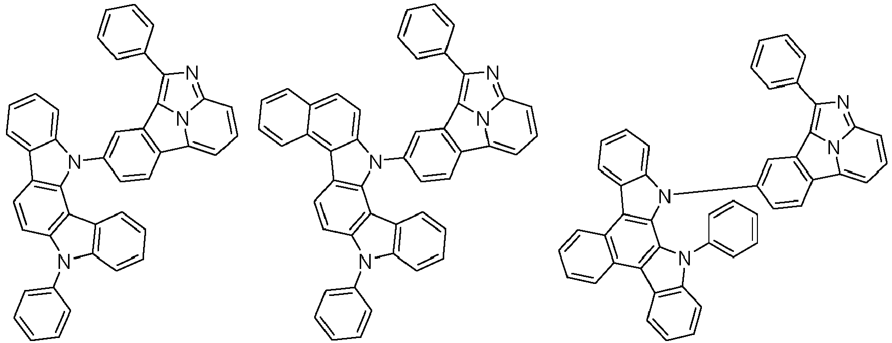

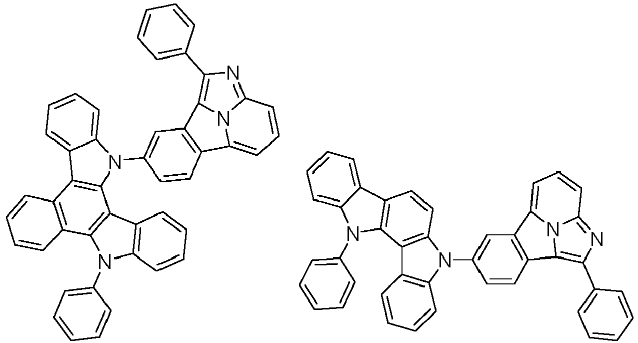

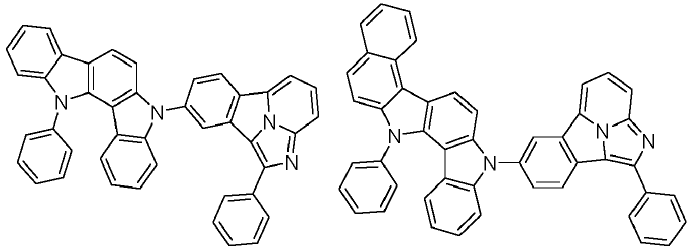

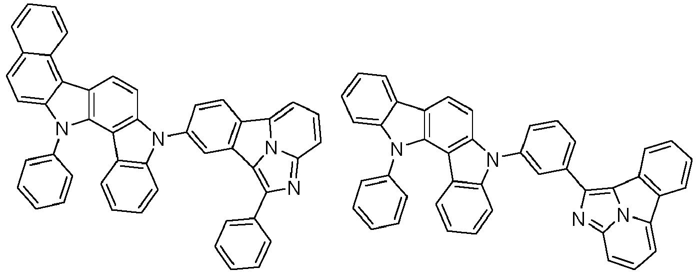

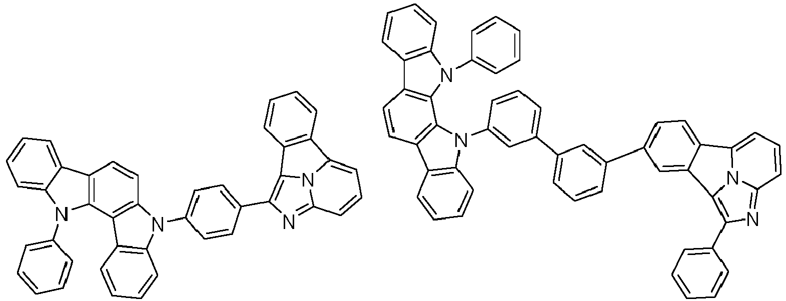

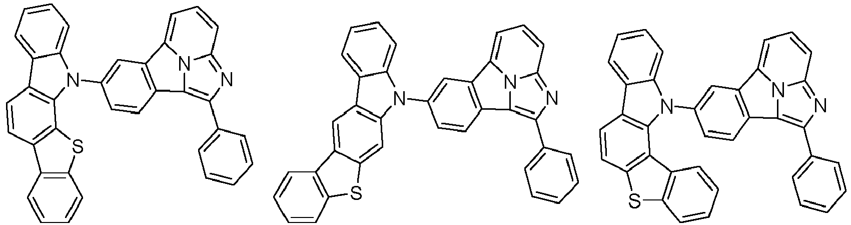

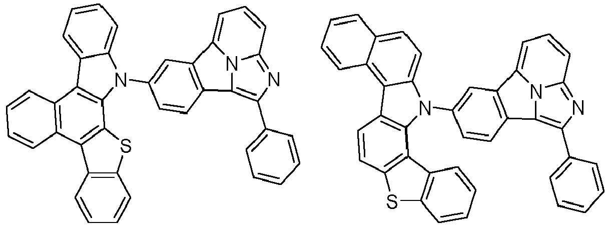

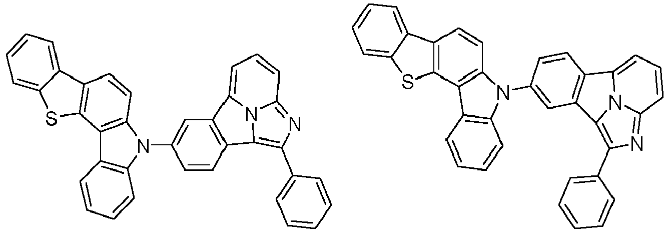

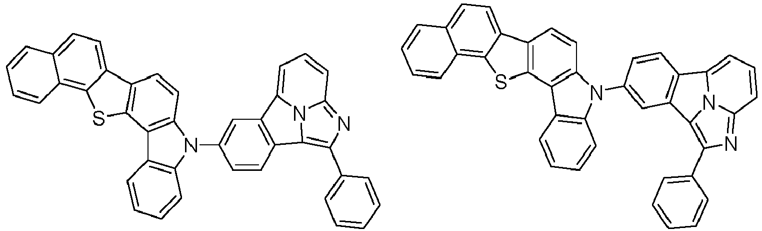

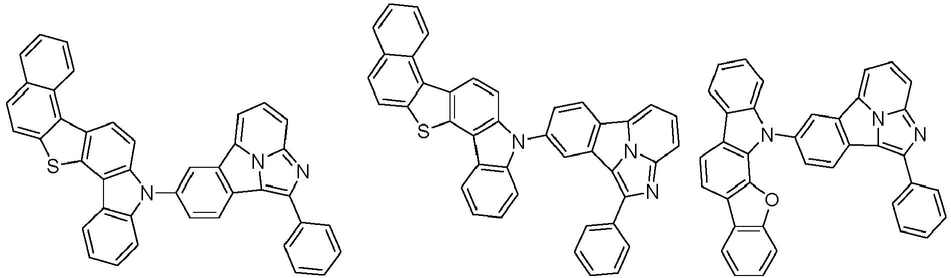

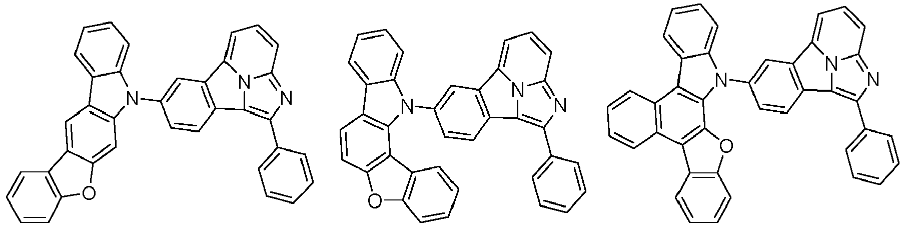

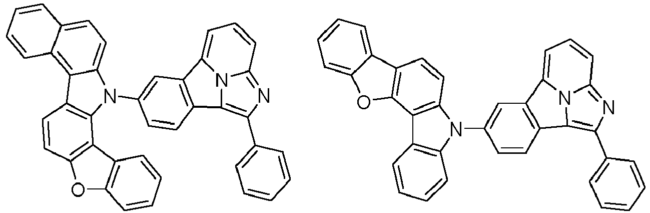

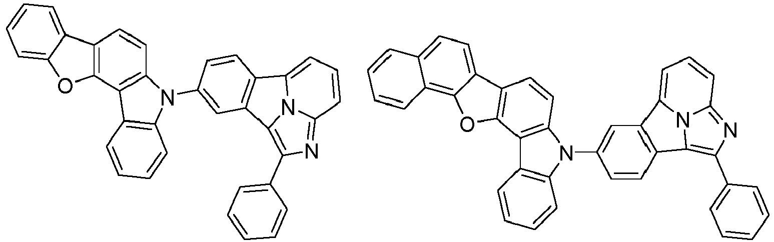

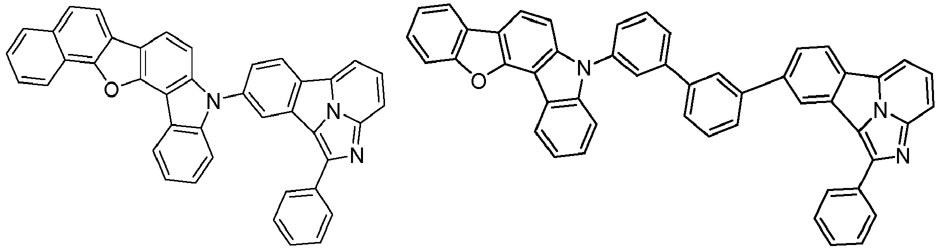

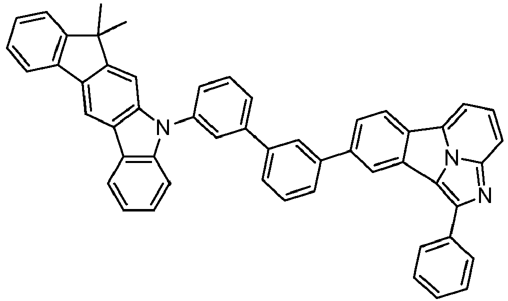

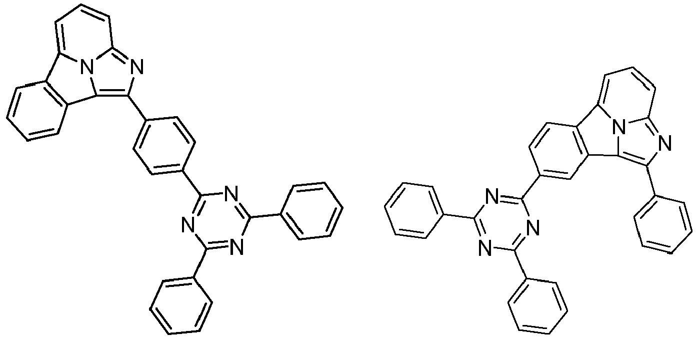

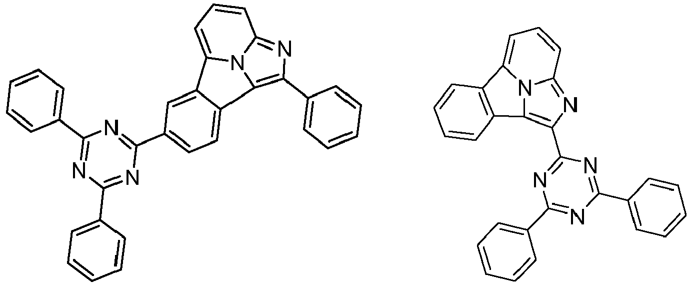

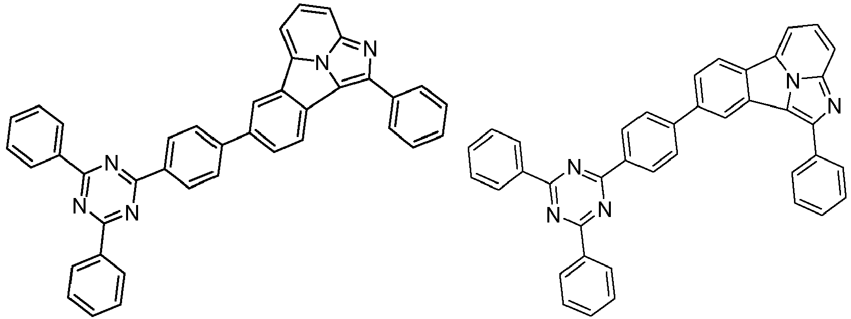

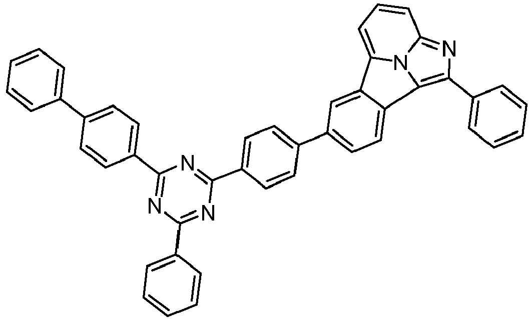

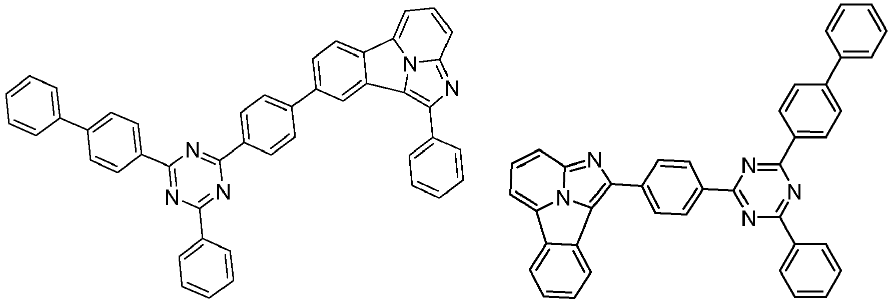

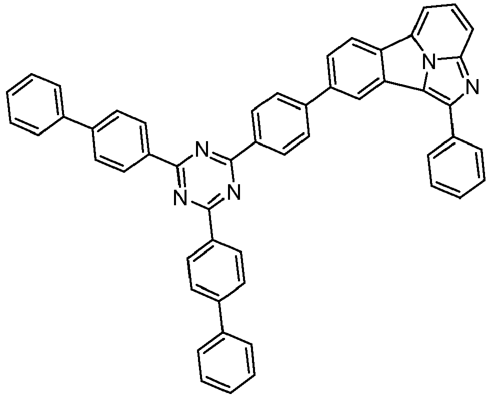

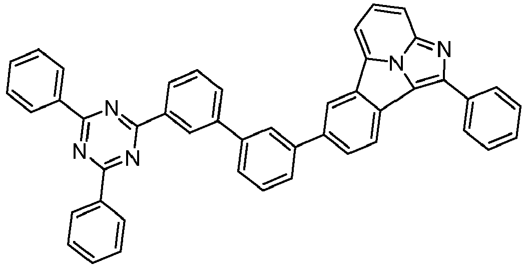

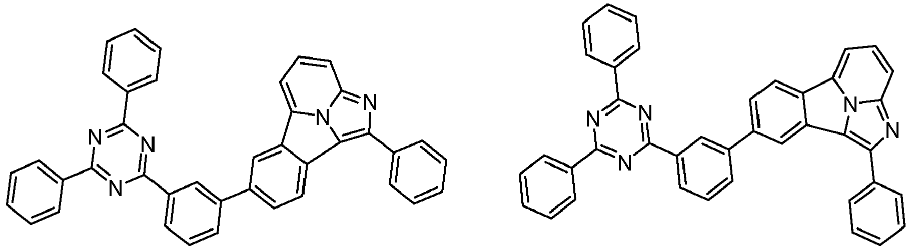

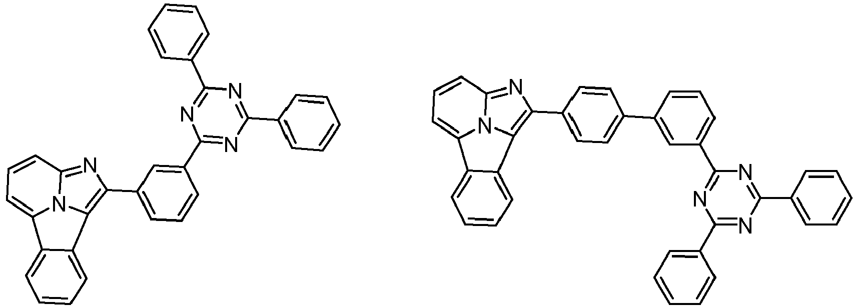

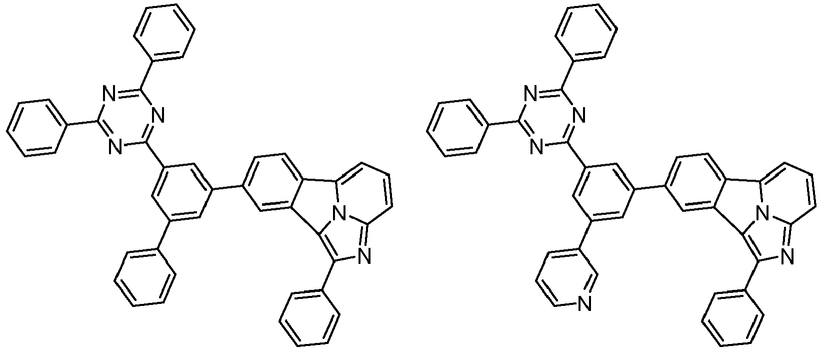

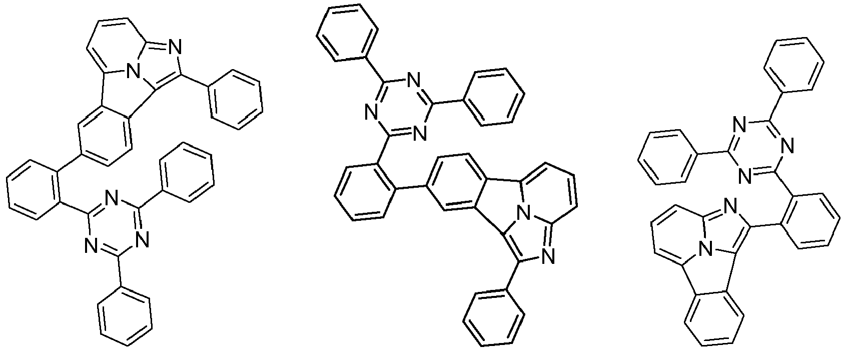

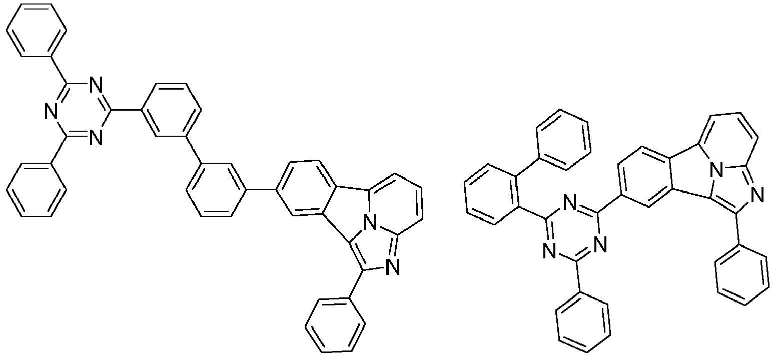

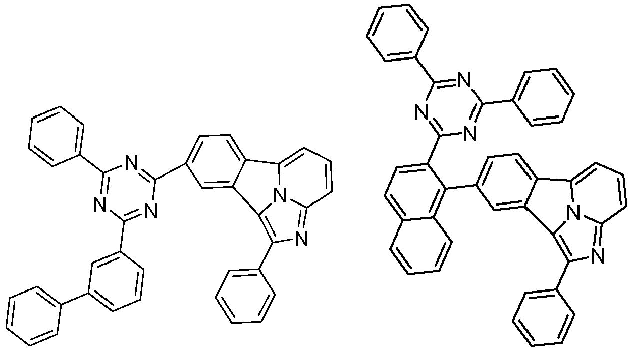

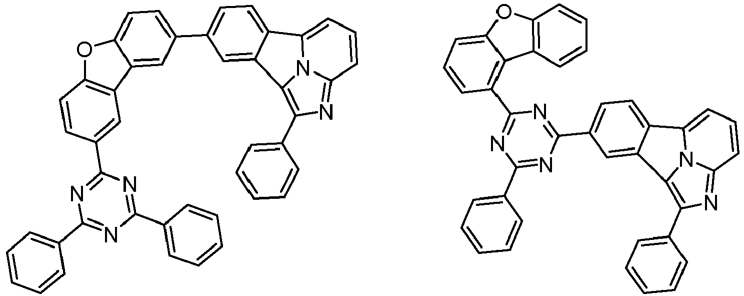

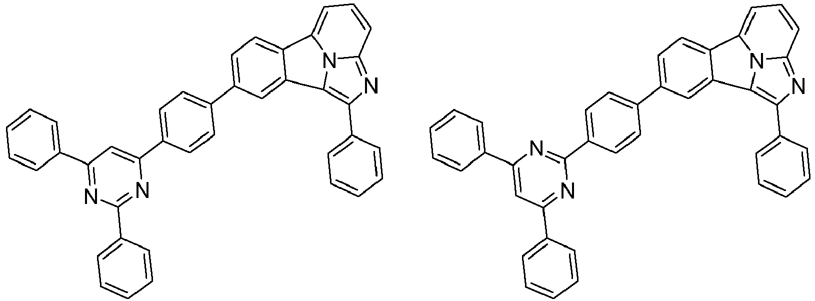

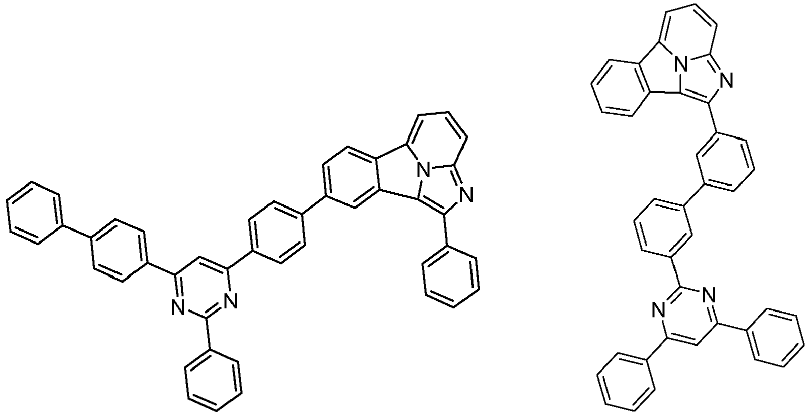

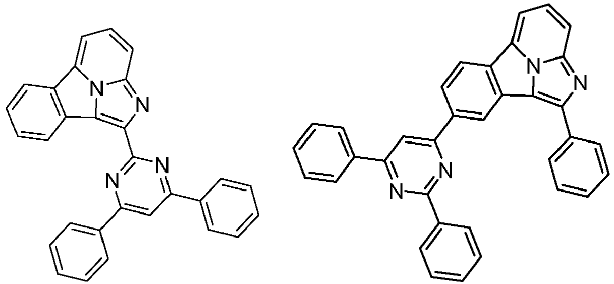

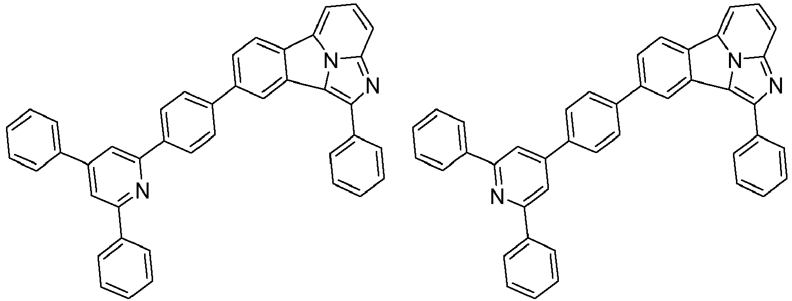

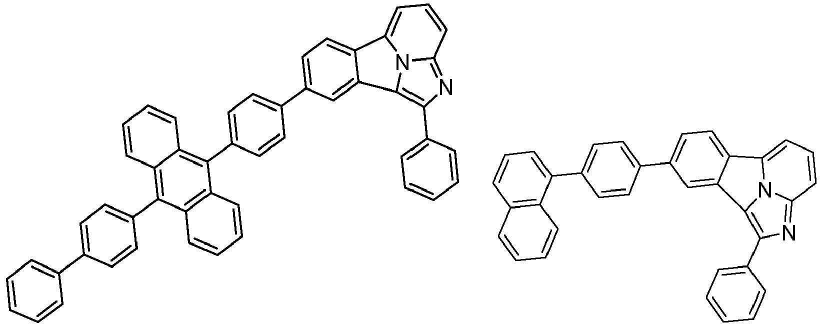

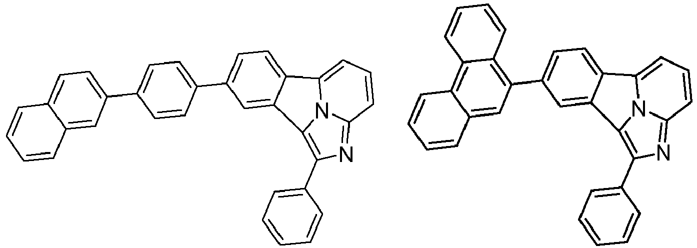

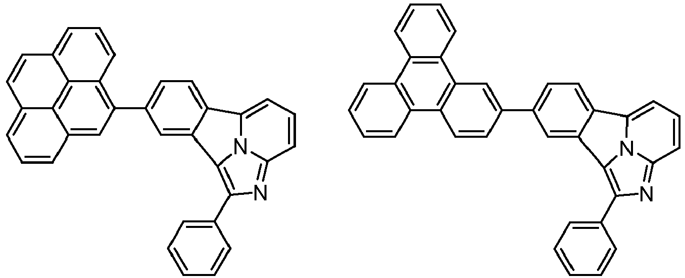

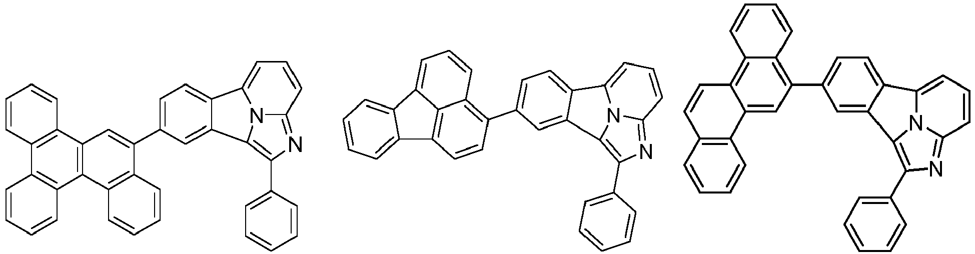

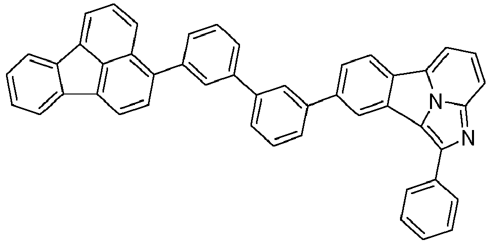

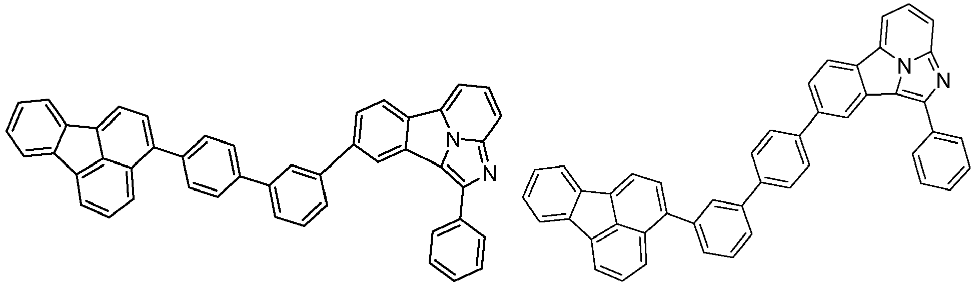

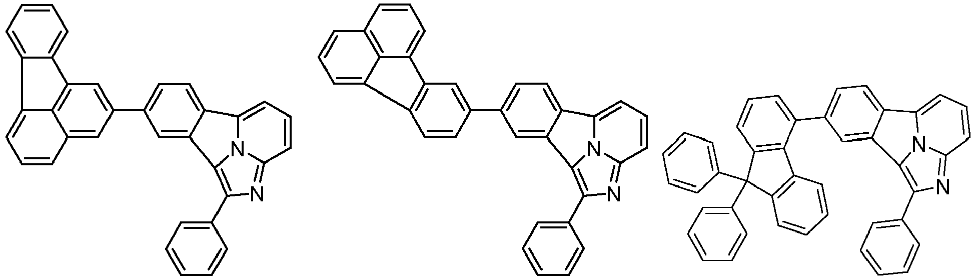

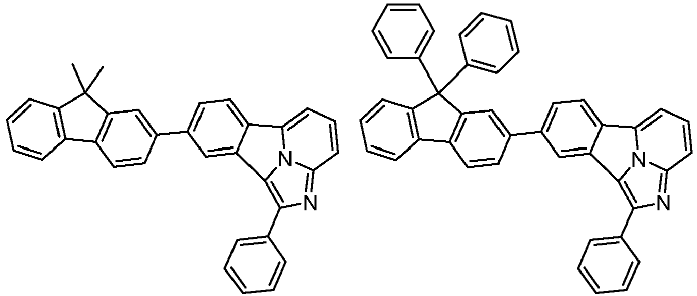

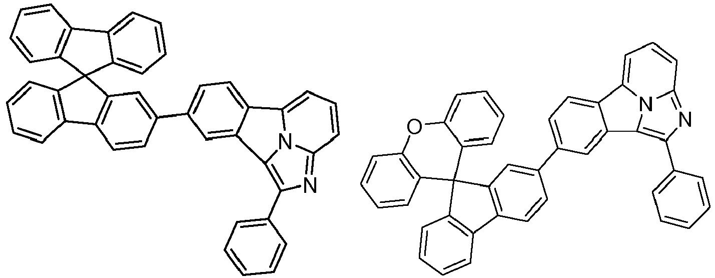

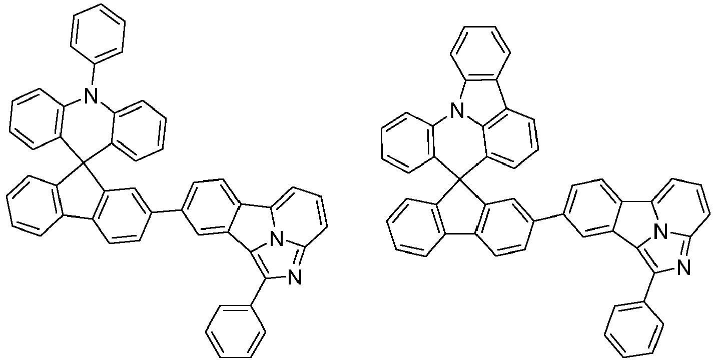

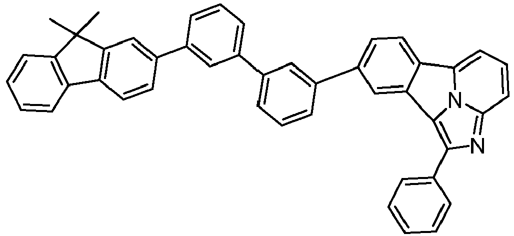

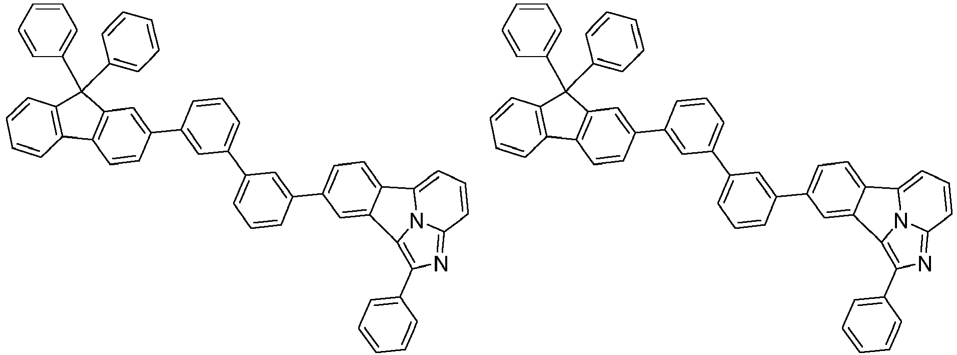

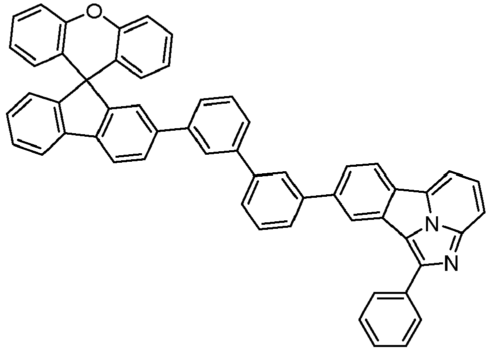

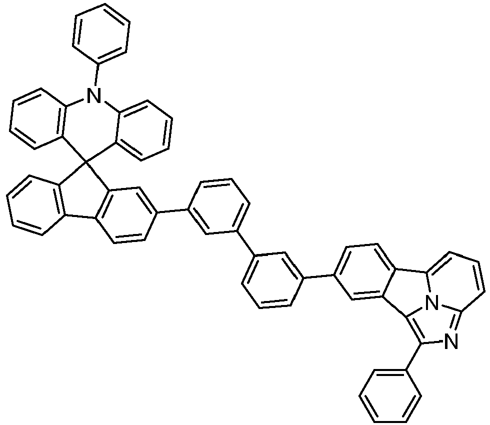

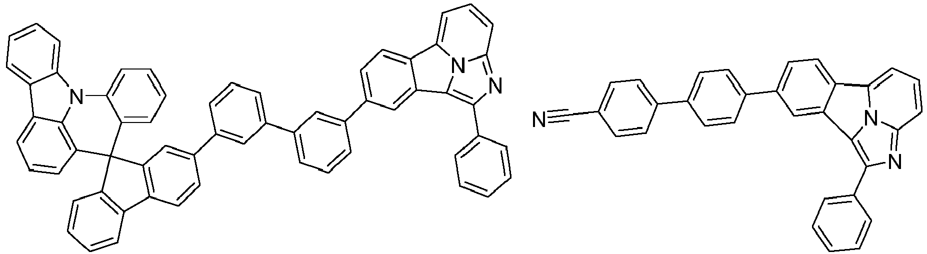

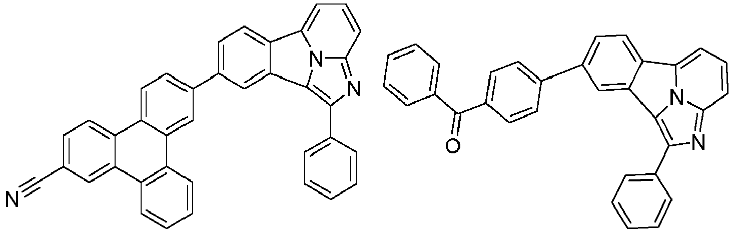

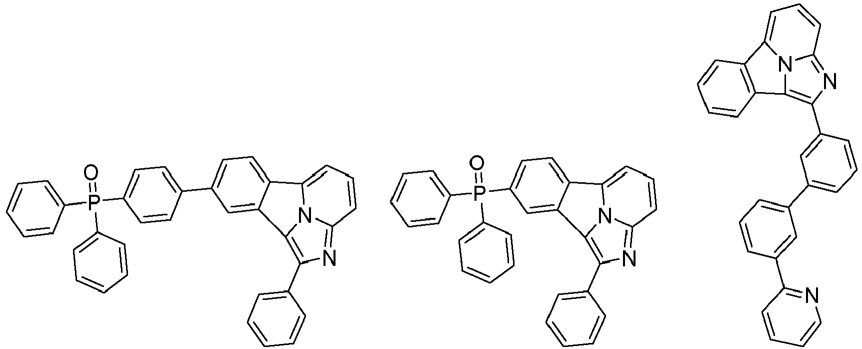

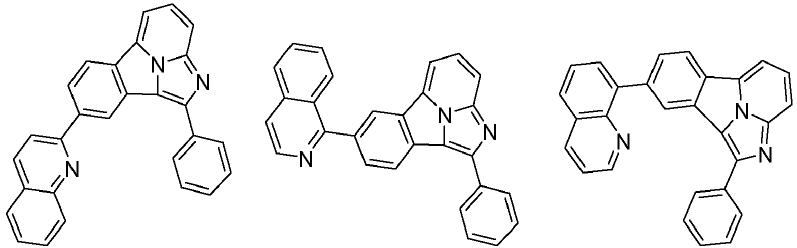

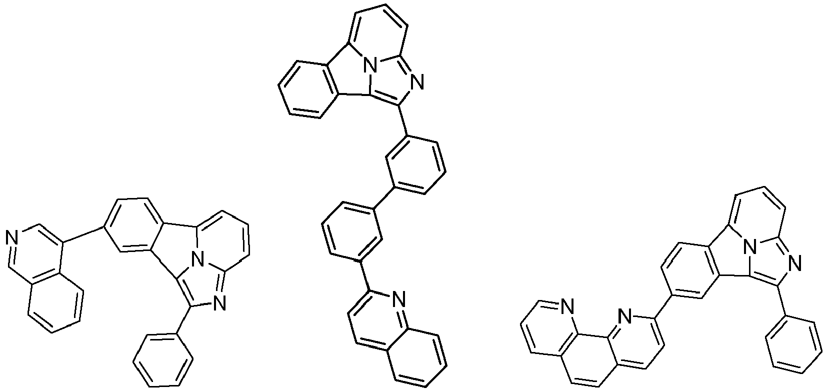

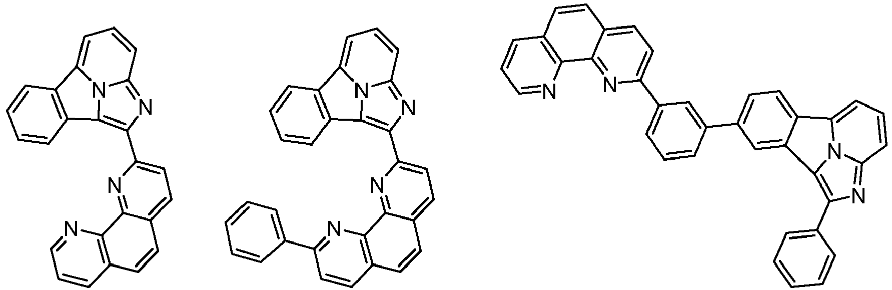

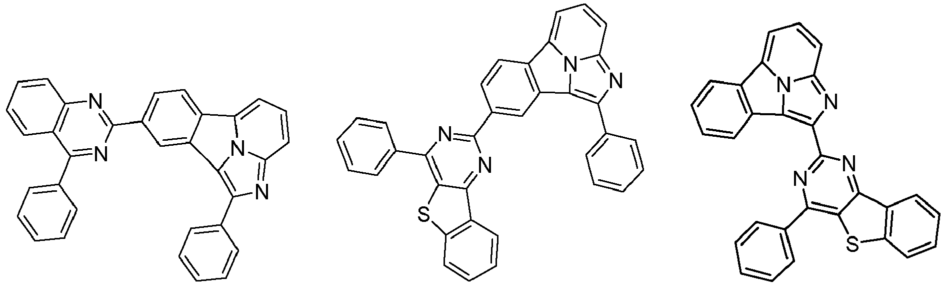

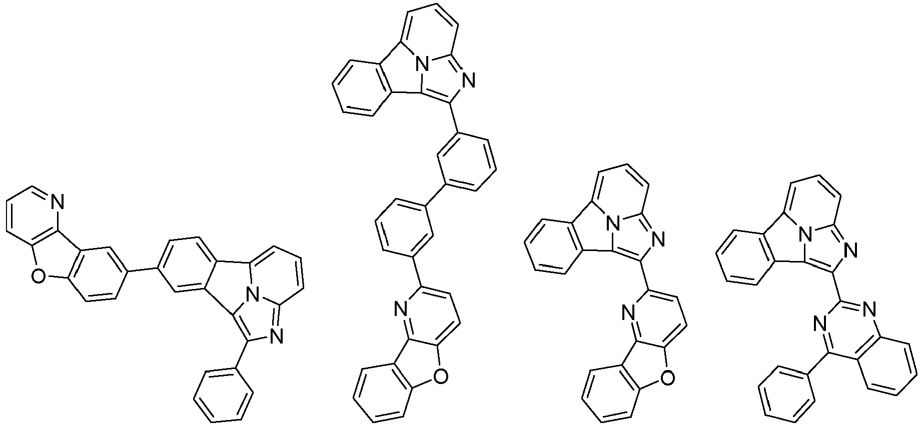

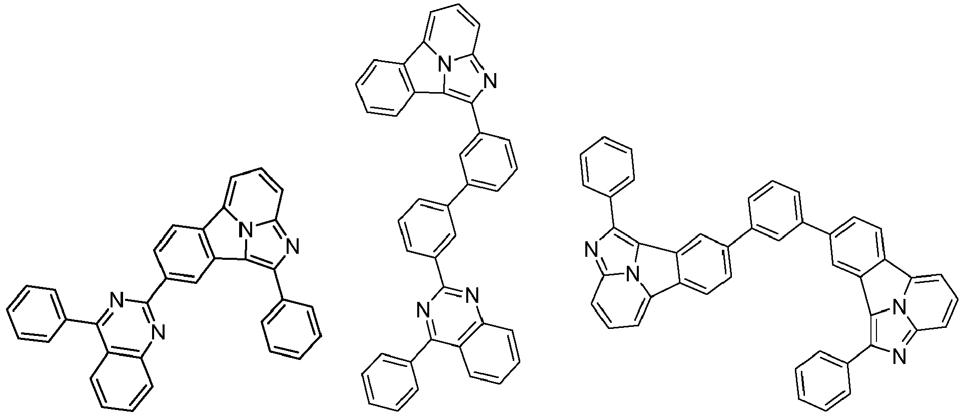

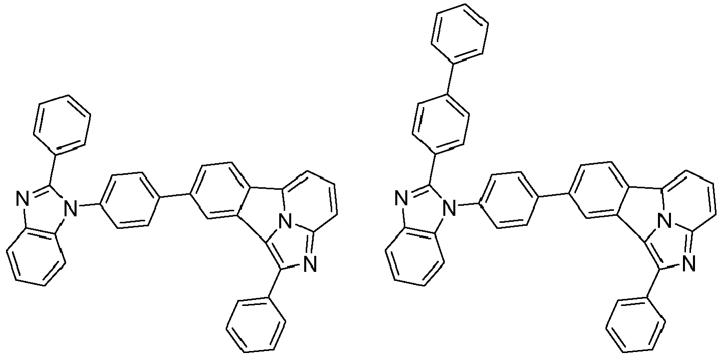

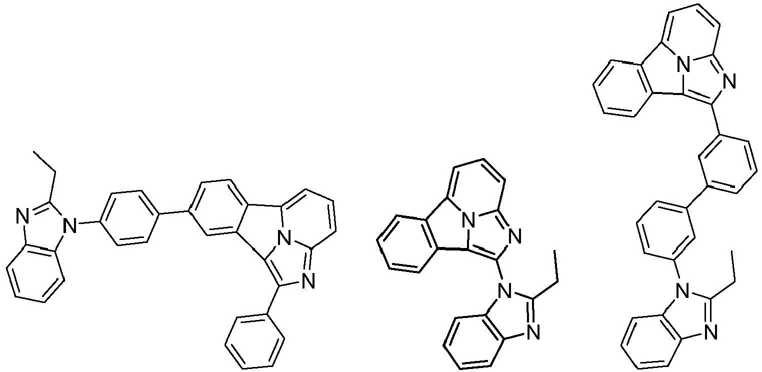

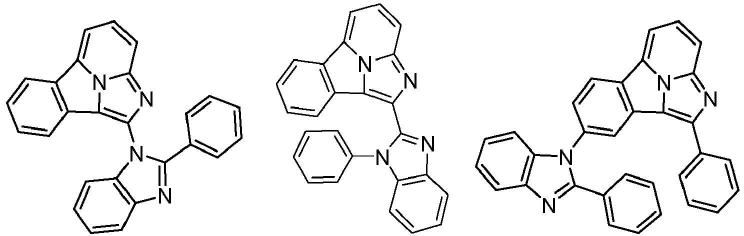

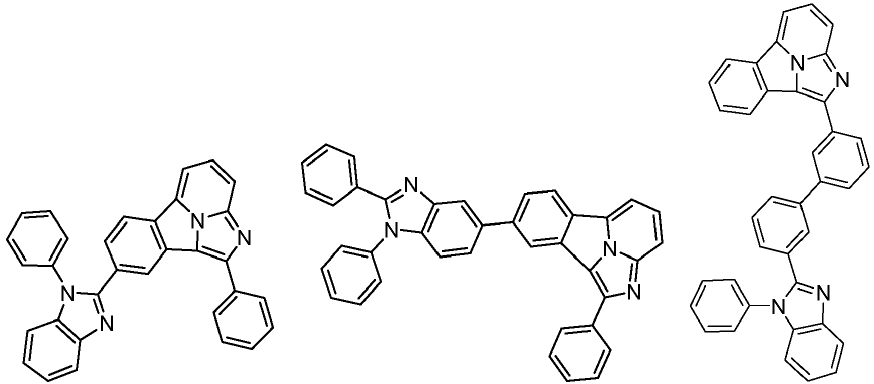

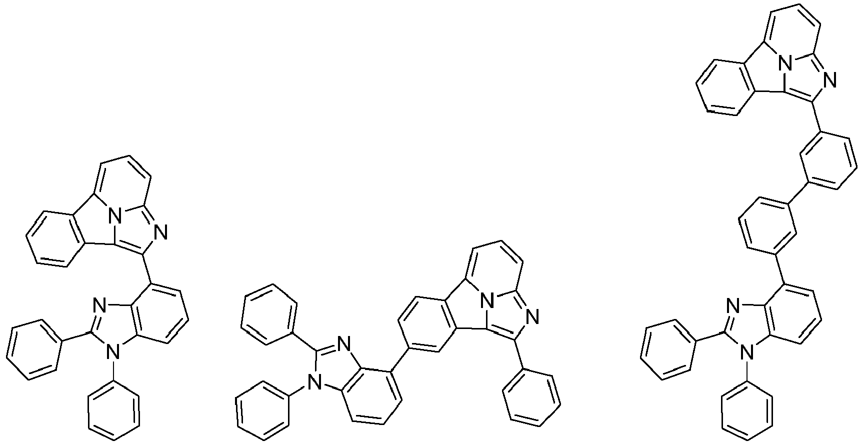

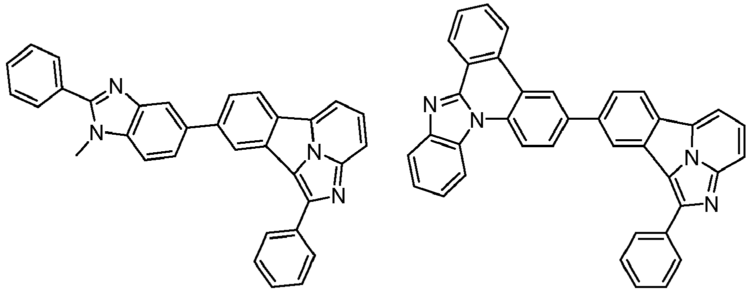

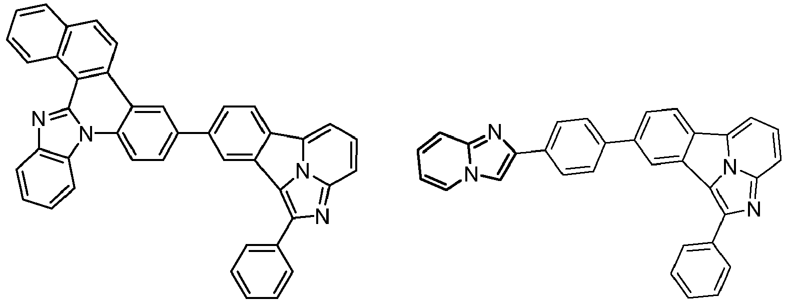

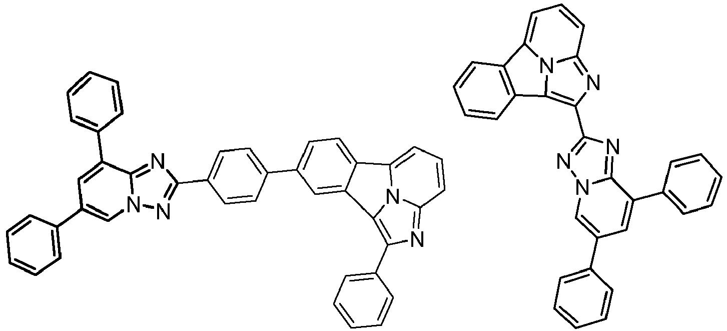

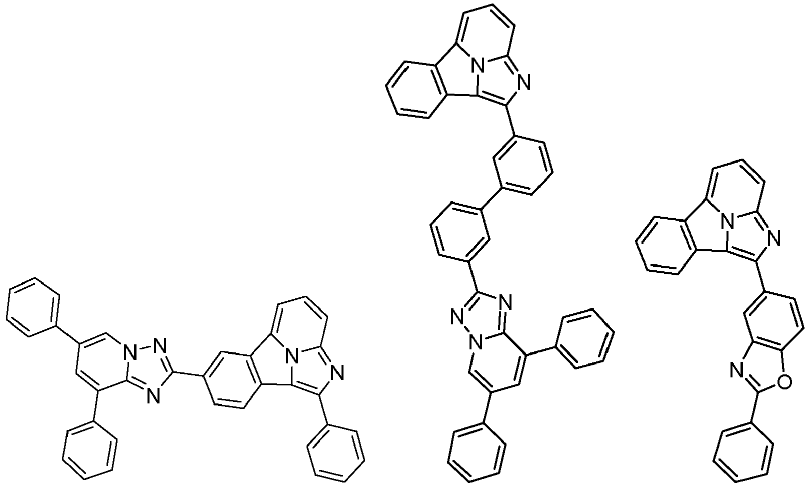

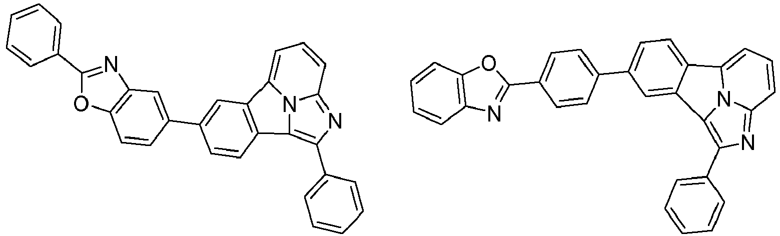

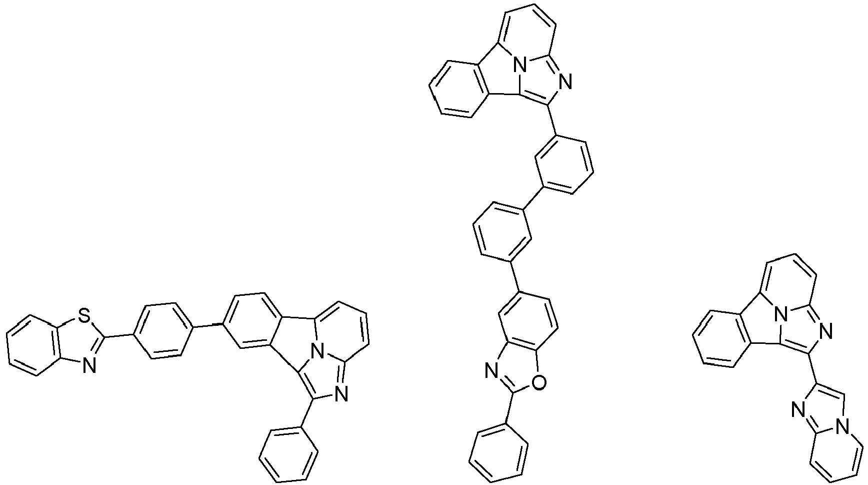

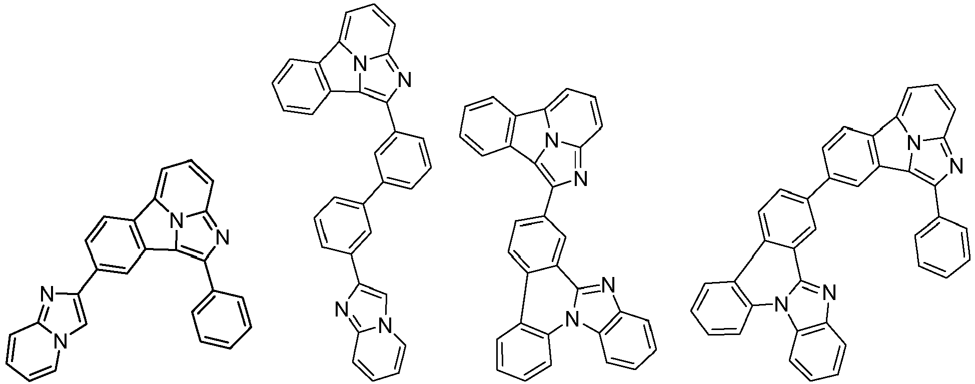

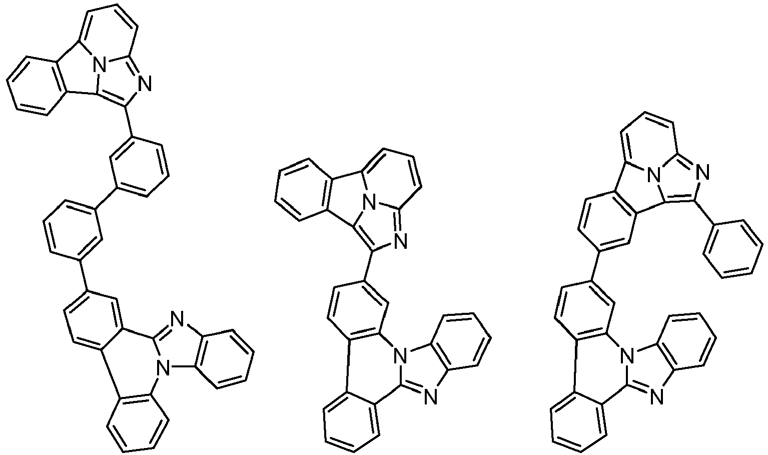

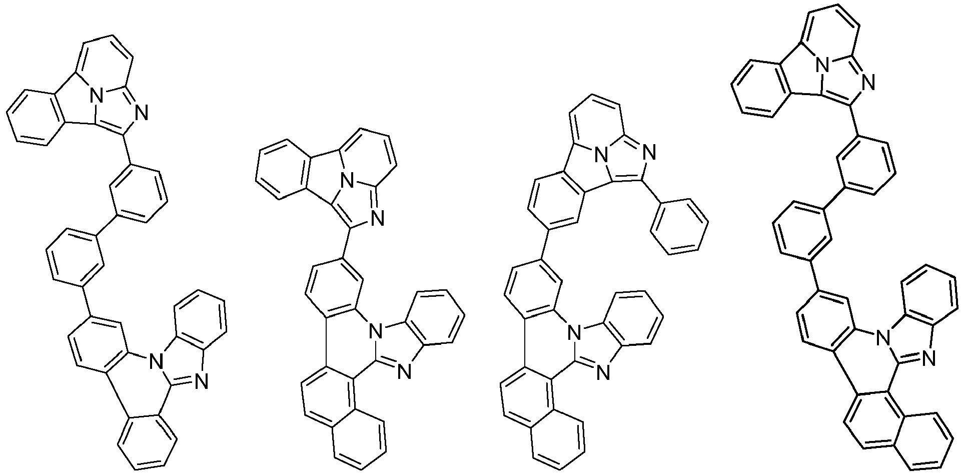

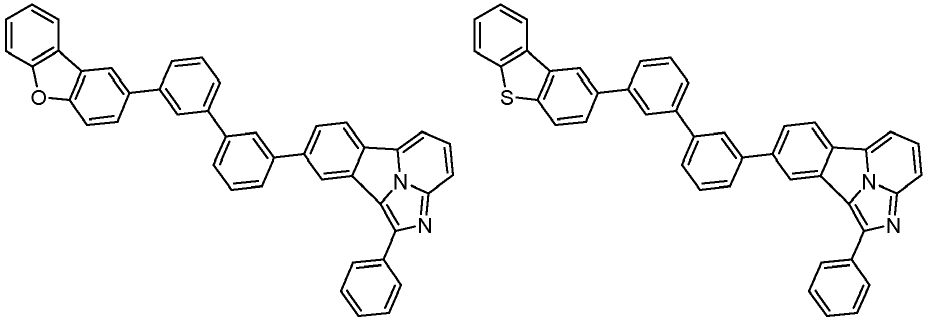

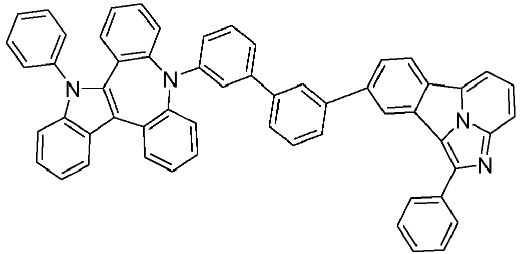

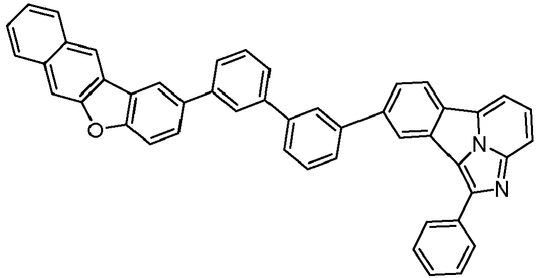

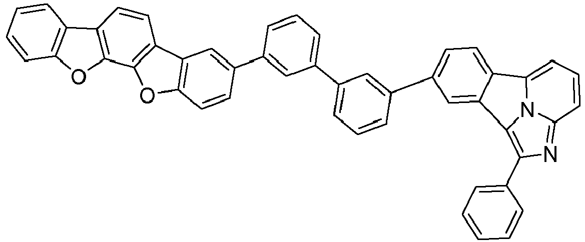

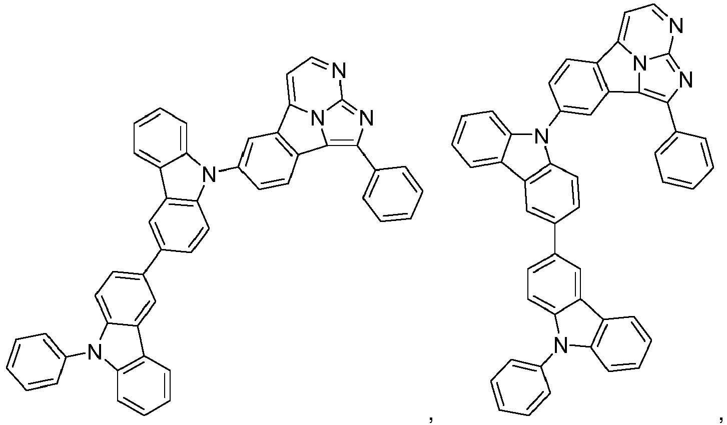

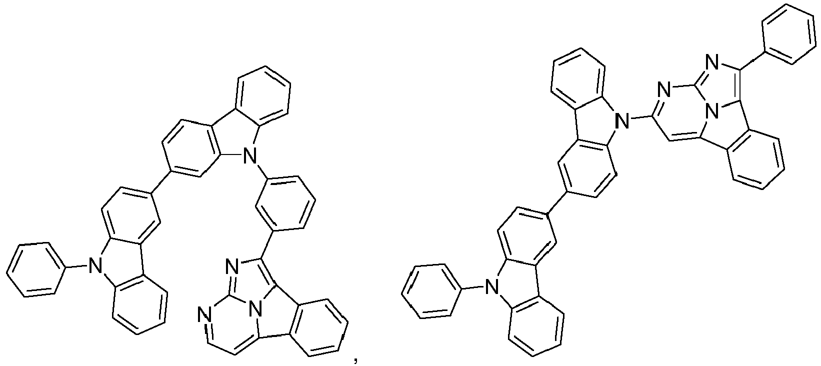

The compound of formula (I) according to the present invention is for example prepared by a process at least comprising step (A)
(A) coupling of a compound according to general formula (Ia) 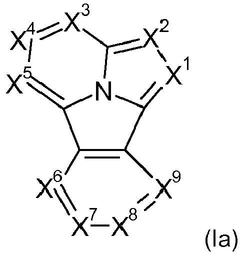 wherein at least one of X¹, X², X³, X⁴, X⁵, X⁶, X⁷, X⁸ and X⁹ is C-A,
   with a compound of formula R¹-H, R²-H, R³-H, R⁴-H, R⁵-H, R⁶-H, R⁷-H, R⁸-H or R⁹-H to obtain a compound according to general formula (I),
   wherein
   A is a selected from Cl, Br, I, F, OSO₂CH₃, and OSO₂CF₃ or OSO₂C₆H₄CH₃,

   wherein
   X¹ is CR¹ or N,
   X² is CR² or N,
   X³ is CR³ or N,
   X⁴ is CR⁴ or N,
   X⁵ is CR⁵ or N,
   X⁶ is CR⁶ or N,
   X⁷ is CR⁷ or N,
   X⁸ is CR⁸ or N,
   X⁹ is CR⁹ or N,
   wherein at least one of X¹ and X² is N,
   R¹, R², R³, R⁴, R⁵, R⁶, R⁷, R⁸ and R⁹
      are independently of each other H, -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, wherein R¹, R², R³, R⁴, R⁵, R⁶, R⁷, R⁸ or R⁹ in R¹-H, R²-H, R³-H, R⁴-H, R⁵-H, R⁶-H, R⁷-H, R⁸-H or R⁹-H is -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
   at least two of R³, R⁴, R⁵, R⁶, R⁷, R⁸ and R⁹ if present at adjacent carbon atoms together form at least one C₆-C₁₈aryl or C₁-C₂₄heteroaryl ring or ring system,
   L¹, L², L³ and L⁴ are independently of each other selected from a C₆-C₄₀aryl group which is unsubstituted or substituted by at least one group E and a C₁-C₂₄heteroaryl group which is unsubstituted or substituted by at least one group E,
   R¹⁰ is H, -CN or a C₁-C₂₅alkyl group which is unsubstituted or substituted by at least one group E and/or interrupted by at least one group D,
   o is independently of each other 0 or 1, p is independently of each other 0 or 1, q is independently of each other 0 or 1 and r is independently of each other 0 or 1, wherein at least one of o, p, q and r is 1;
   D is independently of each other -CO-, -COO-, -S-, -SO-, -SO₂-, -O-,-CR¹⁵=CR¹⁶-, -NR¹⁷-, -SiR²²R²³-, -POR²⁵-, -C≡C-,
   E is independently of each other -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, a C₆-C₄₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₂₄heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
   R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
   R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
   R¹⁷ and R¹⁸ together form a five or six membered aliphatic, aromatic or heteroaromatic ring,
   R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
   R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
   R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
   R²², R²³ and R²⁴ are independently of each other H, a C₁-C₁₈alkyl group, a C₆-C₁₈aryl group which is unsubstituted or substituted by at least one C₁-C₁₈alkyl group, and
   R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
   wherein, in the case that o + p + q + r is 1, L¹, L², L³ respectively L⁴ is selected from a C₁₀-C₄arylene group which is unsubstituted or substituted by at least one group E and a heteroarylene group having 10 to 24 ring atoms, which is unsubstituted or substituted by at least one group E.

Preferred groups X¹, X², X³, X⁴, X⁵, X⁶, X⁷, X⁸ and X⁹ - which are not C-A - are described above.

Suitable reaction conditions are mentioned in the examples.

The compounds of formula (I) according to the present invention are particularly suitable for use in organic electronics applications, especially OLEDs, especially as a host material, a charge transporting material, charge and/or exciton blocking material, preferably having a good overall performance, especially improved efficiency and/or driving voltage, preferably as host material. The structure of organic electronics applications, especially OLEDs is known in the art. Suitable structures are described below.

### Compounds of formula (I) according to the present invention in organic electronics applications

In the following applications of the compounds of formula (I) according to the present invention as mentioned above in organic electronic applications will be explained.

It has been found that the compounds of formula (I) according to the present invention are particularly suitable for use in applications in which charge carrier conductivity is required, especially for use in organic electronics applications, more preferably for use in organic light-emitting diodes (OLEDs).

The compounds of formula (I) according to the present invention being particularly suitable in OLEDs for use in a light-emitting layer, wherein the compound of formula (I) is preferably used as a host material, a charge transporting material, charge and/or exciton blocking material, more preferably as a host material.

In a preferred embodiment, the compounds of formula (I) according to the present invention are present in OLEDs as a host, as a single host or as a host in combination with one or more further hosts.

The present invention therefore relates to an electronic device, preferably an organic electroluminescent device, more preferably an organic light emitting diode (OLED), comprising least one compound of formula (I) according to the present invention.

The present invention preferably further relates to the electronic device according to the present invention, preferably an organic electroluminescence device, more preferably an organic light emitting diode (OLED), comprising a cathode, an anode, and a plurality of organic layers provided between the cathode and the anode, wherein at least one of the plurality of organic layers comprising at least one compound of formula (I) according to the present invention.

The present invention preferably further relates to the electronic device according to the present invention, preferably an organic electroluminescence device, more preferably an organic light emitting diode (OLED), comprising a cathode, an anode, and a plurality of organic layers provided between the cathode and the anode, wherein at least one of the plurality of organic layers is an emitting layer, wherein at least one organic layer, preferably the emitting layer, comprise least one compound of formula (I) according to the present invention.

More preferably, the emitting layer comprises at least one compound of formula (I) according to the present invention as a host material.

In a further preferred embodiment, the emitting layer comprises a heavy-metal complex material.

In a further preferred embodiment, the organic layers in the electronic device according to the present invention, preferably an organic electroluminescence device, more preferably an organic light emitting diode (OLED), comprise an electron transporting layer comprising the at least one compound of formula (I) according to the present invention, said electron transporting layer placed between the anode and the emitting layer.

Further preferably, at least one of the layers between the emitting layer and the anode in the electronic device according to the present invention, preferably an organic electroluminescence device, more preferably an organic light emitting diode (OLED), comprises at least one selected from an alkali metal, an alkaline earth metal, a rare earth metal, a compound comprising an alkali metal, an alkaline earth metal, or a rare earth metal, and a complex comprising an alkali metal, an alkaline earth metal, or a rare earth metal.

The present invention further relates to an electronic equipment comprising the organic electroluminescence device according to the present invention.

The present invention also relates to an emitting layer, preferably present in an electronic device, more preferably in an electroluminescence device, particularly preferably in an organic light emitting diode (OLED), comprising least one compound of formula (I) according to the present invention.

The present invention preferably further relates to the use of least one compound of formula (I) according to the present invention in an electronic device, preferably in an electroluminescence device, particularly preferably in an organic light emitting diode (OLED), preferably in an emitting layer.

According to the present application, the terms matrix and host are used interchangeable.

Suitable structures of organic electronic devices, especially organic light-emitting diodes (OLED), are known to those skilled in the art and are specified below.

Examples of preferred compounds according to general formulae (I) are shown above.

Most preferably, the electronic device according to the present invention is an organic light emitting diode (OLED).

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

A schematic structure of an example of the OLED in an aspect of the invention is for example shown in FIG. 1 wherein the OLED 1 comprises a substrate 2, an anode 3, a cathode 4, and an emission unit 10 disposed between the anode 3 and the cathode 4. The emission unit 10 comprises a light emitting layer 5 which comprises at least one emitting layer, preferably at least one phosphorescent emitting layer, containing a host material, preferably a phosphorescent host material, and a dopant material, preferably a phosphorescent dopant material (phosphorescent material). A hole injecting/transporting layer (an anode-side organic thin film layer) 6 may be disposed between the light emitting layer 5 and the anode 3, and an electron injecting/transporting layer (a cathode-side thin film layer) 7 may be disposed between the light emitting layer 5 and the cathode 4. An electron blocking layer may be disposed on the anode 3 side of the light emitting layer 5, and a hole blocking layer may be disposed on the cathode 4 side of the light emitting layer 5. With these blocking layers, electrons and holes are confined in the light emitting layer 5 to increase the degree of exciton generation in the light emitting layer 5.

The least one compound of formula (I) according to the present invention may be present in any layer of the OLED, preferably a host material, a charge transporting material, charge and/or exciton blocking material. More preferably, the least one compound of formula (I) according to the present invention are present as host material in the emitting layer of the OLED.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA (4,4',4"-Tris[(3-methylphenyl)phenylamino]triphenylamine),

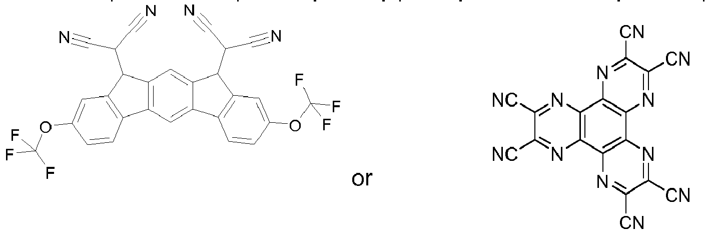

Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

According to a preferred embodiment the OLED according to the present invention comprises at least one compound according to general formula (I) or their preferred embodiments as a charge transporting material, preferably as a hole transporting layer. In addition to the compounds according to general formula (I) or without these compounds either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, Further suitable hole transport materials for layer (c) of the inventive OLED are triarylamines with (di)benzothiophen/(di)benzofuran; indolocarbazoles, and substituted phenylamine compounds. Combination of different hole transport material may be used. Examples are 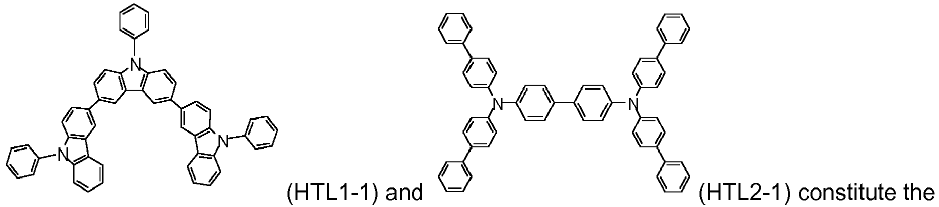 hole transport layer.

Customarily used hole-transporting molecules are selected from the group consisting of 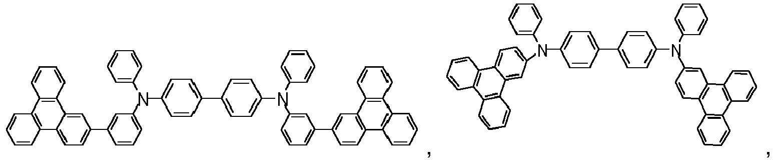 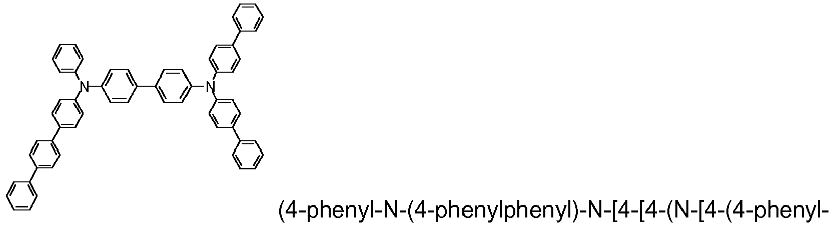 phenyl)phenyl]anilino)phenyl]phenyl]aniline), 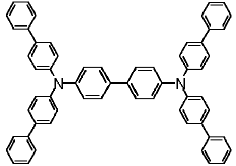 (4-phenyl-N-(4-phenylphenyl)-N-[4-[4-(4-phenyl-N-(4-phenylphenyl)anilino)phenyl]phenyl]aniline), 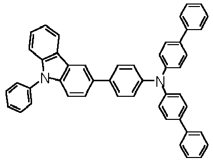 (4-phenyl-N-[4-(9-phenylcarbazol-3-yl)phenyl]-N-(4-phenylphenyl)aniline), 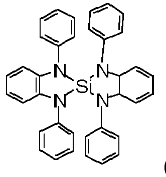 (1,1',3,3'-tetraphenylspiro[1,3,2-benzodiazasilole-2,2'-3a,7a-dihydro-1,3,2-benzodiazasilole]), 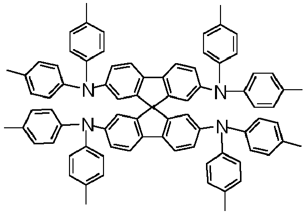 (N2,N2,N2',N2',N7,N7,N7',N7'-octakis(p-tolyl)-9,9'-spirobi[fluorene]-2,2',7,7'-tetramine),

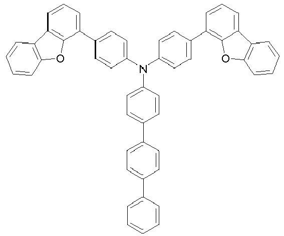

4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), and quinone compounds.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, transition metal, especially Ir, carbene complexes.

According to a preferred embodiment of the present invention, at least one compound of general formula (I) is present in the exciton blocking layer of the OLED according to the present invention.

### Emitting layer (e)

The light emitting layer is an organic layer having a light emitting function and is formed from one or more layers, wherein one of the layers comprises a host material (first host material), optionally a second host material, and the light emitting material.

According to a preferred embodiment of the present invention, at least one compound of general formula (I) is present in the emitting layer of the OLED according to the present invention, preferably as host material.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are used in combination and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one fluorescence or phosphorescence emitter, suitable emitter materials being known to those skilled in the art.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 to 570 nm). In another preferred embodiment, red emitter materials (570 to 680 nm) are preferred.

The phosphorescent dopant (phosphorescent emitter material) is a compound which usually emits light by releasing the energy of excited triplet state.

The compounds according to general formula (I) are most preferably used as the matrix (=host material) in the light-emitting layer.

Suitable metal complexes for use in the inventive OLEDs, preferably as emitter material, are known in the art.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Selected emitters, especially red emitters are:

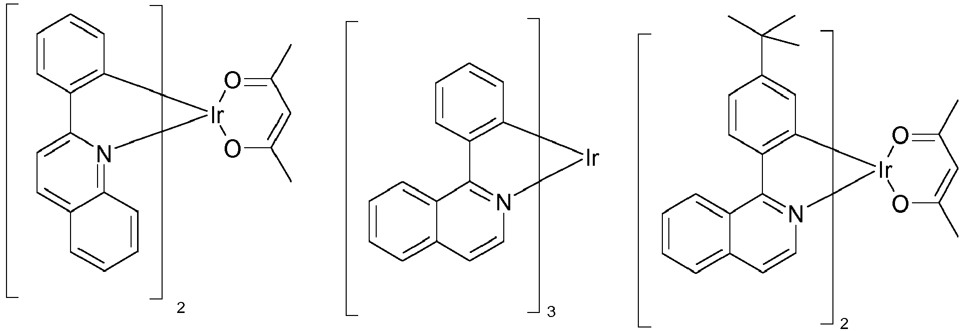

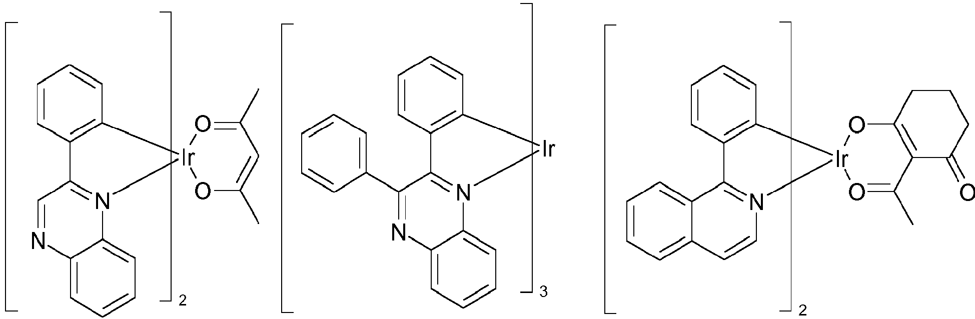

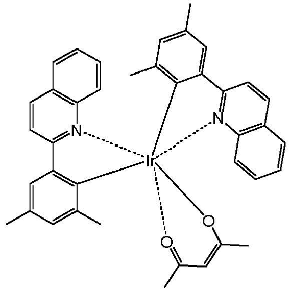

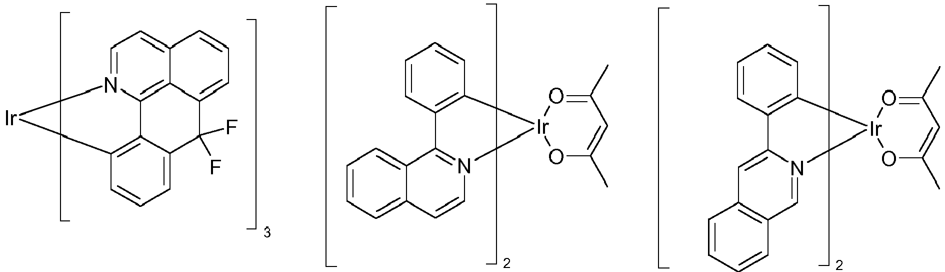

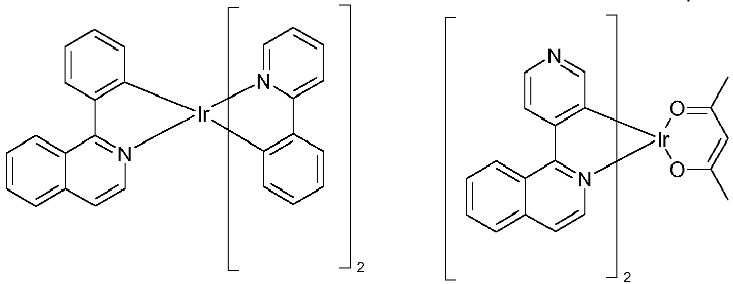

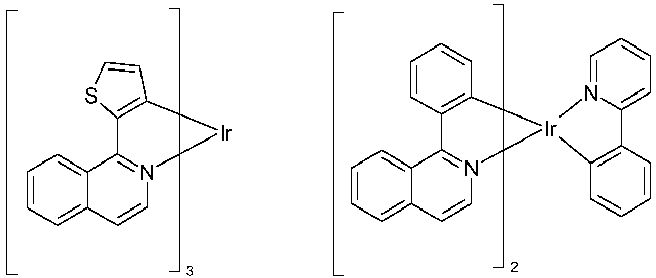

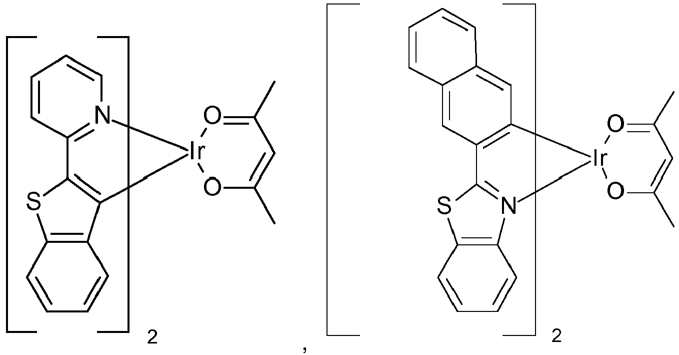

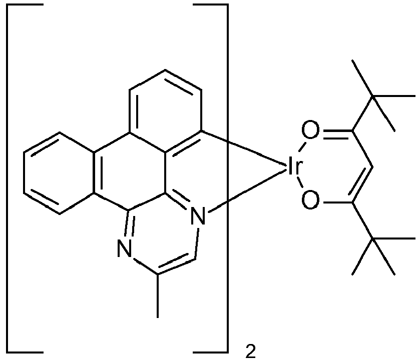

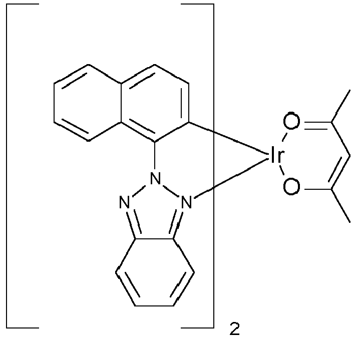

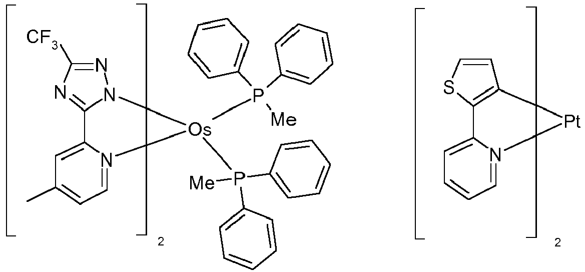

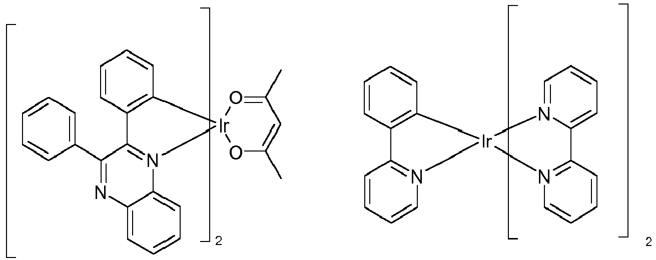

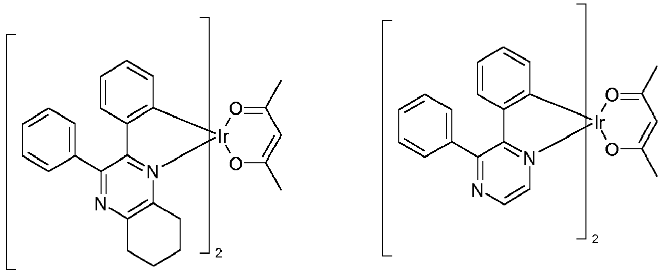

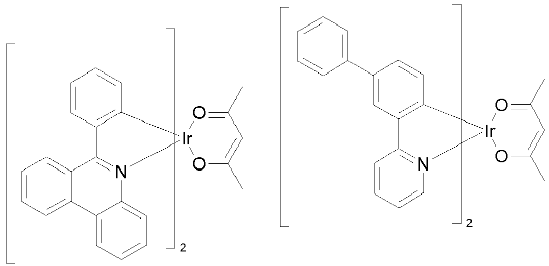

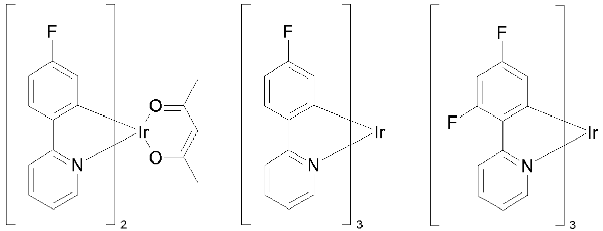

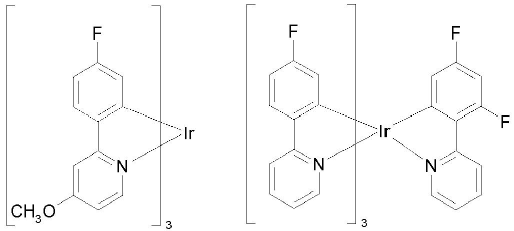

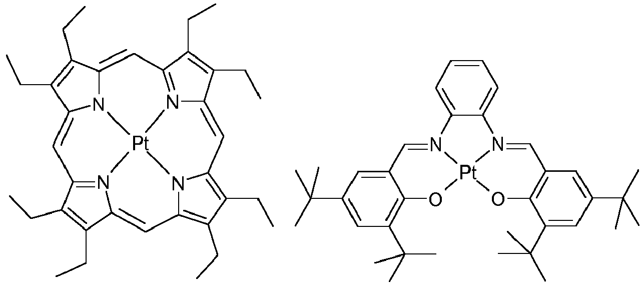

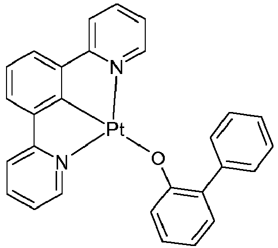

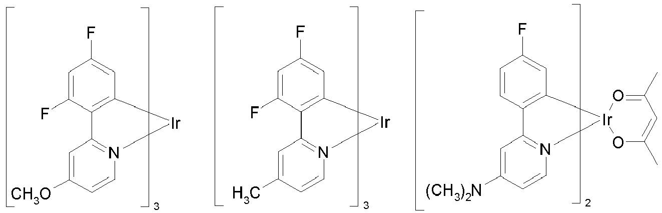

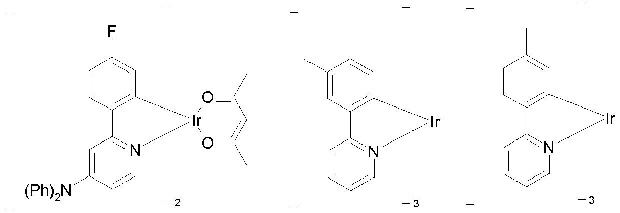

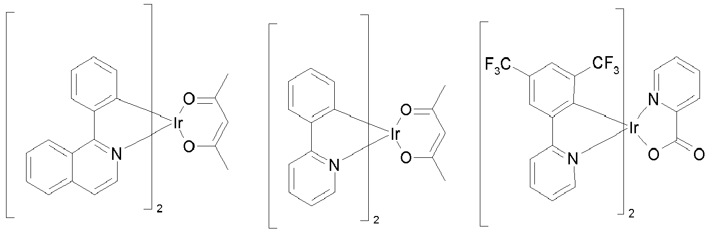

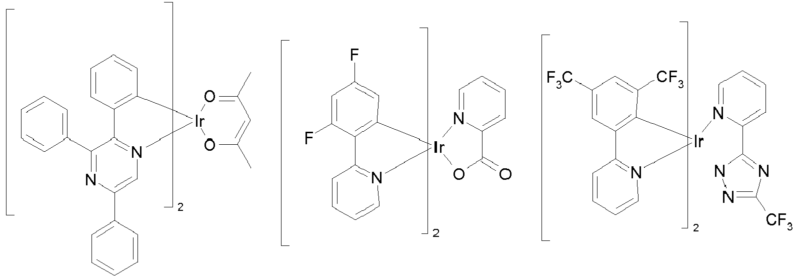

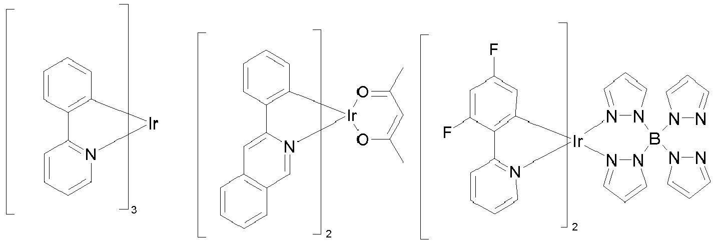

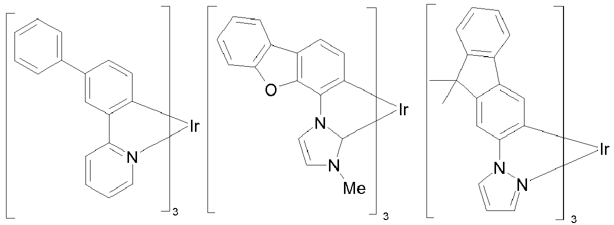

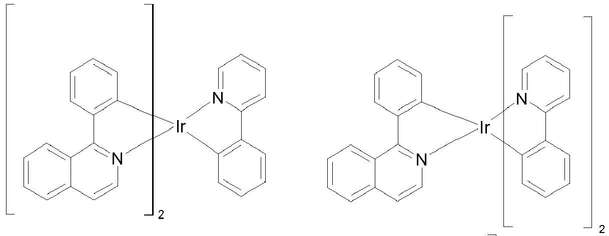

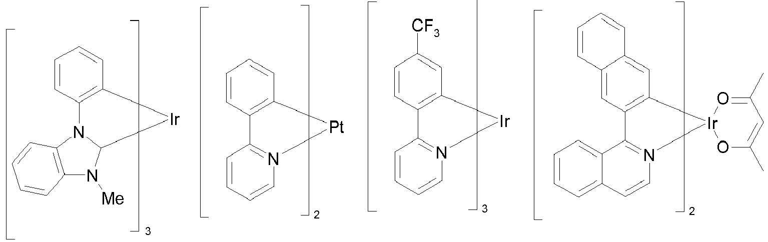

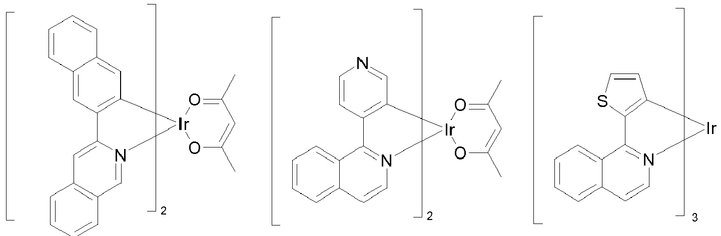

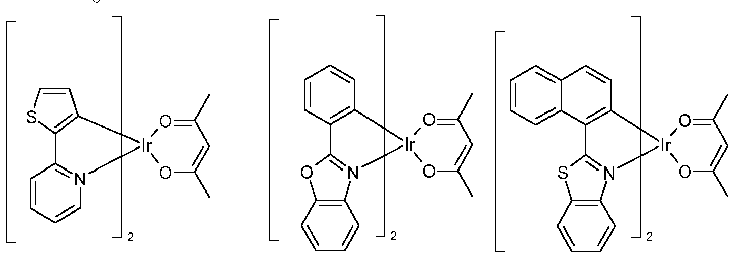

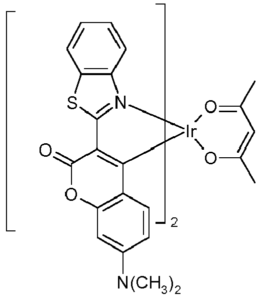

Further preferred red emitters are the following compounds:

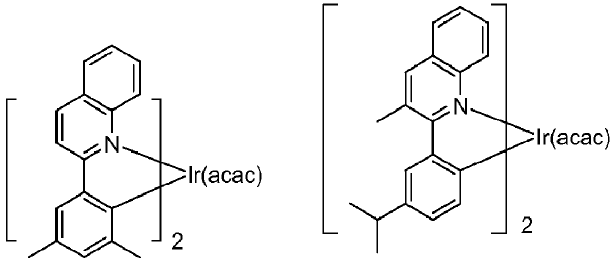

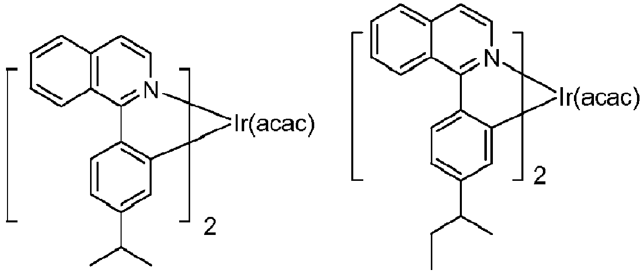

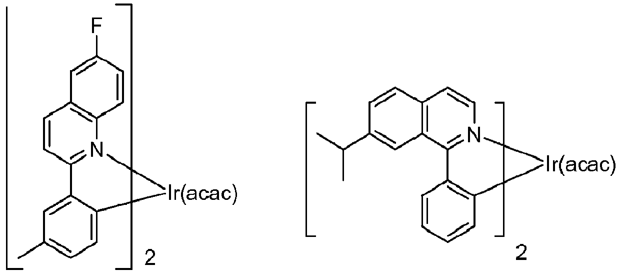

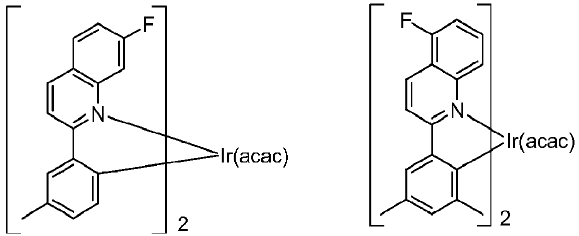

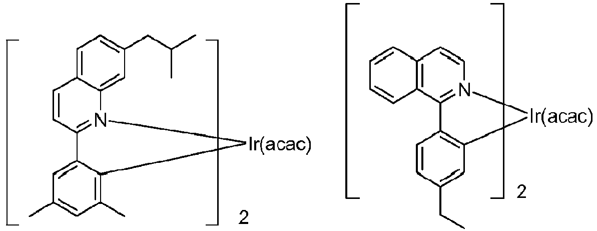

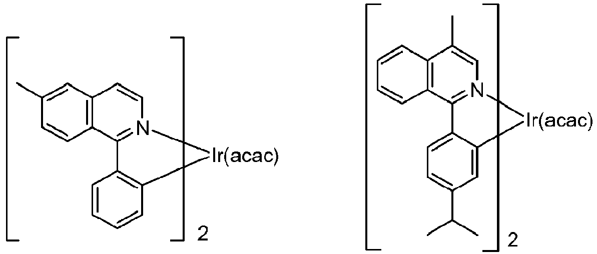

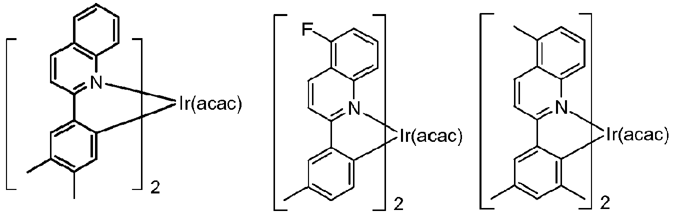

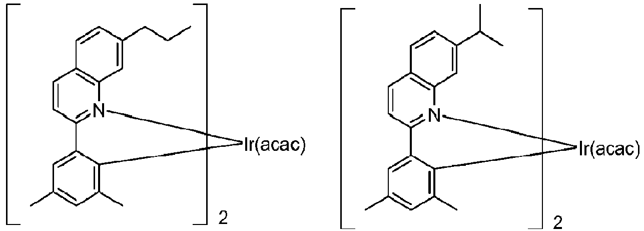

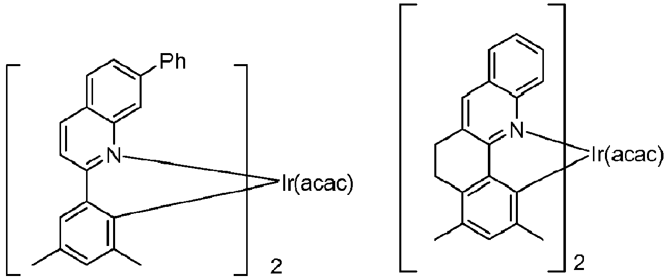

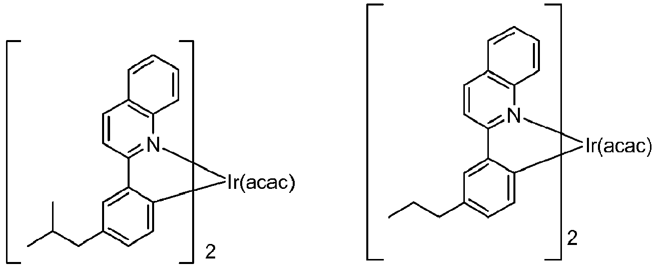

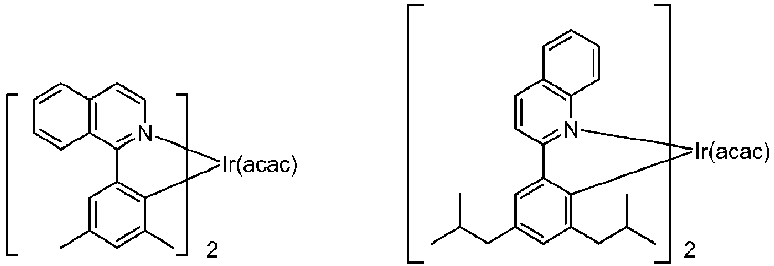

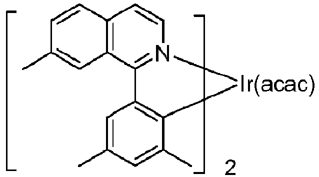

The emitter materials (dopants), preferably the phosphorescent emitter materials, may be used alone or in combination of two or more.

Further red emitters that may be used in the OLEDs according to the present invention are shown in the following:

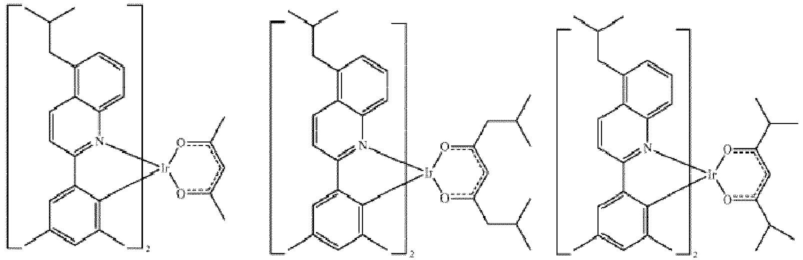

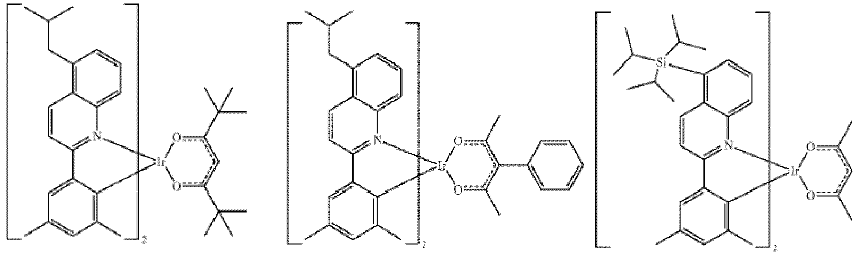

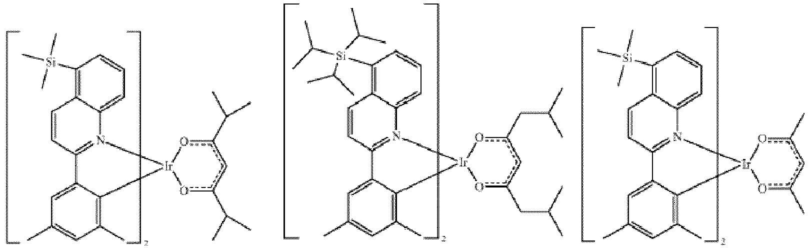

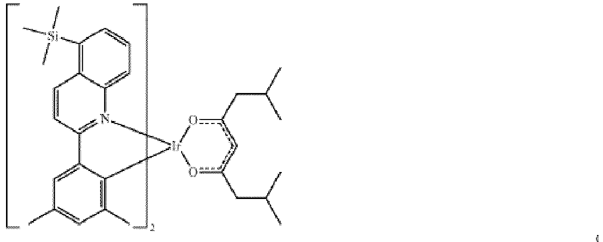

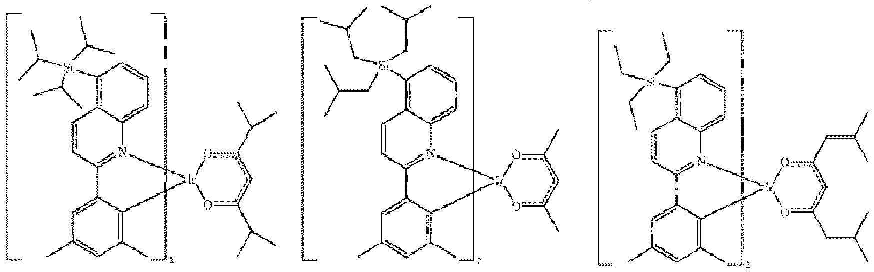

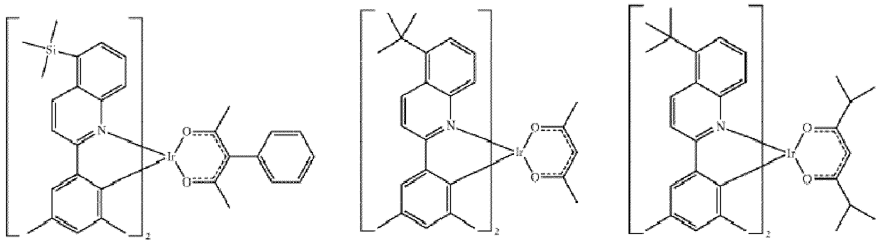

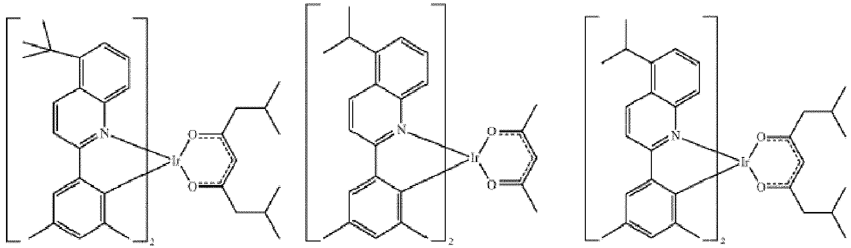

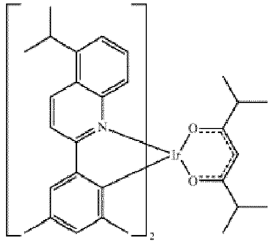

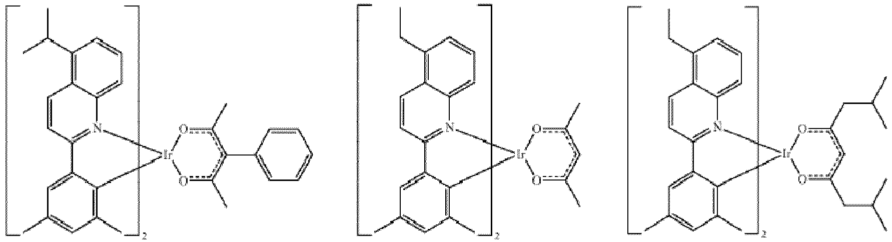

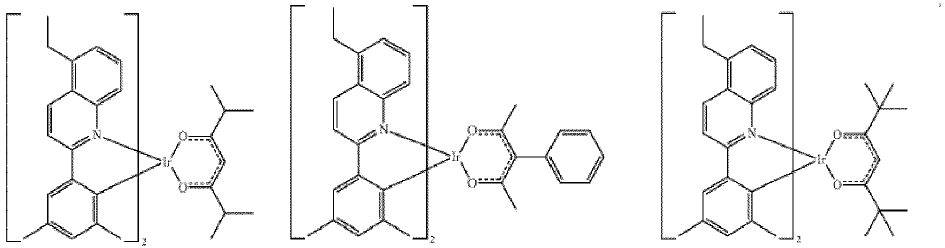

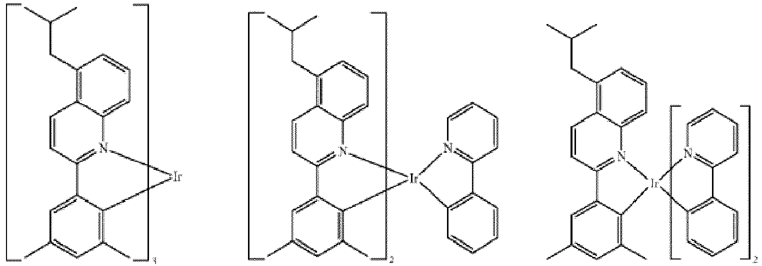

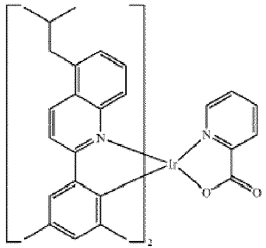

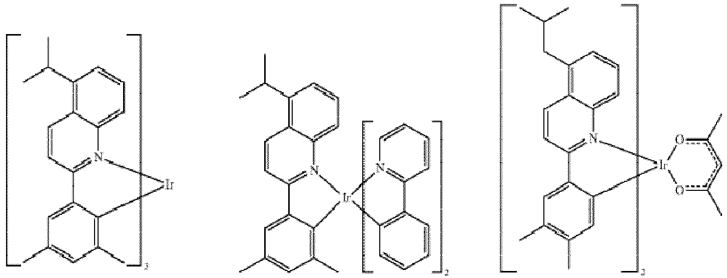

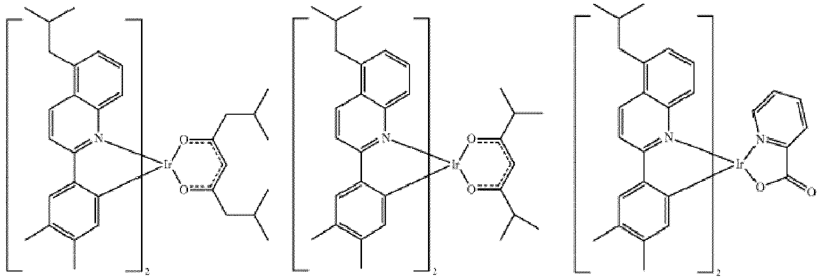

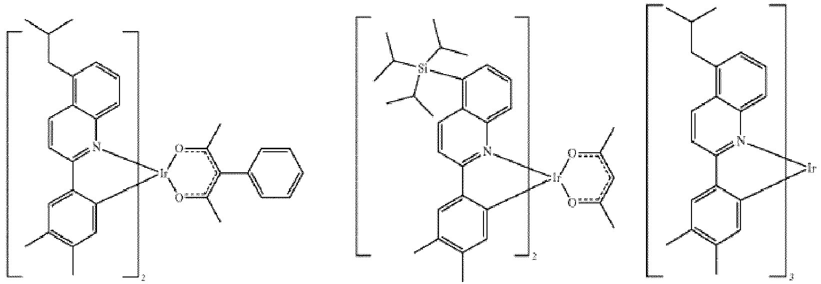

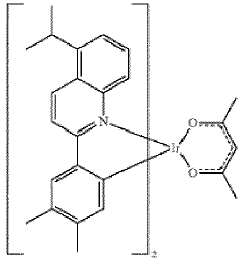

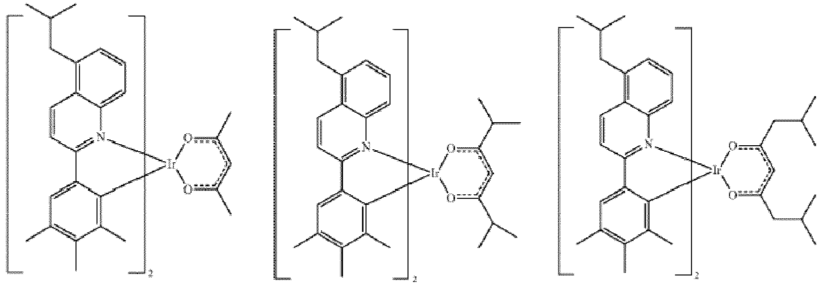

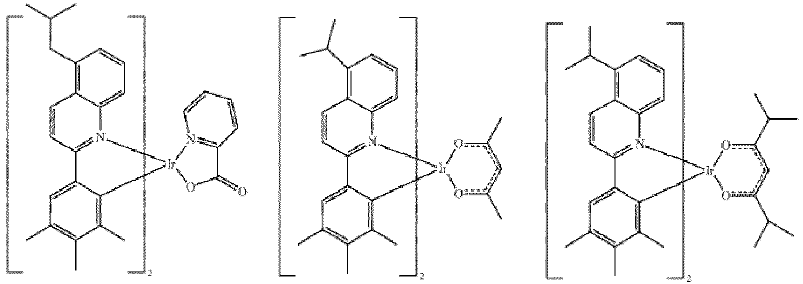

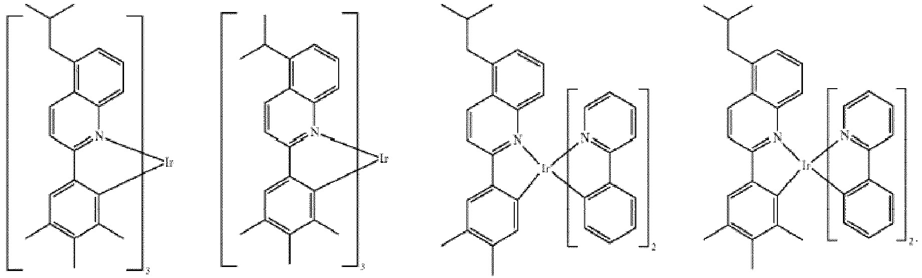

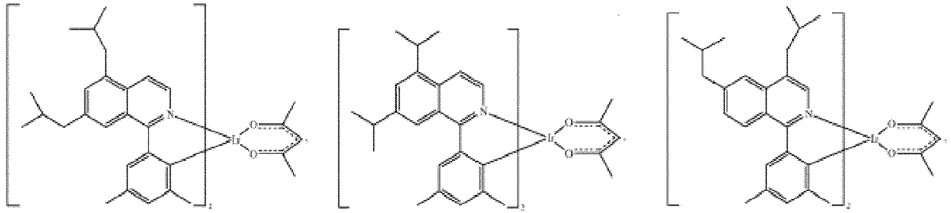

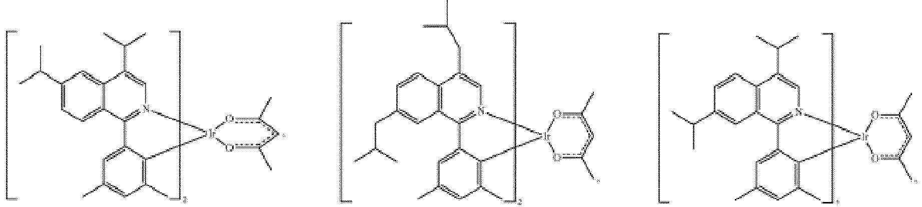

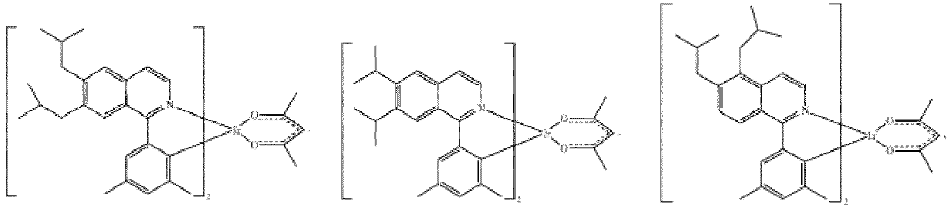

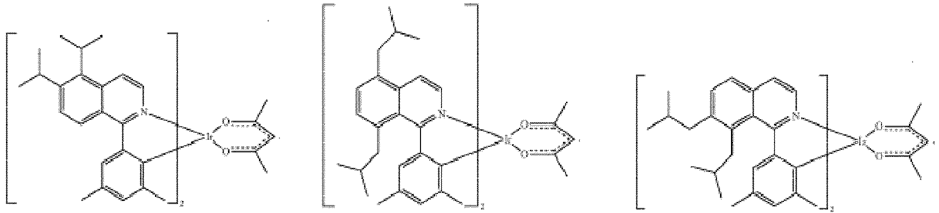

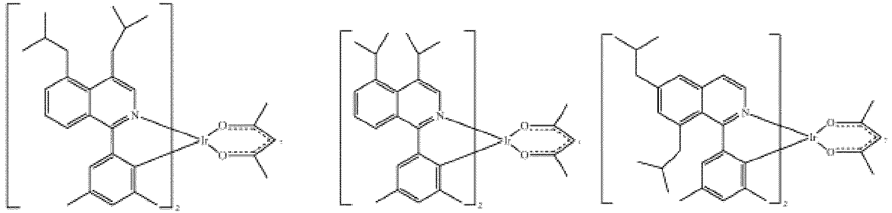

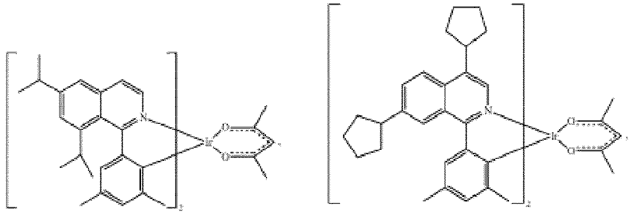

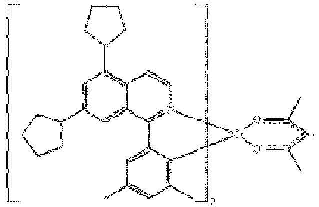

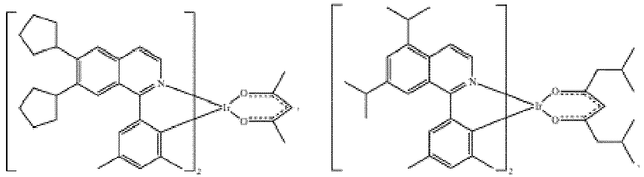

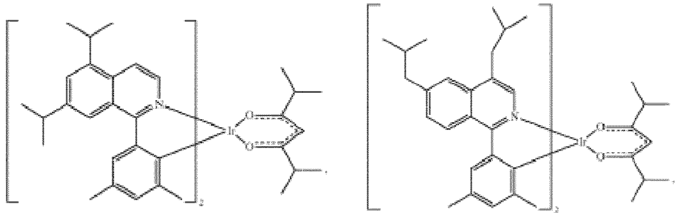

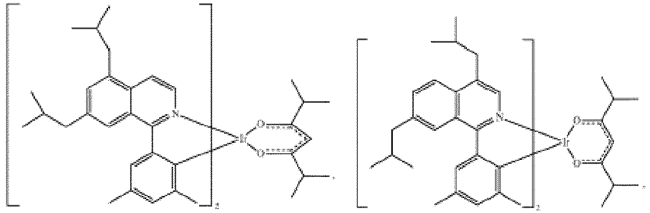

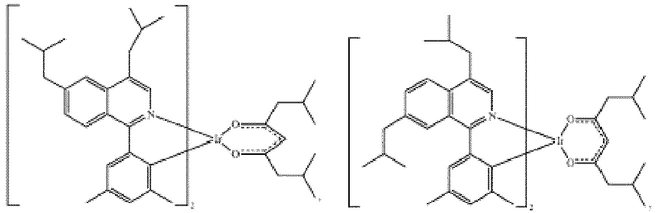

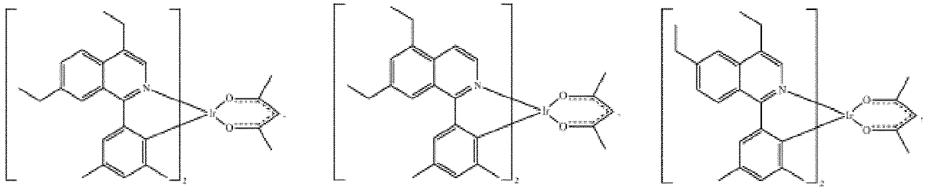

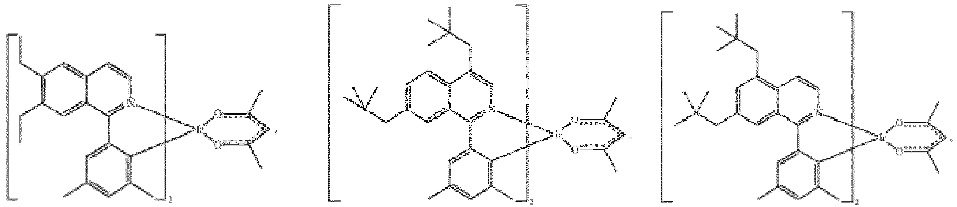

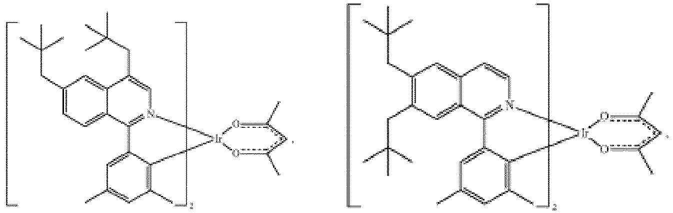

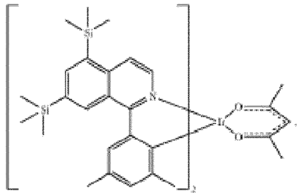

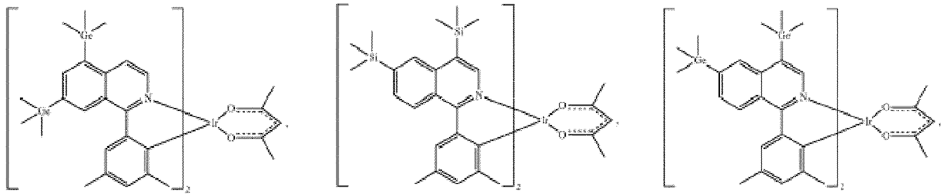

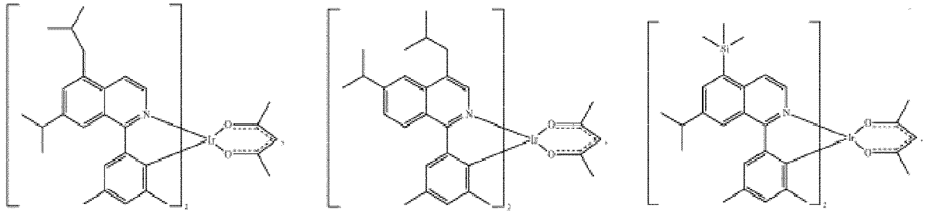

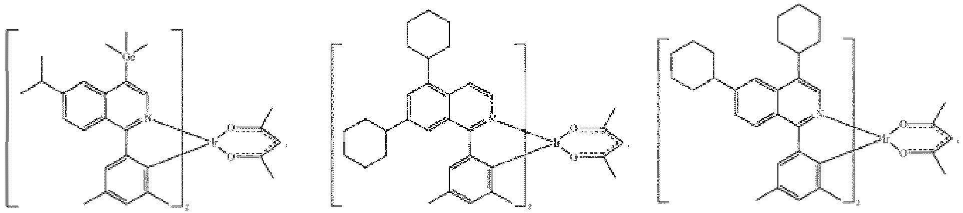

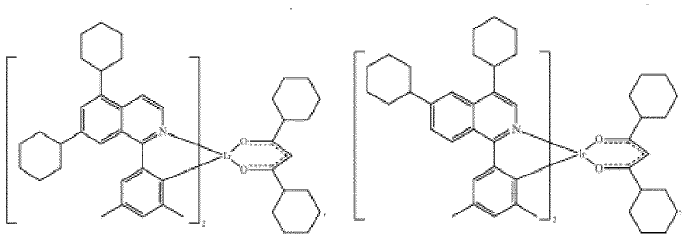

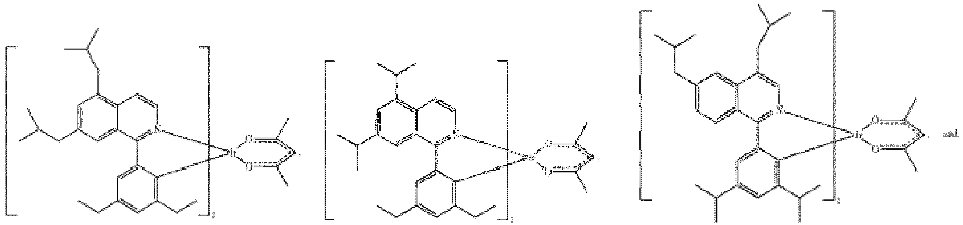

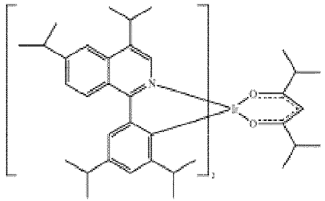

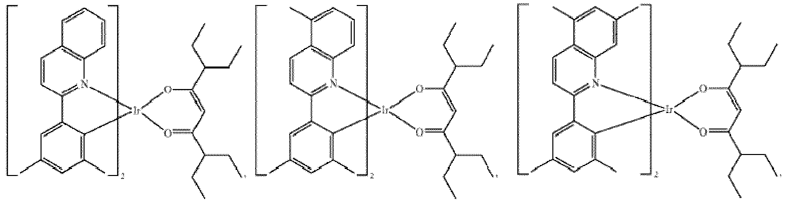

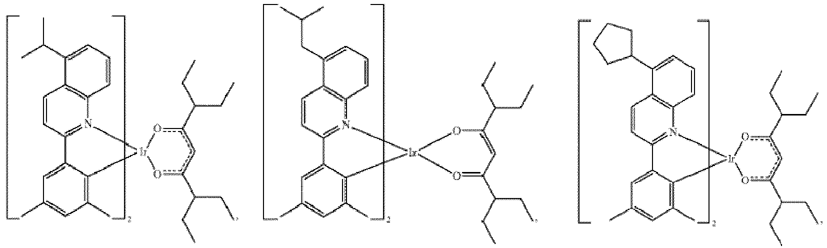

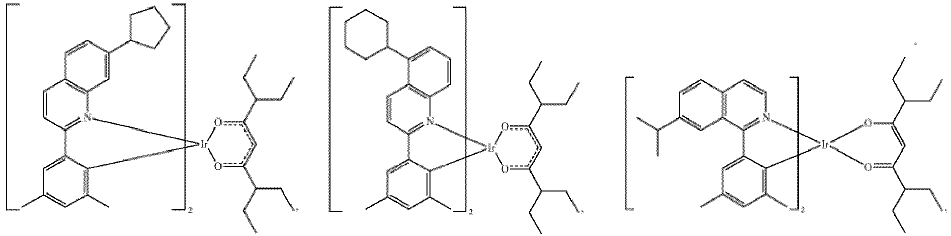

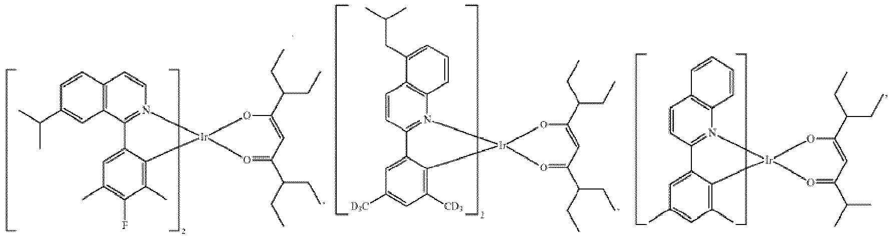

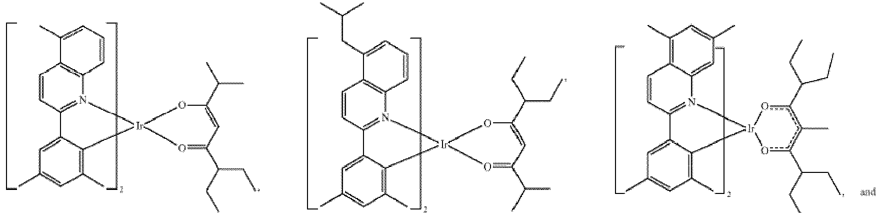

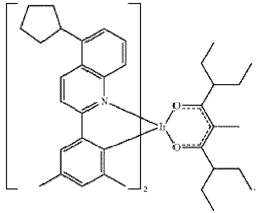

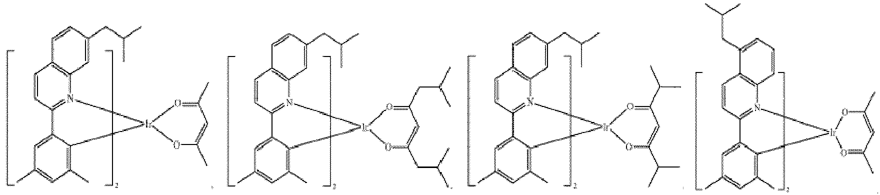

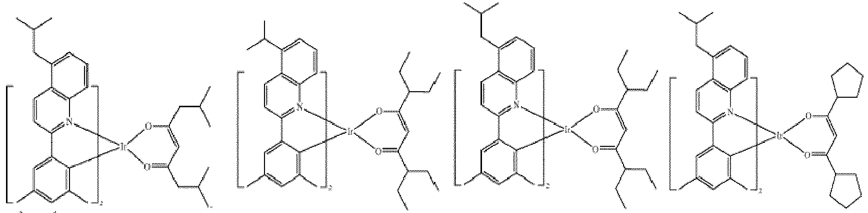

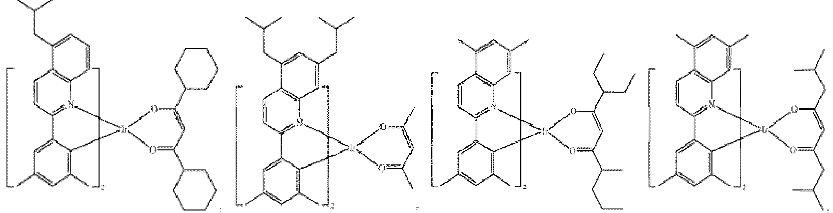

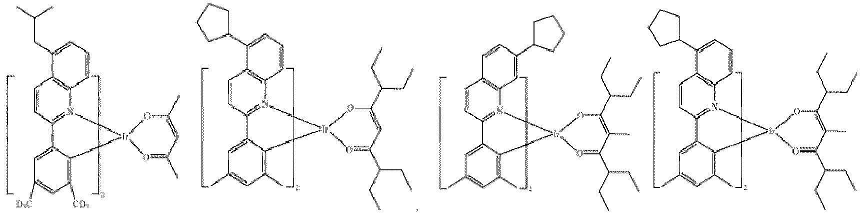

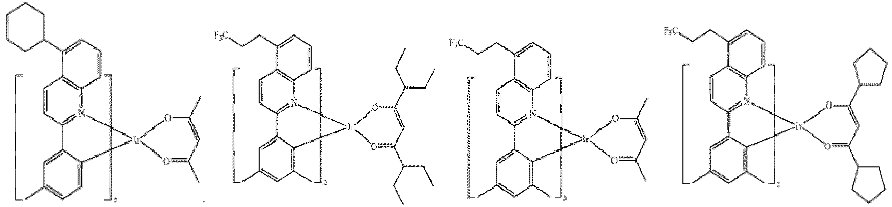

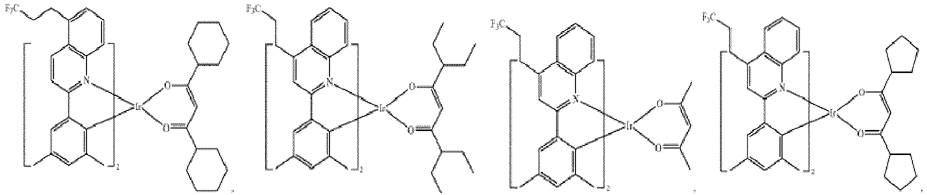

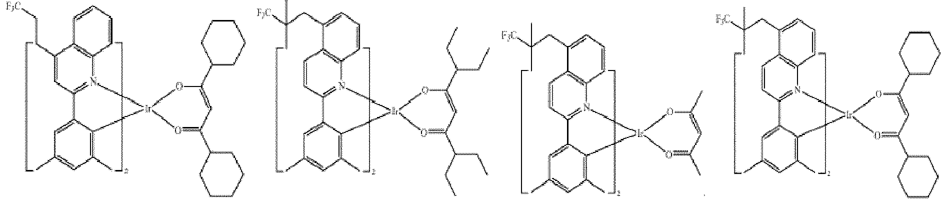

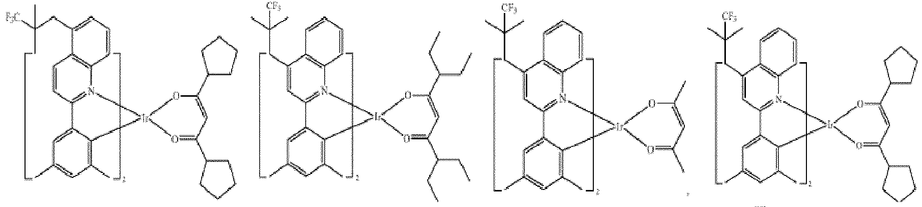

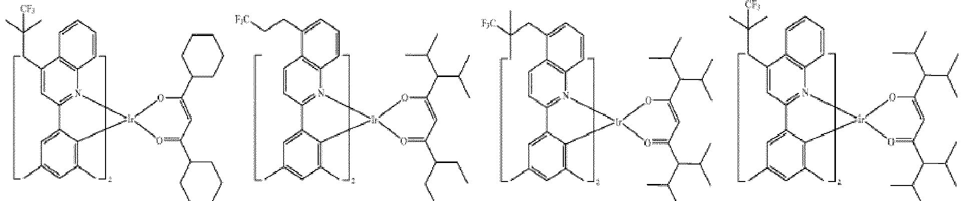

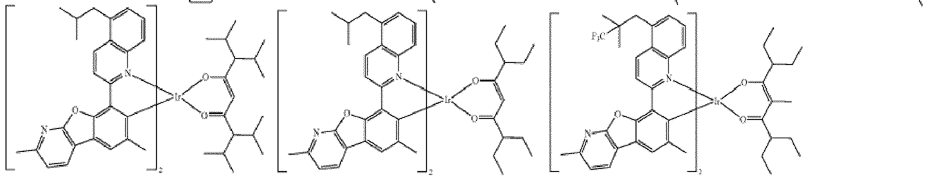

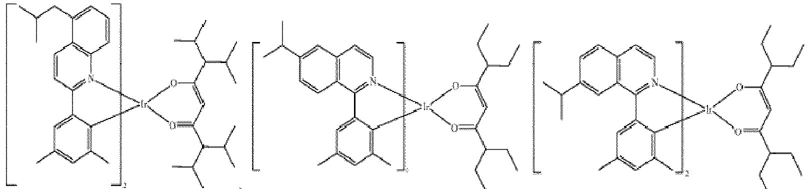

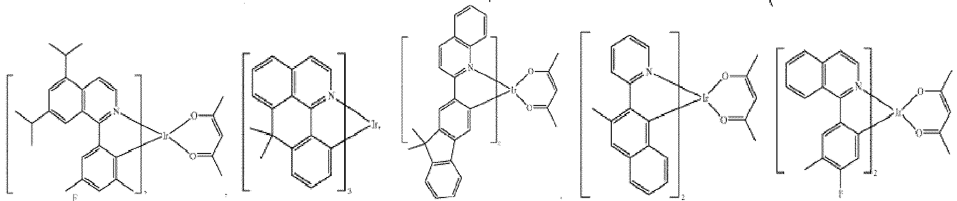

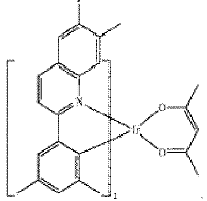

According to a further embodiment of the OLED according to the present invention, the light emitting layer may comprise at least one fluorescent, preferably blue, emitter. Examples of preferred blue dopants that may be present in the light emitting layer of the OLED according to the present invention are polycyclic amine derivatives. Particularly preferred aromatic amine derivatives are selected from compounds according to the following formula (20):

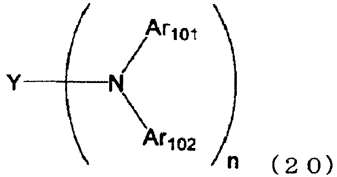

In the formula (20), Y is a substituted or unsubstituted fused aromatic hydrocarbon group including 10 to 50 ring carbon atoms.

Ar₁₀₁, and Ar₁₀₂ are independently a substituted or unsubstituted aryl group including 6 to 50 ring carbon atoms or a substituted or unsubstituted heterocyclic ring group including 5 to 50 ring atoms.

Specific examples of Y include the above-mentioned fused aryl group. Y is preferably a substituted or unsubstituted anthryl group, a substituted or unsubstituted pyrenyl group or a substituted or unsubstituted chrysenyl group.

n is an integer of 1 to 4. It is preferred that n be an integer of 1 to 2.

The above-mentioned formula (20) is preferably one represented by the following formulae (21) to (24).

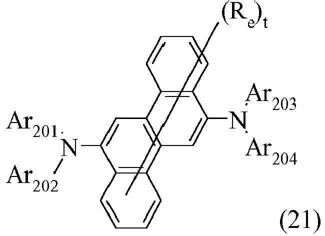

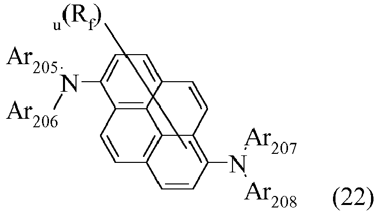

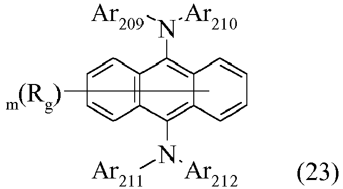

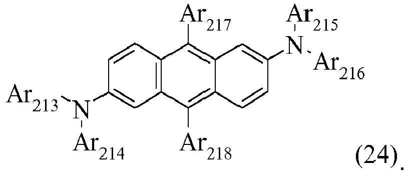

In the formulae (21) to (24), Rₑ, R_{f} and R_{g} are independently a substituted or unsubstituted alkyl group including 1 to 20 carbon atoms, a substituted or unsubstituted alkenyl group including 2 to 50 carbon atoms, a substituted or unsubstituted alkynyl group including 2 to 50 carbon atoms, a substituted or unsubstituted aralykyl group including 1 to 20 carbon atoms, a substituted or unsubstituted cycloalkyl group including 3 to 20 ring carbon atoms, a substituted or unsubstituted alkoxy group including 1 to 20 carbon atoms, a substituted or unsubstituted aryloxy group including 6 to 20 ring carbon atoms, a substituted or unsubstituted aryl group including 6 to 50 ring carbon atoms, a substituted or unsubstituted silyl group, a substituted or unsubstituted alkyl germanium group including 1 to 50 carbon atoms or a substituted or unsubstituted aryl germanium group including 6 to 50 ring carbon atoms. Rₑ, R_{f} and R_{g} may independently be bonded to any of the bonding positions of the benzene rings that constitutes the fused polycyclic skeleton.

As preferable examples of Rₑ, R_{f} and R_{g}, a substituted or unsubstituted aryl group including 6 to 50 ring carbon atoms can be given. More preferably, Rₑ, R_{f} and R_{g} are a substituted or unsubstituted phenyl group, a substituted or unsubstituted naphthyl group, or the like.

t is an integer of 0 to 10. u is an integer of 0 to 8. m is an integer of 0 to 10. Ar₂₀₁ to Ar₂₁₈ are independently an aryl group including 6 to 50 ring carbon atoms or a substituted or unsubstituted heterocyclic group including 5 to 50 ring atoms.

Preferred examples of Ar₂₀₁ to Ar₂₁₈ include a substituted or unsubstituted phenyl group, a substituted or unsubstituted dibenzofuranyl group or the like. As preferable examples of the substituent of Ar₂₀₁ to Ar₂₁₈, an alkyl group, a cyano group and a substituted or unsubstituted silyl group can be given.

In the formulae (21) to (24), as examples of the alkyl group, the alkoxy group, the aryl group, the aryloxy group and the heterocyclic group, those exemplified above can be given.

As the alkenyl group including 2 to 50, preferably 2 to 30, more preferably 2 to 20, and particularly preferably 2 to 10, carbon atoms, a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2-dimethylallyl group, a 1-phenyl-1-butenyl group, a 3-phenyl-1-butenyl group or the like can be given. Preferred are a styryl group, a 2,2-diphenylvinyl group, a 1,2-diphenylvinyl group or the like.

As the alkynyl group including 2 to 50 (preferably 2 to 30, more preferably 2 to 20, particularly preferably 2 to 10) carbon atoms, a propargyl group, a 3-pentynyl group or the like can be given.

As the alkyl germanium group, a methylhydrogermyl group, a trimethylgermyl group, a triethyl-germyl group, a tripropylgermyl group, a dimethyl-t-butylgermyl group or the like can be given.

As the aryl germanium group, a phenyldihydrogermyl group, a diphenylhydrogermyl group, a triphenylgermyl group, a tritolylgermyl group, a trinaphthylgermyl group or the like can be given.

As the styrylamine compound and the styryldiamine compound, those represented by the following formulas (17) and (18) are preferable.

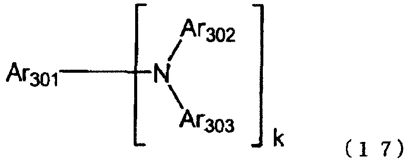

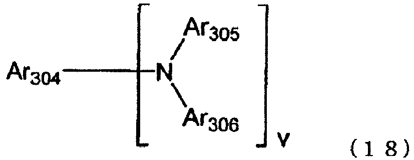

In the formula (17), Ar₃₀₁ is a k-valent group; a k-valent group corresponding to a phenyl group, a naphthyl group, a biphenyl group, a terphenyl group, a stilbene group, a styrylaryl group and a distyrylaryl group. Ar₃₀₂ and Ar₃₀₃ are independently an aryl group including 6 to 20 ring carbon atoms, and Ar₃₀₁, Ar₃₀₂ and Ar₃₀₃ may be substituted.

k is an integer of 1 to 4, with an integer of 1 and 2 being preferable. Any one of Ar₃₀₁ to Ar₃₀₃ is a group including a styryl group. It is further preferred that at least one of Ar₃₀₂ and Ar₃₀₃ be substituted by a styryl group.

As for the aryl group including 6 to 20 ring carbon atoms, the above-mentioned aryl group can be specifically given. Preferable examples include a phenyl group, a naphthyl group, an anthranyl group, a phenanthryl group, a terphenyl group or the like.

In the formula (18), Ar₃₀₄ to Ar₃₀₆ are a v-valent substituted or unsubstituted aryl group including 6 to 40 ring carbon atoms. v is an integer of 1 to 4, with an integer of 1 and 2 being preferable.

Here, as the aryl group including 6 to 40 ring carbon atoms in the formula (18), the above-mentioned aryl group can be specifically given. A naphthyl group, an anthranyl group, a chrysenyl group, a pyrenyl group or an aryl group represented by the formula (20) is preferable.

As preferable substituents that substitute on the aryl group, an alkyl group including 1 to 6 carbon atoms, an alkoxy group including 1 to 6 carbon atoms, an aryl group including 6 to 40 ring carbon atoms, an amino group substituted by an aryl group including 6 to 40 ring carbon atoms, an ester group including an aryl group that includes 5 to 40 ring carbon atoms, an ester group including an alkyl group that includes 1 to 6 carbon atoms, a cyano group, a nitro group, a halogen atom or the like can be given.

A further suitable emitter material is shown below:

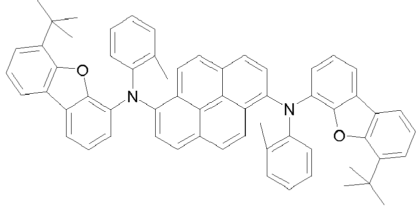

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

### Host (matrix) materials

The compound of formula (I) is preferably employed as host material, more preferably as phosphorescent host material.

The light-emitting layer may comprise further components in addition to the emitter material. For example, a fluorescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are usually employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials (hosts) mentioned in this application. According to a preferred embodiment, the electronic device according to the present invention, preferably the OLED according to the present invention, comprises at least one compound according to general formula (I) as matrix (host) material.

According to one embodiment, the light-emitting layer comprises at least one emitter material and at least two matrix materials (co-host system), wherein one of the matrix materials is a compound according to general formula (I) and the other matrix material(s) is/are used as co-host(s). Suitable other host materials than the compounds of general formula (I) (co-hosts) are known by a person skilled in the art. "Further host materials" means in the sense of the present application, host materials different from the compounds of general formula (I). However, it is also possible to use two or more different compounds of general formula (I) as host material in the light-emitting layer in an OLED of the present application. This embodiment is preferably realized with emitter materials that emit red light.

According to another embodiment, the light-emitting layer comprises at least one emitter material and a compound according to general formula (I) as a single matrix material. Examples of preferred compounds of general formula (I) useful as single host material are shown above. This embodiment is preferably realized with emitter materials that emit red light.

In the case that the compound of formula (I) is not employed as host material, host materials known in the art are employed. A suitable host material (especially useful in the case of an organic emitter material) is for example:

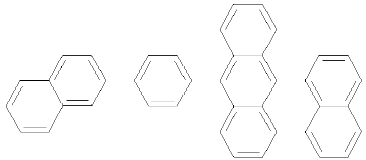

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one preferred embodiment at least one compound according to general formula (I) - where the sum total of the emitter material and of the matrix material adds up to 100% by weight.

In a further more preferred embodiment, the light-emitting layer comprises a compound of general formula (I) as matrix material, at least one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound according to general formula (I) and the further matrix material, where the sum total of the at least one emitter material, the further matrix material and of the compound of general formula (I) adds up to 100% by weight.

The content ratio of the compound according to general formula (I) as first host material and the second matrix material as co-host in the light emitting layer is not particularly limited and may be selected accordingly, and the ratio of first host material: second host material is preferably 1:99 to 99:1, more preferably 10:90 to 90:10, each based on mass.

Suitable host materials that may be used in the electronic device according to the present invention as host materials, if the compounds according to the present invention are not used as host material, but for example as charge transporting material, i.e. as electron transporting material or hole transporting material, are also known by a person skilled in the art.

According to the present invention, the compounds according to general formula (I) are preferably be used as host material in the light emitting layer of the electronic device, preferably in a OLED, according to the present invention. The compounds according to general formula (I) can be used (a) as single host materials or can be used (b) in combination with any compounds suitable as host materials as mentioned above.

### Electron transport layer (g):

Electron transport layer may include a material capable of transporting electrons. Electron transport layer may be intrinsic (undoped), or doped. Doping may be used to enhance conductivity.

The compound according to general formula (I) according to the present invention is also suitable as electron transport material, either alone or in combination with one or more of the electron transport materials mentioned below. The compound according to general formula (I) according to the present invention is preferably suitable as electron transport material, if a blue fluorescent emitter is present in the emitting layer.

Further suitable electron-transporting materials for layer (g) of the inventive OLEDs, which may be used in combination with the compound of general formula (I) according to the present invention or in absence of the compound of general formula (I) according to the present invention as electron transport material, comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA), and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials, which may be used in combination with the compound of general formula (I) according to the present invention or in absence of the compound of general formula (I) according to the present invention as electron transport material, are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 (ETM, HTM*).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula **(XVI')** below, preferably a compound of the formula **(XVI'a)** below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below **(formula XVII').**

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄.

In a preferred embodiment, the electron-transport layer comprises at least one compound of the general formula **(XVII')** 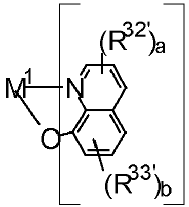
p , in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula **(XVII')** is 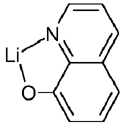 **(Liq),** which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula **(XVI'),** 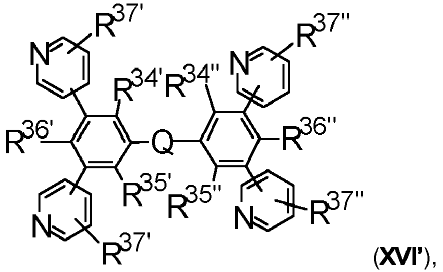 in which
R^{34"}, R^{35"}, R^{36"}, R^{37"}, R^{34'}, R^{35'}, R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-; E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which R^{38'} and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R⁴⁰' and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula **(XVI')** are compounds of the formula **(XVI'a)** 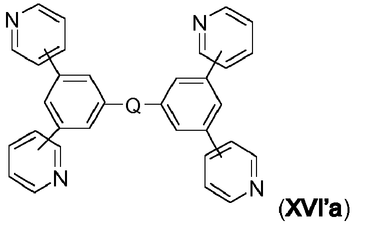 in which Q is: 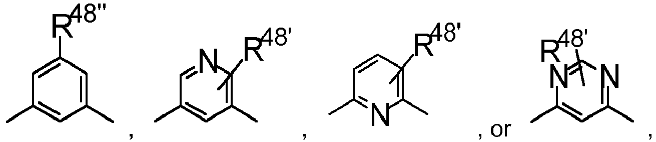 R^{48'} is H or C₁-C₁₈-alkyl and
R^{48"} is H, C₁-C₁₈-alkyl or

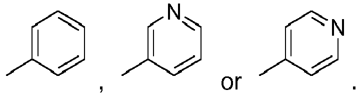

Particular preference is given to a compound of the formula

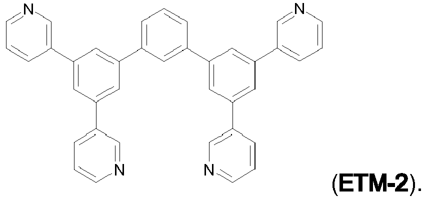

In a further, very particularly preferred embodiment, the electron-transport layer comprises a compound **Liq** and a compound **ETM-2.**

A further suitable electron transport material is:

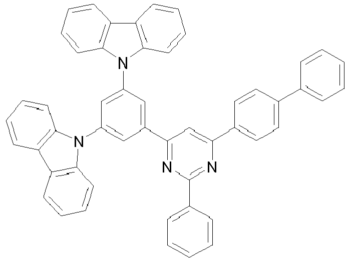

In a preferred embodiment, the electron-transport layer comprises at least one compound of the formula (**XVII'**) in an amount of 99 to 1% by weight, preferably 75 to 25% by weight, more preferably about 50% by weight, and at least one compound of the formula **(XVI')** in an amount of 1 to 99% by weight, preferably 25 to 75% by weight, more preferably about 50% by weight, where the amount of the compounds of the formulae **(XVII')** and the amount of the compounds of the formulae (**XVI'**) adds up to a total of 100% by weight.

The preparation of the compounds of the formula **(XVI')** is described in J. Kido et al., Chem. Commun. (2008) 5821-5823, J. Kido et al., Chem. Mater. 20 (2008) 5951-5953, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Dibenzofuran compound 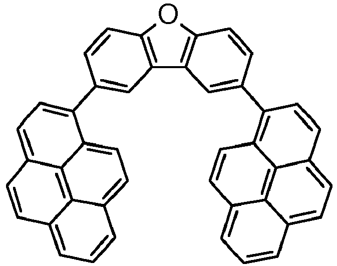 **(A-10;** = **ETM-1)** is most preferred.

In a preferred embodiment, the electron-transport layer comprises **Liq** in an amount of 99 to 1% by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises **ETM-1.**

In a further preferred embodiment, the electron-transport layer comprises a compound such as, for example, a compound of formula 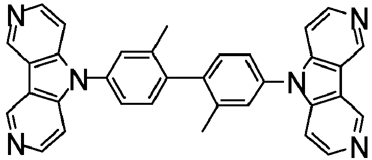 **(ETM-3),** a compound of formula 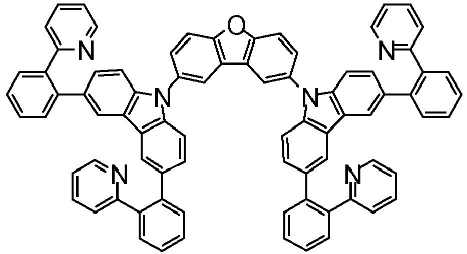 **(ETM-4),** and a compound of formula 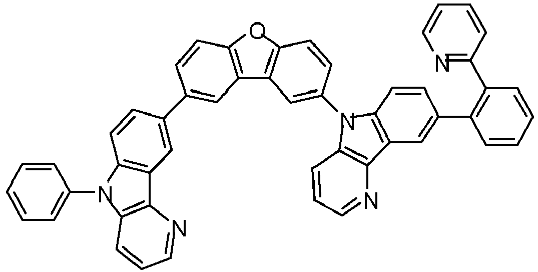 **(ETM-5),** and a compound of formula

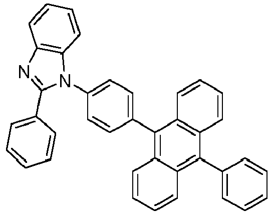

For example, the electron transporting material that may be present in the electron transporting layer of the OLED according to the present invention is an electron transporting material represented by formula (1):

A1(-L1-L2-L3-L4-Ar1)m (1)

wherein:
each of L1, L2, L3, and L4 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar1 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A1 represents an m-valent residue of a ring-containing compound represented by formula (2); and
m represents an integer of 1 or more: 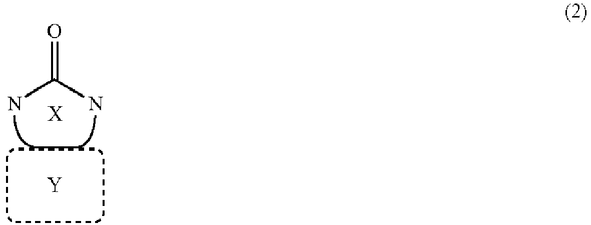
wherein:
ring X is a substituted or unsubstituted, saturated or unsaturated 5- to 8-membered ring having a ring nitrogen atom and a ring carbon atom;
the ring X may be fused to one or more rings Y; and
the ring Y represents a substituted or unsubstituted hydrocarbon ring or a substituted or unsubstituted heteroring;

The ring Y preferably represents a substituted or unsubstituted non-fused aromatic hydrocarbon ring having 6 to 30 ring carbon atoms, a substituted or unsubstituted fused aromatic hydrocarbon ring having 10 to 30 ring carbon atoms, a substituted or unsubstituted non-fused heteroring having 5 to 30 ring atoms, or a substituted or unsubstituted fused heteroring having 10 to 30 ring atoms.

The electron transporting material of the invention is preferably represented by formula (1-1) or (1-2):

A11(-L11-L21-L31-L41-Ar11)p (1-1)

wherein:
each of L11, L21, L31, and L41 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar11 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A11 represents a p-valent residue of a ring-containing compound represented by formula (2-1); and
p represents an integer of 1 or more: 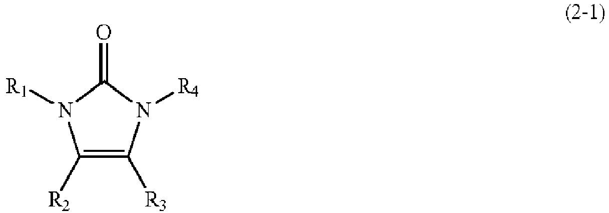
wherein;
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, a boryl group, a phosphino group, an amino group, a halogen atom, a cyano group, a nitro group, a hydroxyl group, a carboxyl group, or a valence bonded to L11; or a pair of R1 and R2, R2 and R3, or R3 and R4 are bonded to each other to form a ring Y represented by a substituted or unsubstituted hydrocarbon ring or a substituted or unsubstituted heteroring.

A11 of formula (1-1) preferably represents a p-valent residue of a compound represented by formula (2-1-1), (2-1-2), or (2-1-3): 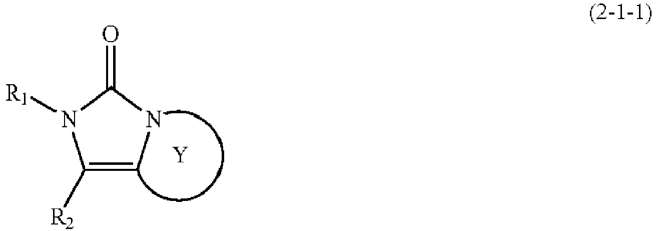 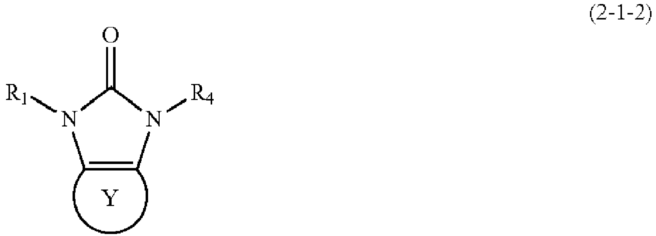 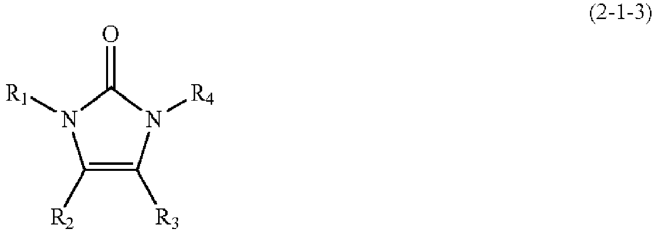 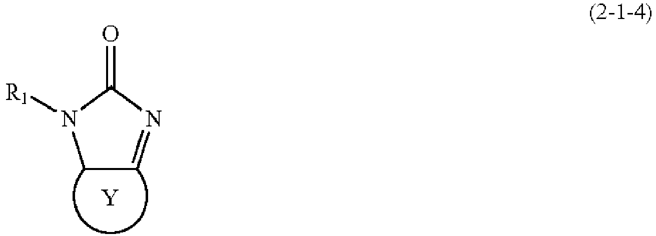 wherein:
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, an amino group substituted by a substituted or unsubstituted alkyl group having 1 to 50 carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; and
Y represents the ring Y.

Further, A11 preferably represents a p-valent residue of a compound represented by formula (2-1-2-1): 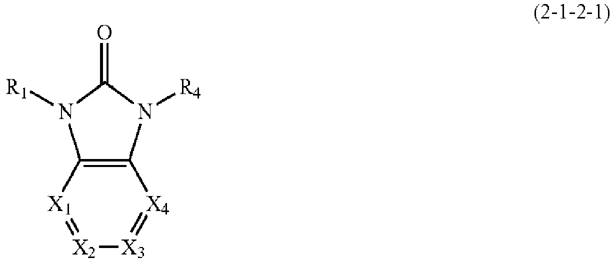 wherein:
each of X1 to X4 independently represents CR5 or N;
each of R1, R4, and R5 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; or R1, R4, and R5 are each bonded to each other to form a ring which forms a part of the ring Y.

In one embodiment, the electron transporting layer of the electronic device, preferably of the OLED according to the present invention, between the light emitting layer and the cathode, preferably comprises at least one compound of the general formula (I).

In a preferred embodiment, the electron transporting layer comprising at least one compound of the general formula (I) further comprises a reducing dopant.

Examples of the reducing dopant include a donating metal, a donating metal compound, and a donating metal complex. The reducing dopant may be used alone or in combination of two or more.

The reducing dopant referred to herein is an electron-donating material. The electron-donating material is a material which generates radical anions by the interaction with a coexisting organic material in the electron transporting layer or an organic material in a layer adjacent to the electron transporting layer, or a material having an electron-donating radical.

The donating metal is a metal having a work function of 3.8 eV or less, preferably an alkali metal, an alkaline earth metal, or a rare earth metal, and more preferably Cs, Li, Na, Sr, K, Mg, Ca, Ba, Yb, Eu, or Ce.

The donating metal compound is a compound comprising the above donating metal, preferably a compound comprising an alkali metal, an alkaline earth metal, or a rare earth metal, and more preferably a halide, an oxide, a carbonate, or a borate of these metals, for example, a compound represented by MOₓ (M: donating metal, x: 0.5 to 1.5), MFₓ (x: 1 to 3), or M(CO₃)ₓ (x: 0.5 to 1.5).

The donating metal complex is a complex comprising the above donating metal, preferably an organic metal complex of an alkali metal, an alkaline earth metal or a rare earth metal, and more preferably an organic metal complex represented by formula (I'):

MQₙ (I')

wherein M is a donating metal, Q is a ligand, preferably a carboxylic acid derivative, a diketone derivative, or a quinoline derivative, and n is an integer of 1 to 4.

Examples of the donating metal complex include watermill-shaped tungsten compounds and phthalocyanine compounds having an alkali metal or an alkaline earth metal as the central metal.

The reducing dopant is preferably at least one selected from the group consisting of an alkali metal, an alkaline earth metal, a rare earth metal, an alkali metal oxide, an alkali metal halide, an alkaline earth metal oxide, an alkaline earth metal halide, a rare earth metal oxide, a rare earth metal halide, an organic complex having an alkali metal, an organic complex having an alkaline earth metal, and an organic complex having a rare earth metal, and more preferably a 8-quinolinol complex of an alkali metal.

Examples of the alkali metal includes:
Li (lithium, work function: 2.93 eV),
Na (sodium, work function: 2.36 eV),
K (potassium, work function: 2.3 eV),
Rb (rubidium, work function: 2.16 eV), and
Cs (cesium, work function: 1.95 eV).

The values of work functions are based on Handbook of Chemistry (Pure Chemistry II, 1984, p. 493, edited by The Chemical Society of Japan). The same applies hereafter

Preferred examples of the alkaline earth metals are:
Ca (calcium, work function: 2.9 eV),
Mg (magnesium, work function: 3.66 eV),
Ba (barium, work function: 2.52 eV), and
Sr (strontium, work function: 2.0 to 2.5 eV).

The work function of strontium is based of Physics of Semiconductor Device (N.Y., Wiley, 1969, p. 366).

Preferred examples of the rare earth metal are:
Yb (ytterbium, work function: 2.6 eV),
Eu (europium, work function: 2.5 eV),
Gd (gadolinium, work function: 3.1 eV), and
Er (erbium, work function: 2.5 eV).

Examples of the alkali metal oxide include Li₂O, LiO, and NaO. The alkaline earth metal oxide is preferably CaO, BaO, SrO, BeO, or MgO.

Examples of the alkali metal halide include a fluoride, for example, LiF, NaF, CsF, and KF and a chloride, for example, LiCI, KCI, and NaCl.

The alkaline earth metal halide is preferably a fluoride, such as CaF₂, BaF₂, SrF₂, MgF₂, and BeF₂ and a halide other than fluoride.

An electronic device, preferably an OLED, wherein at least one compound according to general formula (I) used in the electron transporting layer is particularly preferred because the driving voltage is reduced or/and the efficiency increases.

The electron transporting layer facilitates the injection of electrons into the light emitting layer and transports the electrons to the light emitting zone, and has a large electron mobility and an electron affinity generally as large as 2.5 eV or more. The electron transporting layer is preferably formed from a material capable of transporting electrons to the light emitting layer at a lower strength of electric field, preferably having an electron mobility of, for example, at least 10⁻⁶ cm²/V·s under an electric field of 10⁴ to 10⁶ V/cm.

The material for forming the electron injecting/transporting layer in combination with the compound according to general formula (I) or without the compound according to general formula (I) is not particularly limited as long as having the preferred properties mentioned above and may be selected from those commonly used as the electron transporting material in the field of photoconductive materials and those known as the materials for the electron injecting/transporting layer of organic EL devices.

In the present invention, an electron injecting layer including an insulating material or a semiconductor may be disposed between the cathode and the organic layer. By such an electron injecting layer, the leak of electric current is effectively prevented to improve the electron injecting ability. Preferred examples of the insulating material include at least one metal compound selected from the group consisting of an alkali metal chalcogenide, an alkaline earth metal chalcogenide, an alkali metal halide, and an alkaline earth metal halide. An electron injecting layer including the above alkali metal chalcogenide is preferred because the electron injecting property is further improved. Preferred alkali metal chalcogenides include Li₂O, K₂O, Na₂S, Na₂Se, and Na₂O; preferred alkaline earth metal chalcogenides include CaO, BaO, SrO, BeO, BaS, and CaSe; preferred alkali metal halides include LiF, NaF, KF, LiCl, KCl, and NaCl; and preferred alkaline earth metal halides include fluoride such as CaF₂, BaF₂, SrF₂, MgF₂, and BeF₂ and halides other than fluoride.

Examples of the semiconductor for the electron transporting layer include an oxide, a nitride and an oxynitride of at least one element selected from Ba, Ca, Sr, Yb, Al, Ga, In, Li, Na, Cd, Mg, Si, Ta, Sb, and Zn, which are used singly or in combination of two or more. It is preferred that the inorganic compound constituting the electron transporting layer forms a microcrystalline or amorphous insulating thin film. When constituted of the insulating thin film described above, the electron injecting layer is made more uniform to reduce the pixel defect such as dark spots. Examples of such a inorganic compound include the alkali metal chalcogenide, the alkaline earth metal chalcogenide, the alkali metal halide and the alkaline earth metal halide which are described above.

### Electron injection layer (h):

The electron injection layer may be any layer that improves the injection of electrons into an adjacent organic layer.

Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångstrom), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 A.

The person skilled in the art is aware (for example on the basis of electrochemical studies) of how suitable materials have to be selected.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive electronic device, preferably OLED, can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds according to general formula (I) in at least one layer of the OLED, preferably in the light-emitting layer, preferably as a host material, a charge transporting material, particularly preferably as a host material and hole or electron transporting material, makes it possible to obtain OLEDs, with high efficiency and with low use and operating voltage. Frequently, the OLEDs obtained by the use of the compounds according to general formula (I) additionally have long lifetime. The efficiency of the electronic devices, preferably OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

The following examples are included for illustrative purposes only and do not limit the scope of the claims. Unless otherwise stated, all parts and percentages are by weight.

### Examples

### Compounds synthesized

### Synthesis Example 1

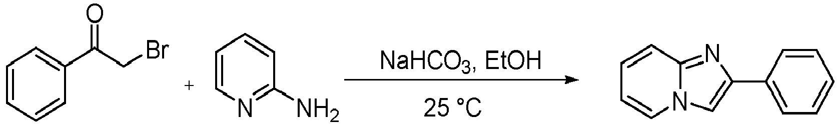

To a 100 mL round-bottom-flask were added 2-aminopyridine (10.4 g, 110.53 mmol), 2-bromoacetophenone (20.0 g, 100.48 mmol) and sodium bicarbonate (13.17 g, 156.75 mmol) in EtOH (80 mL). The reaction was allowed to stir at room temperature under argon for 20 h. The solids were filtered off, and the resulting filtrate solution was evaporated. The crude was dissolved in DCM and washed with water (30 mL). The *aqueous* layer was extracted with DCM (2 x 100 mL). The combined organic layers were washed with brine, dried (Na₂SO₄), filtered and concentrated *in vacuo.* The resulting solid was recrystallized from *i*PrOH to give 14 g (72%) white powder. The molecular mass of the product was confirmed by LC-MS [M-H] 194.9.

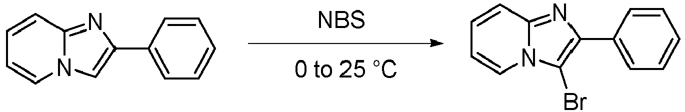

To a 100 mL round-bottom-flask was added 2-phenylimidazo[1,2-a]pyridine (5 g, 25.74 mmol) and acetonitrile (26 mL). The suspension was cooled to 0 °C. NBS (4.81 g, 27.03 mmol) was added portionwise over 10 min. The orange reaction was stirred at room temperature for 1 h. The reaction was concentrated *in vacuo,* then the residue was dissolved in EtOAc (40 mL) and washed with 1 M NaOH (30 mL). The aqueous layer was extracted with EtOAc (2 x 40 mL), and the combined organic layers were dried (Na₂SO₄), filtered and concentrated to give a black solid. The solid was further purified by filtration over a silica plug, rinsing with EtOAc/heptanes/DCM. After concentration *in vacuo,* 5.6 g (80%) of brown solid were obtained. The molecular mass of the product was confirmed by LC-MS [M-H] 275.0.

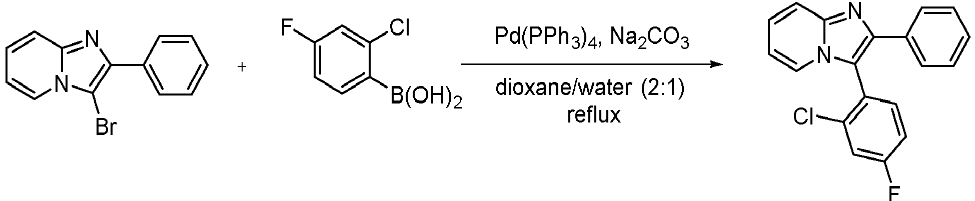

To a 100 mL 3-necked round-bottom-flask were added 3-bromo-2-phenylimidazo[1,2-a]pyridin (3.28 g, 12.0 mmol), 2-chloro-4-fluorophenylboronic acid (2.72 g, 15.6 mmol), sodium carbonate (2.54 g, 24.0 mmol), Pd(PPh₃)₄ (0.416 g, 0.36 mmol), dioxane (24 mL) and water (12 mL). The reaction was fitted with a reflux condenser and purged with Ar for 5 min. The reaction was gently refluxed for 17 h. After cooling to room temperature, the reaction was filtered over a pad of silica/celite, rinsing with EtOAc. The filtrate was transferred to a 250 mL separation funnel where it was washed with water (40 mL). The layers were separated and the aqueous layer was further extracted with EtOAc (2 x 30 mL). The combined organic layers were washed with brine, dried (Na₂SO₄), filtered and concentrated *in vacuo.* The residue was purified via flash chromatography (EtOAc/heptanes) to give 3.39 g (87%) of a yellow solid. The molecular mass of the product was confirmed by LC-MS [M-H] 323.1.

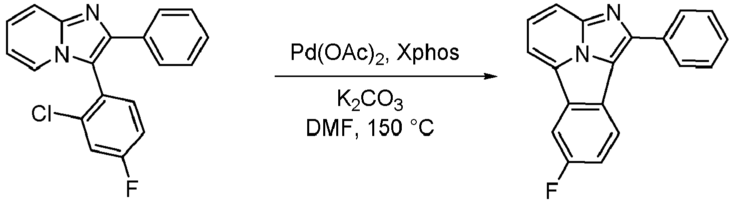

To a dried Schlenk flask was added 3-(2-chloro-4-fluorophenyl)-2-phenylimidazo[1,2-a]pyridine (3 g, 9.29 mmol), K₂CO₃ (1.93 g, 13.94 mmol), Xphos (0.266 g, 0.56 mmol) and Pd(OAc)₂ (0.063 g, 0.29 mmol). The reaction was evacuated and backfilled with Ar (x3), then degassed DMF (40 ml) was added. The reaction was heated to 150 °C for 15 h. The reaction was cooled down, then filtered over a pad of silica and celite, rinsing with EtOAc. The filtrate was concentrated, then diluted with DCM (30 mL) and transferred to a 250 mL separation funnel. The organic layer was washed with water (25 mL). The layers were separated and the aq layer was further extracted with DCM (2 x 20 mL). The combined organic layers were washed with brine, dried (Na₂SO₄), filtered and concentrated *in vacuo.* The residue was purified via flash chromatography (EtOAc/heptanes) to give 1.72 g (60%) of a yellow solid. The molecular mass of the product was confirmed by LC-MS [M-H] 287.2.

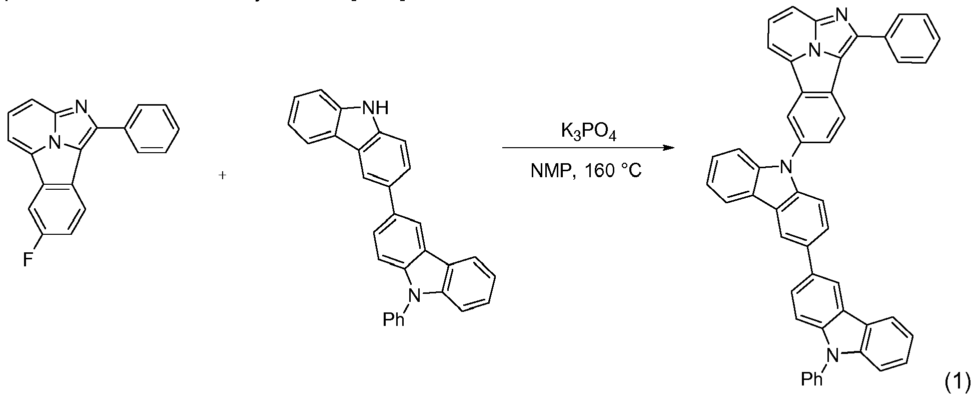

To a dried 100 mL 3-necked round-bottom-flask was added 7-fluoro-1-phenylbenzo[a]imidazo[5,1,2-cd]indolizine (1.6 g, 5.59 mmol), 9-phenyl-9H,9'H-3,3'-bicarbazole (2.51 g, 6.5 mmol), K₃PO₄ (2.97 g, 14.0 mmol) and NMP (27 mL) under Ar. The reaction was heated at 160 °C for 22 h. The reaction was allowed to cool down to room temperature and filtered, rinsing with EtOH and H₂O. The yellow solid was purified via flash chromatography (PhMe/heptanes) to give 3.37 g (89%) yellow solid. The molecular mass of the product was confirmed by LC-MS [M+H] 675.5.

### Application Examples

### Application Example 1

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As the first layer, 5 nm-thick of electron accepting Compound A is vapor-deposited. Then 210 nm-thick of aromatic amine Compound B is applied as a hole transporting layer. Then, a mixture of 2% by weight of an emitter compound (compound C), 98% by weight of a host (Compound 1) are applied to form a 40 nm-thick phosphorescent-emitting layer. On the emitting layer, a mixture of 50% by weight of an electron transporting compound (Compound D), 50% by weight of Liq (8-hydroxyquinolate lithium) is applied to form a 30 nm-thick as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

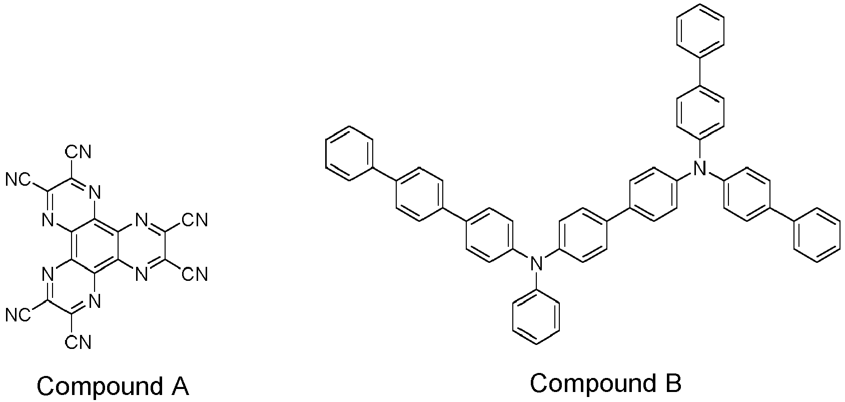

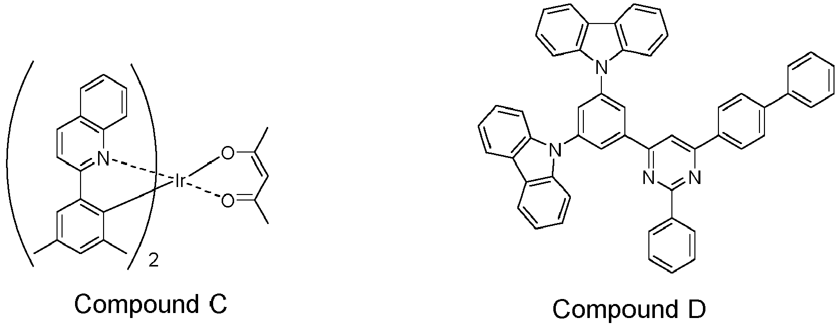

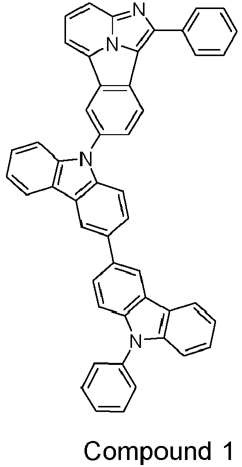

### Comparative Application Example 2

Application Example 2 is repeated except that the host (compound 1) is replaced by a comparative compound (Comparative 1). The device results are shown in Table 1.

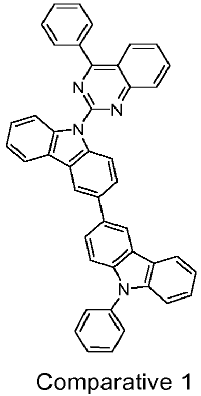

### OLED characterization

To characterize the OLED, electroluminescence spectra were recorded at various currents and voltages. In addition, the current-voltage characteristic was measured in combination with the luminance to determine luminous efficiency and external quantum efficiency (EQE). Driving voltage U, EQE and Commission Internationale de l'Éclairage (CIE) coordinate are given at 10mA/cm² except otherwise stated.

**Table 1**

| Appl. Ex. | Host | U (V) | EQE (%) | CIE x, y |
|---|---|---|---|---|
| Appl. Ex. 1 | Compound 1 | 4.98 | 16.47 | 0.66, 0.34 |
| Appl. Ex. 2 | Comparative 1 | 5.04 | 14.98 | 0.65, 0.34 |

The CIE values from table 1 show that the electroluminescence is originated from the red emitter compound (Compound C). The compound 1 shows a lower driving voltage and a higher EQE than the comparative compound (Comparative 1).

### Application Example 3

A glass substrate with 130 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, 5 nm-thick of compound E is applied. Then 80 nm-thick of compound B and 10 nm-thick of compound F are applied as a first and a second hole transporting layers, respectively. Subsequently, a mixture of 4% by weight of an emitter compound H and 96% by weight of a host (compound G) are applied to form a 25 nm-thick of fluorescent-emitting layer. On the emitting layer, a mixture of 50% by weight of Liq and 50% by weight of compound 1 are applied to form a 25 nm-thick electron transport layer (ET). Finally, 1 nm-thick Liq is deposited as electron injection layer and 80 nm-thick Al is deposited as a cathode to complete the device. The device is sealed with a glass lid and a getter in an inert nitrogen atmosphere with less than 1 ppm of water and oxygen.

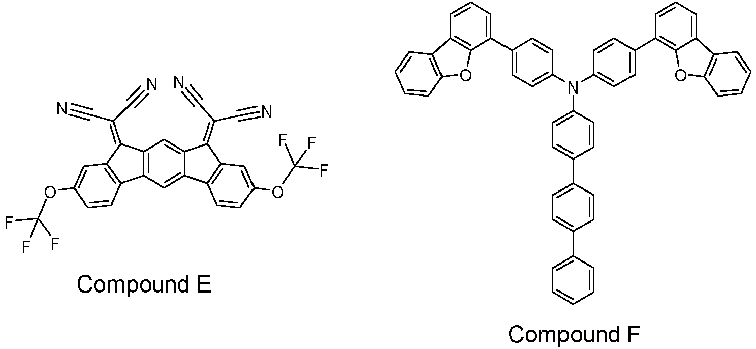

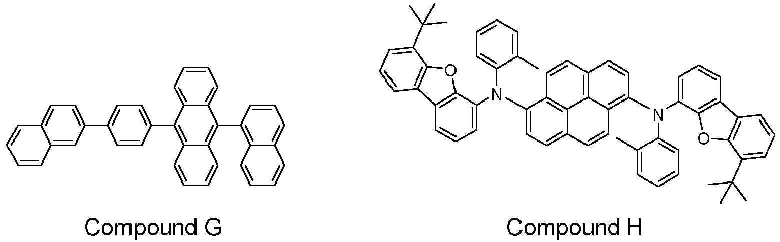

### Comparative Application Example 4

Application Example 4 is repeated as above except that the ET (compound 1) is replaced by a comparative compound (Comparative 2). The device results are shown in Table 2.

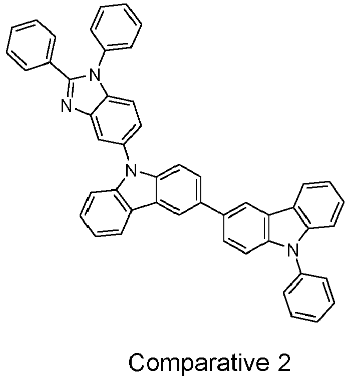

### OLED Characterization

To characterize the OLED, electroluminescence spectra were recorded at various currents and voltages. In addition, the current-voltage characteristic was measured in combination with the luminance to determine luminous efficiency and external quantum efficiency (EQE). Driving voltage U, EQE and Commission Internationale de l'Éclairage (CIE) coordinate are given at 10mA/cm² except otherwise stated.

**Table 2**

| Appl. Ex. | ET | U (V) | EQE (%) | CIE x, y |
|---|---|---|---|---|
| Appl. Ex. 3 | Compound 1 | 4.31 | 8.5 | 0.14, 0.09 |
| Appl. Ex. 4 | Comparative 2 | 8.26 | 4.1 | 0.14, 0.09 |

The results shown in table 2 demonstrate that the driving voltage and EQE are improved when compound 1 is used as electron transport material (ET) instead of the reference compound (Comparative 2).

### Comparative Application Example 5

Fabrication of an organic EL device was carried out in the same manner as in example 3 except for using the following comparative compound 2 as an electron transporting material. However, the device comprising comparative compound 2 could not be fabricated due to the unstable vapor deposition properties of comparative compound 2.

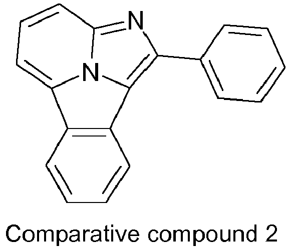

## Claims

1. A compound of general formula (I) 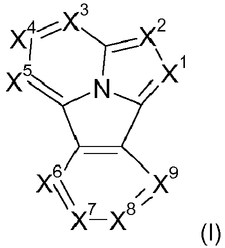 wherein
X¹ is CR¹ or N,
X² is CR² or N,
X³ is CR³ or N,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
wherein at least one of X¹ and X² is N,
R¹, R², R³, R⁴, R⁵, R⁶, R⁷, R⁸ and R⁹
are independently of each other H, -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which unsubstituted or substituted by at least one group E, a C₅-C₁₂cycloalkyl group which unsubstituted or substituted by at least one group E, -OR21, -SR²¹, -NR¹⁷R¹⁸, -COR²⁰, -COOR¹⁹, -CONR¹⁷R¹⁸, -SiR²²R²³R²⁴, -POR²⁵R²⁷, or halogen,
wherein at least one of X¹, X², X³, X⁴, X⁵, X⁶, X⁷, X⁸ and X⁹ is CR¹, CR², CR³, CR⁴, CR⁵, CR⁶, CR⁷, CR⁸ or CR⁹, wherein R¹, R², R³, R⁴, R⁵, R⁶, R⁷, R⁸ or R⁹ is -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen,
or
at least two of R³, R⁴, R⁵, R⁶, R⁷, R⁸ and R⁹ if present at adjacent carbon atoms together form at least one C₆-C₁₈aryl or C₁-C₂₄heteroaryl ring or ring system,
L¹, L², L³ and L⁴ are independently of each other selected from a C₆-C₄₀arylene group which is unsubstituted or substituted by at least one group E and a C₁-C₂₄heteroarylene group which is unsubstituted or substituted by at least one group E,
R¹⁰ is H, -CN or a C₁-C₂₅alkyl group which unsubstituted or substituted by at least one group E and/or interrupted by at least one group D,
o is independently of each other 0 or 1, p is independently of each other 0 or 1, q is independently of each other 0 or 1 and r is independently of each other 0 or 1, wherein at least one of o, p, q and r is 1;
D is independently of each other -CO-, -COO-, -S-, -SO-, -SO₂-, -O-, -CR¹⁵=CR¹⁶-, -NR¹⁷-, -SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN, -SiR²²R²³R²⁴, -POR²⁵R²⁷, halogen, a C₆-C₄₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₂₄heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein, in the case that o + p + q + r is 1, L¹, L², L³ respectively L⁴ is selected from a C₁₀-C₄arylene group which is unsubstituted or substituted by at least one group E and a heteroarylene group having 10 to 24 ring atoms, which is unsubstituted or substituted by at least one group E.

2. The compound according to claim 1, wherein X¹ is CR¹ and X² is N.

3. The compound according to claim 2 , wherein X³ is CR³, X⁴ is CR⁴, X⁵ is CR⁵, X⁶ is CR⁶, X⁷ is CR⁷, X⁸ is CR⁸ and X⁹ is CR⁹.

4. The compound according to any one of claims 1 to 3, wherein
R¹, R², R³, R⁴, R⁵, R⁶, R⁷, R⁸ and R⁹
are independently of each other H or a group of formula -(L¹)ₒ-(L²)ₚ-(L³)_{q}-(L⁴)ᵣ-R¹⁰; wherein at least one of R¹, R², R³, R⁴, R⁵, R⁶, R⁷, R⁸ and R⁹ is a group of formula -(L¹)ₒ-(L²)ₚ-(L³)_{q}-(L⁴)ᵣ-R¹⁰;
wherein
L¹, L², L³ and L⁴ are independently of each other selected from a C₆-C₄₀arylene group which is unsubstituted or substituted by at least one group E and an N comprising C₁-C₂₄heteroarylene group which is unsubstituted or substituted by at least one group E.

5. The compound according to any one of claims 1 to 4, wherein
R³, R⁴, R⁵, R⁶, R⁷ and R⁹ are H, and one or both of R¹ and R⁸ are independently of each other a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, preferably a group of formula -(L¹)ₒ-(L²)ₚ-(L³)_{q}-(L⁴)ᵣ-R¹⁰; and the other of R¹ and R⁸ which is not a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E is H or
R³, R⁴, R⁵, R⁶, R⁸ and R⁹ are H, and one or both of R¹ and R⁷ are independently of each other a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, preferably a group of formula -(L¹)ₒ-(L²)ₚ-(L³)_{q}-(L⁴)ᵣ-R¹⁰ and the other of R¹ and R⁷ which is not a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E is H.

6. The compound according to any one of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XXIV) 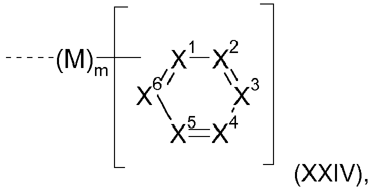 wherein
X¹, X², X³, X⁴, X⁵ and X⁶ are independently of each other CR^{c} or N, wherein in formula at least one of X¹, X², X³, X⁴, X⁵ and X⁶ is N;
R^{c} is in each case independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{c}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites, and
wherein the group - - -(M)ₘ- is either attached to the ring formed by X¹ to X⁶, wherein one of X¹ to X⁶ is a group CR^{c}, wherein R^{c} is a bonding site to - - -(M)ₘ-, or
the group - - -(M)ₘ- is attached to the ring formed of at least two of R^{c}, if present at adjacent carbon atoms.

7. The compound according to any one of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein the at least one of of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XXV) 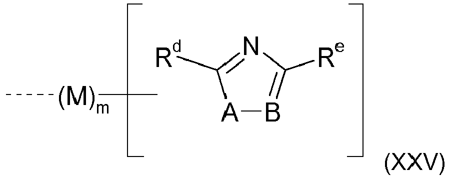 wherein
B is CR^{g} or N;
A is O, S or NR^{h};
R^{d}, R^{e}, R^{g} and R^{h} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of R^{d}, R^{e}, R^{g} and R^{h}, if present at adjacent atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites;
wherein the group - - -(M)ₘ- is either attached to the five membered ring formed by N, A, B and two carbon atoms, wherein one of R^{d}, R^{e}, R^{g} or R^{h} is a bonding site to - - -(M)ₘ-, or the group - - -(M)ₘ- is attached to the ring formed of at least two of R^{d}, R^{e}, R^{g} or R^{h}, if present at adjacent carbon atoms.

8. The compound according to any one of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an C₆-C₄₀aryl group according to general formula (XXXIV), (XXXV), (XXXVI), (XXXVII), (XXXVIII) or (XXXIX) 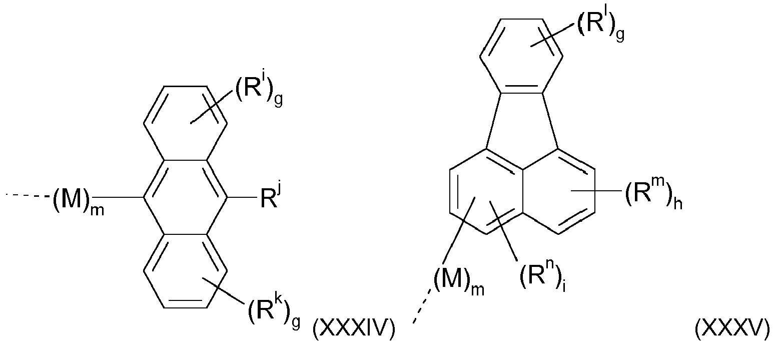 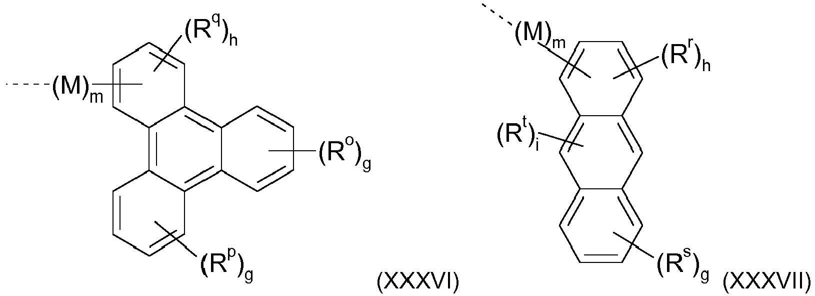 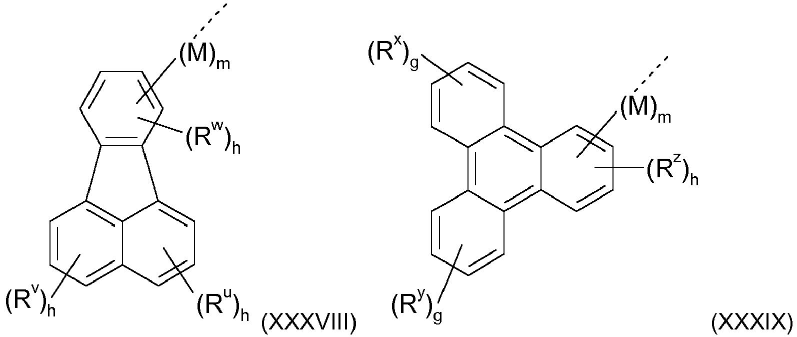 wherein
Rⁱ, R^{j}, R^{k}, R^{l}, R^{m}, Rⁿ, R^{o}, R^{p}, R^{q}, R^{s}, R^{t}, R^{u}, R^{v}, R^{w}, R^{x}, R^{y} and R^{z} are independently of each other H, a C₆-C₁₈aryl group which is unsubstituted or substituted by at least one C₁-C₁₈alkyl group or by at least one C₁-C₁₈alkoxy group, a C₁-C₂₄ heteroaryl group which is unsubstituted or substituted by at least one C₁-C₁₈alkyl group or by at least one C₁-C₁₈alkoxy group, a C₇-C₂₅aralkyl group which is unsubstituted or substituted by at least one C₁-C₁₈alkyl group or by at least one C₁-C₁₈alkoxy group, a C₅-C₁₂cycloalkyl group which is unsubstituted or substituted by at least one C₁-C₁₈alkyl group or by at least one C₁-C₁₈alkoxy group, or a C₁-C₁₈alkyl group which is optionally interrupted by at least one O;
or at least two of Rⁱ, R^{k}, R^{l}, R^{m}, Rⁿ, R^{o}, R^{p}, R^{q}, R^{s}, R^{t}, R^{u}, R^{v}, R^{w}, R^{x}, R^{y} and R^{z}, if present at adjacent carbon atoms, may form at least one five or six membered, substituted or unsubstituted, saturated, unsaturated, aromatic or heteroaromatic ring, to which at least one further substituted or unsubstituted, saturated, unsaturated, aromatic or heteroaromatic ring may be fused;
g is 0, 1, 2, 3 or 4;
h is 0, 1, 2 or 3,
i is 0, 1 or 2;
M is a C₆-C₄₀ arylene group which is unsubstituted or substituted by at least one group E, a C₁-C₂₄heteroarylene group which is unsubstituted or substituted by at least one group E;
m is 0, 1, 2 or 3, preferably 0, 1 or 2;
wherein the dotted lines are bonding sites.

9. The compound according to any one of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XII) 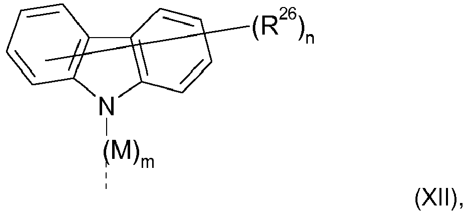 wherein
n is 0, 1, 2, 3, 4, 5, 6, 7 or 8,
m is 0, 1, 2 or 3,
M is a C₆-C₄₀ arylene group which is unsubstituted or substituted by at least one group E or a C₁-C₂₄heteroarylene group which is unsubstituted or substituted by at least one group E,
R²⁶ is independently of each other selected from E, a group of formula -(L¹)ₒ-(L²)ₚ-(L³)_{q}-(L⁴)ᵣ-R¹⁰, a C₁-C₂₅alkyl group, which is unsubstituted or substituted by at least one group E and/or interrupted by at least one group D, wherein o, p, q, r, L¹, L², L³, L⁴, R¹⁰, E and D have the meanings as defined in claim 1,
or at least two of R²⁶, if present at adjacent carbon atoms, may form at least one five or six membered, substituted or unsubstituted, saturated, unsaturated, aromatic or heteroaro-matic ring or ring system, to which at least one further aromatic and/or heteroaromatic ring or ring system may be fused,
wherein the dotted lines are bonding sites.

10. The compound according to any of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XIII), (XV) or (XVII) 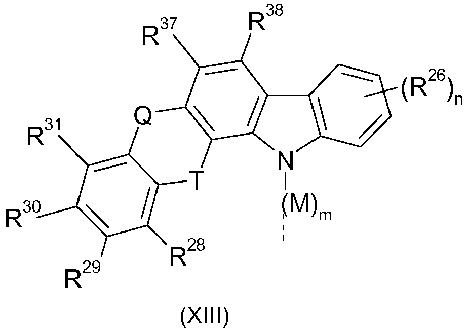
wherein M, m and R²⁶ have the meanings as defined in claim 9, n is 0, 1, 2, 3 or 4,
R²⁸, R²⁹, R³⁰, R³¹, R³⁷ and R³⁸ are independently of each other selected from H, E, a group of formula -(L¹)ₒ-(L²)ₚ-(L³)_{q}-(L⁴)ᵣ-R¹⁰, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein o, p, q, r, L¹, L², L³, L⁴, R¹⁰, D and E have the same meanings as defined in claim 1, 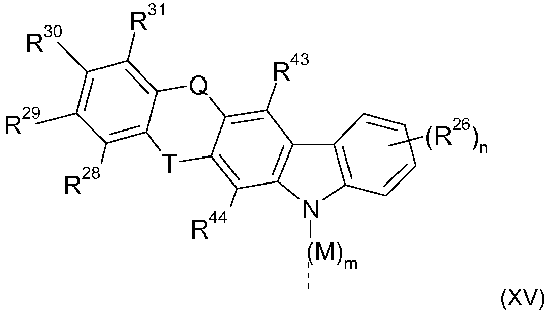
wherein M, m and R²⁶ have the meanings as defined in claim 9, n is 0, 1, 2, 3 or 4,
R²⁸, R²⁹, R³⁰, R³¹, R⁴³ and R⁴⁴ are independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R²⁸, R²⁹, R³⁰ or R³¹, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein o, p, q, r, L¹, L², L³, L⁴, R¹⁰, D and E have the same meanings as defined in claim 1, 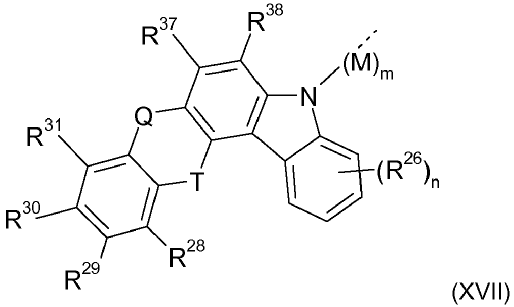
wherein M, m and R²⁶ have the meanings as defined in claim 9, n is 0, 1, 2, 3 or 4,
R²⁸, R²⁹, R³⁰, R³¹, R⁴³ and R⁴⁴ are independently of each other selected from H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R²⁸, R²⁹, R³⁰ or R³¹, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ or NR³⁶,
wherein R³² and R³³ are independently of each other H, a C₁-C₁₈alkyl group, a C₆-C₁₈aryl group which is unsubstituted or substituted by at least one C₁-C₁₈alkyl group,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein o, p, q, r, L¹, L², L³, L⁴, R¹⁰, D and E have the same meanings as defined in claim 1,
wherein the dotted lines are bonding sites.

11. The compound according to claim 10, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XIV) or (XVIII) 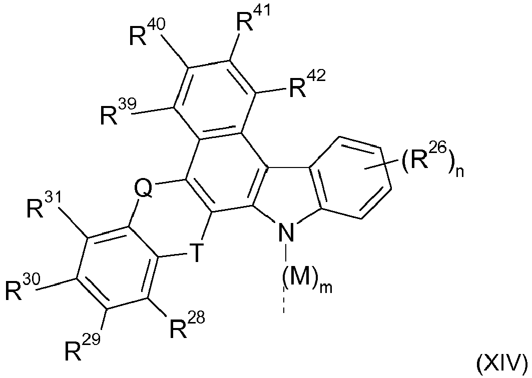 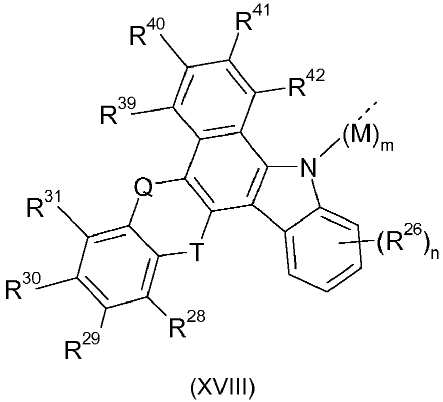
wherein R²⁶, R²⁸, R²⁹, R³⁰, R³¹, M, Q, T, n and m have the same meanings as defined in claim 10 and
R³⁹, R⁴⁰, R⁴¹, R⁴² are independently of each other selected from H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
wherein the dotted lines are bonding sites.

12. The compound according to any of claims 9 to 11, wherein m is 0, 1 or 2, M is a C₆-C₄₀arylene group which is unsubstituted or substituted by at least one group E, and at least two of R²⁶, if present at adjacent carbon atoms, form at least one five or six membered, substituted or unsubstituted, saturated, unsaturated, aromatic or heteroaromatic ring, wherein E has the meanings as defined in claim 1.

13. The compound according to any of claims 1 to 5, wherein R¹, R², R³, R⁴, R⁵, R⁶, R⁷, R⁸ and R⁹ are each independently H or a group of formula -(L¹)ₒ-(L²)ₚ-(L³)_{q}-(L⁴)ᵣ-R¹⁰, wherein at least one of R¹, R², R³, R⁴, R⁵, R⁶, R⁷, R⁸ and R⁹ in the at least one group CR¹, CR², CR³, CR⁴, CR⁵, CR⁶, CR⁷, CR⁸ or CR⁹ is a group of formula -(L¹)ₒ-(L²)ₚ-(L³)_{q}-(L⁴)ᵣ-R¹⁰ comprising an N comprising C₁-C₂₄heteroaryl group according to general formula (XXI) 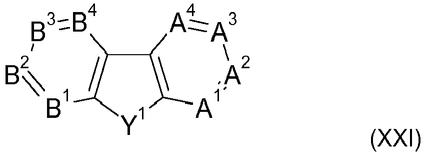 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is independently of each other NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a group of formula -(L¹)ₒ-(L²)ₚ-(L³)_{q}-(L⁴)ᵣ-R¹⁰, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁶², R⁶³, R⁶⁴ and R⁶⁵ , if present are directly bonded to the moiety represented by the general formula (XXII) by the two *-locations. 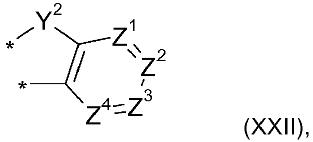
wherein
Y² is independently of each other NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and/or
R⁶², R⁶³, R⁶⁴ or R⁶⁵, if present at adjacent carbon atoms, together form at least one C₆-C₁₈aryl or C₂-C₁₈heteroaryl ring or ring system,
and/or
at least two of R⁶⁶, R⁶⁷, R⁶⁸ and R⁶⁹, if present are directly bonded to the moiety represented by the general formula (XXIII) by the two *-locations. 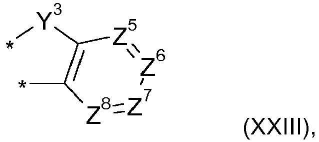 wherein
Y³ is independently of each other NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from direct bond, H, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
R⁷¹, R⁷², R⁷⁴, R⁷⁵, R⁸¹ and R⁸² are, independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other selected from H, direct bond, E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁷⁶, R⁷⁷, R⁷⁸, R⁷⁹, R⁸³, R⁸⁴, R⁸⁵ or R⁸⁶, if present at adjacent carbon atoms, together form at least one C₆-C₁₈aryl or C₂-C₁₈heteroaryl ring or ring system,
wherein the substituent according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
m is 0, 1, 2 or 3,
M is a C₆-C₄₀ arylene group which is unsubstituted or substituted by at least one group E or a C₁-C₂₄heteroarylene group which is unsubstituted or substituted by at least one group E,
wherein o, p, q, r, L¹, L², L³, L⁴, R¹⁰, D and E have the same meanings as defined in claim 1.

14. The compound according to claim 13, wherein the heterocyclic group according to general formula (XXI) is represented by any one of general formula (XXIa), (XXIb) or (XXIc) 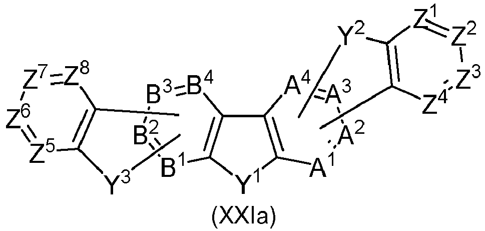 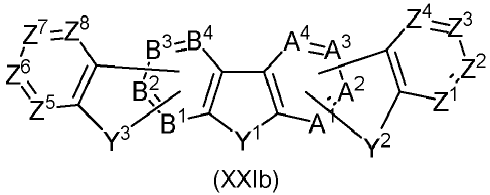 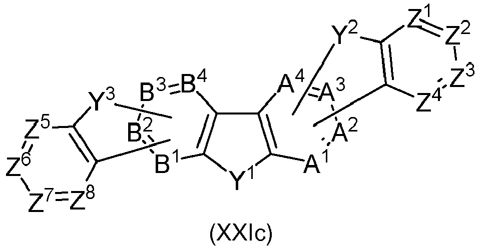 wherein A¹, A², A3, A4, B¹, B², B³, B⁴, Z¹, Z², Z³, Z⁴, Z⁵, Z⁶, Z⁷, Z⁸, Y¹, Y² and Y³ have the same meanings as defined in claim 13.

15. The compound according to claim 13 or 14 wherein one or two of Y¹, Y² and Y³ is NR⁷⁰, NR⁷³ or NR⁸⁰ respectively.

16. The compound according to any one of claim 13 to 15 wherein the substituent according to general formula (XXI) is connected to the compound according to general formula (I) via one of R⁷⁰, R⁷³ or R⁸⁰, wherein this respective R⁷⁰, R⁷³ or R⁸⁰ is a direct bond, optionally interrupted by a group of formula -(M)ₘ-, wherein M and m have the meanings as defined in claim 13.

17. The compound according to claim 9, wherein the N-heteroaryl group according to general formula (XII) corresponds to a group according to general formula (XX) 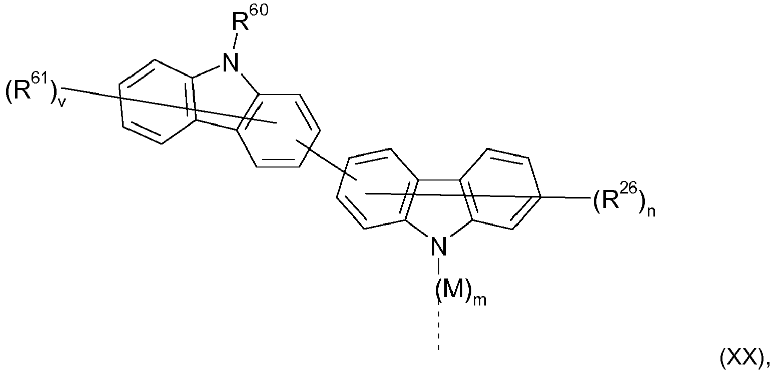 wherein
m, M, R²⁶ have the same meanings as defined in claim 9,
n is 0, 1, 2, 3, 4, 5, 6 or 7,
v is 0, 1, 2, 3, 4, 5, 6 or 7,
R⁶⁰ is independently of each other selected from H, E, a group of formula -(L¹)ₒ-(L²)ₚ-(L³)_{q}-(L⁴)ᵣ-R¹⁰, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, R²⁶ is independently of each other selected from E, a group of formula -(L¹)ₒ-(L²)ₚ-(L³)_{q}-(L⁴)ᵣ-R¹⁴, a C₁-C₂₅alkyl group, which is unsubstituted or substituted by at least one group E and/or interrupted by at least one group D, wherein o, p, q, r, L¹, L², L³, L⁴, R¹⁰, E and D have the meanings as defined in claim 1,
R⁶¹ is independently of each other selected from E, a group of formula -(L¹)ₒ-(L²)ₚ-(L³)_{q}-(L⁴)ᵣ-R¹⁰, a C₁-C₂₅alkyl group, which is unsubstituted or substituted by at least one group E and/or interrupted by at least one group D, wherein o, p, q, r, L¹, L², L³, L⁴, R¹⁰, E and D have the meanings as defined in claim 1,
or at least two of R⁶¹, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
wherein the dotted lines are bonding sites.

18. A process for the preparation of a compound according to general formula (I) as defined in any one of claims 1 to 17, at least comprising step (A)
(A) coupling of a compound according to general formula (Ia) 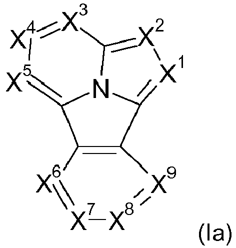
wherein at least one of X¹, X², X³, X⁴, X⁵, X⁶, X⁷, X⁸ and X⁹ is C-A,
with a compound of formula R¹-H, R²-H, R³-H, R⁴-H, R⁵-H, R⁶-H, R⁷-H, R⁸-H or R⁹-H to obtain a compound according to general formula (I),
wherein
A is a selected from Cl, Br, I, F, OSO₂CH₃, and OSO₂CF₃ or OSO₂C₆H₄CH₃,
wherein
X¹ is CR¹ or N,
X² is CR² or N,
X³ is CR³ or N,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
wherein at least one of X¹ and X² is N,
R¹, R², R³, R⁴, R⁵, R⁶, R⁷, R⁸ and R⁹
are independently of each other H, -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen,
wherein R¹, R², R³, R⁴, R⁵, R⁶, R⁷, R⁸ or R⁹ in R¹-H, R²-H, R³-H, R⁴-H, R⁵-H, R⁶-H, R⁷-H, R⁸-H or R⁹-H is -CN, a C₁-C₂₅alkyl group which is unsubstituted or substituted by at least one group E and/or interrupted by at least one group D, a group of formula -(L¹)ₒ-(L²)ₚ-(L³)_{q}-(L⁴)ᵣ-R¹⁰, a C₇-C₂₅aralkyl group which is unsubstituted or substituted by at least one group E, a C₅-C₁₂cycloalkyl group which is unsubstituted or substituted by at least one group E, -OR²¹, -SR²¹, -NR¹⁷R¹⁸, -COR²⁰, -COOR¹⁹, -CONR¹⁷R¹⁸, -SiR²²R²³R²⁴, -POR²⁵R²⁷, or halogen, or
at least two of R³, R⁴, R⁵, R⁶, R⁷, R⁸ and R⁹ if present at adjacent carbon atoms together form at least one C₆-C₁₈aryl or C₁-C₂₄heteroaryl ring or ring system,
L¹, L², L³ and L⁴ are independently of each other selected from a C₆-C₄₀aryl group which is unsubstituted or substituted by at least one group E and a C₁-C₂₄heteroaryl group which is unsubstituted or substituted by at least one group E,
R¹⁰ is H, -CN or a C₁-C₂₅alkyl group which is unsubstituted or substituted by at least one group E and/or interrupted by at least one group D,
o is independently of each other 0 or 1, p is independently of each other 0 or 1, q is independently of each other 0 or 1 and r is independently of each other 0 or 1, wherein at least one of o, p, q and r is 1,
D is independently of each other -CO-, -COO-, -S-, -SO-, -SO₂-, -O-, -CR¹⁵=CR¹⁶-, -NR¹⁷-, -SiR²²R²³-, -POR²⁵-, -C≡C-,
E is independently of each other -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₄₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl or a C₁-C₁₈alkyl group which is interrupted by at least one O, a C₁-C₂₄heteroaryl group which is unsubstituted or substituted by at least one -F, -CF₃, -CF₂CF₃, -CF₂CF₂CF₃, -CF(CF₃)₂, -(CF₂)₃CF₃ or -C(CF₃)₃,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R^{22,} R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein, in the case that o + p + q + r is 1, L¹, L², L³ respectively L⁴ is selected from a C₁₀-C₄₀arylene group which is unsubstituted or substituted by at least one group E and a heteroarylene group having 10 to 24 ring atoms, which is unsubstituted or substituted by at least one group E.

19. An electronic device comprising a cathode, an anode, and a plurality of organic layers provided between the cathode and the anode, wherein at least one of the plurality of organic layers is an emitting layer, wherein at least one organic layer comprises at least one compound as defined in any of claims 1 to 17.

20. The electronic device according to claim 19, wherein the emitting layer comprises the at least one compound as defined in any of claims 1 to 17 as a host material.

21. The electronic device according to claim 19 or 20, wherein the emitting layer comprises a heavy-metal complex material.

22. The electronic device according to any one of claims 19 to 21, wherein the organic layers comprise an electron transporting layer comprising the at least one compound as defined in any of claims 1 to 17, said electron transporting layer placed between the anode and the emitting layer.

23. The electronic device according to any one of claims 19 to 22, wherein at least one of the layers between the emitting layer and the anode comprises at least one selected from an alkali metal, an alkaline earth metal, a rare earth metal, a compound comprising an alkali metal, an alkaline earth metal, or a rare earth metal, and a complex comprising an alkali metal, an alkaline earth metal, or a rare earth metal.

24. An electronic equipment comprising the electronic device according to any one of claims 19 to 23.

25. An emitting layer or an electron transport layer or a hole and/or exciton blocking layer comprising at least one compound of the general formula (I) as defined in any one of claims 1 to 17.

26. Use of the compound according to general formula (I) as defined in any of claims 1 to 17 in an electronic device as a host material, an electron transporting material and/or a hole and/or exciton blocking material.
